# EUROPEAN PATENT APPLICATION

(11) **EP 4 209 221 A1**
(43) Date of publication of application: **12.07.2023**
(21) Application number: 22212024.8
(22) Date of filing: 05.05.2017
(51) Int. Cl.: A61K 31/7068, A61K 9/00, A61M 31/00, A61P 13/10, A61K 45/06

(54) **METHOD OF TREATING LOWER TRACT UROTHELIAL CANCER**

(30) Priority: 06.05.2016 US 201662333151 P; 06.01.2017 US 201762443614 P
(62) Divisional of application: 17793523.6
(71) Applicant: TARIS Biomedical LLC, Lexington, Massachusetts 02421 (US)
(72) Inventor: GIESING, Dennis, Lee's Summit, 64082 (US); CUTIE, Christopher, Lexington, 02421 (US); SARMA, Purnanand, Carlisle, 01741 (US); LARRIVEE-ELKINS, Cheryl, Framingham, 01701 (US); SEARCY, Christopher, Charlestown, 02129 (US); AGARWAL, Vikas, Lexington, 02421 (US)
(74) Representative: Carpmaels & Ransford LLP

(57) **Abstract**

The present invention provides methods, devices, and kits related to treatment of urothelial carcinomas of the lower tract with antimetabolite (such as gemcitabine). In some aspects the methods, devices and kits relate to treatment of urothelial carcinomas of the lower tract with an antimetabolite (such as gemcitabine) and an immunomodulating agent.

## Description

### CROSS-REFERENCE TO RELATED APPLICATIONS

This application claims the benefit of, and priority to U.S. provisional application 62/333,151, filed May 6, 2016 and U.S. provisional application 62/443,614 filed January 6, 2017. The contents of each of these applications are incorporated herein by reference in their entireties.

### BACKGROUND OF THE INVENTION

Bladder cancer is a significant medical problem, and currently available treatment options are unsatisfactory for a number of reasons. In general, bladder cancers are classified as muscle invasive bladder cancer (MIBC) or non-muscle invasive bladder cancer (NMIBC). The pathological classification and staging of bladder cancer is as follows: pTa (urothelial involvement); pTis (high risk urothelial confined); pT1 (lamina propria invasion); pT2 (muscularis invasion); pT3 (perivesical fat invasion); and pT4 (pelvic organ extension). Bladder cancers can also be classified by grade as Grade 1/3 (well differentiated); Grade 2/3 (moderately differentiated); Grade 3/3 (poorly differentiated). In addition, bladder cancers can be classified by stage as Stages 0-IV. Most bladder cancers are transitional cell carcinomas of epithelial origin and classified as non-muscle invasive cancer (NMIBC) confined to the inner lining of the bladder. At initial presentation, most bladder cancers are superficial NMIBCs and include stages pTa, pTis and pT1 disease. MIBC include stages pT2, pT3 and pT4.

The typical clinical protocol of early stage NMIBC is cystoscopy visualization followed by surgical removal of the tumor(s), known as transurethral resection (TUR). However, there is a high rate of recurrence after surgery and the cancer may progress to muscle-invasive disease. Therefore, surgery is often combined with adjuvant intravesicular (intravesical) instillation (direct delivery of the chemotherapeutic agent into the bladder through a urinary catheter) of chemotherapeutic or immunotherapeutic agents to help prevent or delay the incidence and severity of recurrence. Bacile Calmette-Guerin (BCG) is such an immunotherapeutic and is typically instilled into the bladder following surgery. However, many patients do not respond to BCG, and BCG treatment can also induce a range of adverse effects leading to discontinuation of treatment. Chemotherapeutic agents are usually reserved for patients who have failed BCG therapy. Chemotherapy is typically applied intravesically to concentrate the chemotherapeutic agent at the tumor sites and eliminate any residual tumor after resection while avoiding systemic exposure of the drug.

One such chemotherapeutic agent used in clinical trials for treating bladder cancer is gemcitabine. Gemcitabine (2'',2'-difluorodeoxycytidine) is a pyrimidine analogue with activity against metastatic bladder cancer. Gemcitabine has also been used in clinical trials to treat superficial bladder cancers and NMIBC by instillation in the bladder with various weekly schedules. Gemcitabine is typically instilled over 1 to 2 hours once or twice a week for several weeks at doses typically ranging from 500 to 2000 mg in up to 100 mL of saline.

It is known that such liquid formulations are voided from the bladder after a short dwell times of 1 to 2 hours thus limiting their therapeutic benefit. In addition, high concentrations (40 mg/mL) and high doses (up to 2 grams per instillation) are used in an attempt to achieve therapeutic tissue levels in order to try to overcome the dwell time limitations. However, intravesicular (intravesical) delivery of high doses of gemcitabine can lead to significant systemic absorption and cause gastrointestinal, bladder and bone marrow toxicity further limiting the clinical utility in addition to local tolerability issues.

Accordingly, there remains a need for improved methods for treating urothelial carcinomas of the lower tract.

Published Applications US2012/0203203, US2013/0158675, US2015/0360012, US20150165177, US2015/0165178, US20160199544, WO2014/145638, WO2015200752, WO2011/031855 are hereby incorporated by reference in their entirety. All other references disclosed herein are incorporated by reference in their entirety.

### BRIEF SUMMARY OF THE INVENTION

Local administration of an antimetabolite, such as gemcitabine, to the bladder leads to multiple biological effects, such as modulating immune responses in the tumor microenvironment, inducing systemic immunity and antigen presentation, and inducing cytotoxicity. Accordingly, the application discloses a method of treating urothelial carcinoma of the lower tract comprising administering to an individual an effective amount of an antimetabolite locally to the bladder.

In some embodiments, the method comprises a method of enhancing an immune response against a urothelial carcinoma of the lower tract of an individual comprising administering to the individual an effective amount of an antimetabolite, wherein the antimetabolite is delivered locally to the bladder.

In some embodiments, the method comprises a method of reducing recurrence or progression of a urothelial carcinoma of the lower tract in an individual comprising administering to the individual an effective amount of an antimetabolite, wherein the antimetabolite is delivered locally to the bladder.

In some embodiments, the method comprises a method of improving tumor microenvironment for cancer immunotherapy in an individual having a urothelial carcinoma of the lower tract, comprising administering to the individual an effective amount of an antimetabolite, wherein the antimetabolite is delivered locally to the bladder.

In some embodiments, the method comprises a method of sensitizing an individual having a urothelial carcinoma of the lower tract for radiation therapy, comprising administering to the individual an effective amount of an antimetabolite, wherein the antimetabolite is delivered locally to the bladder.

In some embodiments according to any of the methods described above, the method comprises a method of treating muscle invasive bladder cancer in an individual comprising delivering an effective amount of gemcitabine locally to the bladder, wherein gemcitabine is delivered continuously for at least 24 hours.

In some embodiments according to any of the methods described above, the method comprises a method of bladder preservation in an individual comprising delivering an effective amount of gemcitabine locally to the bladder, wherein gemcitabine is delivered continuously for at least 24 hours.

In some embodiments according to any of the methods described above, the method comprises a method of treating non-muscle invasive bladder cancer in an individual comprising delivering gemcitabine locally to the bladder, wherein gemcitabine is delivered continuously for at least 24 hours.

In some embodiments according to any of the methods described above, the antimetabolite may be a nucleoside analog. In some of these embodiments, the antimetabolite is gemcitabine. In some embodiments according to any of the methods described above, the antimetabolite is delivered continuously into the bladder over a period of at least about 24 hours. In some embodiments according to any of the methods described above, the antimetabolite is delivered in a first phase of the delivery at a first dose followed by a second phase of delivery at a second dose. In some of embodiments, the first phase and the second phase are contiguous. Alternatively, in some embodiments, the first and second phase can be separated by a resting period.

In some embodiments according to any of the methods described above, the antimetabolite is delivered at a dose of from about 1 mg/day to about 300 mg/day. In some embodiments according to any of the methods described above, the concentration of antimetabolite in the urine is from about 0.1 µg/mL to about 200 µg/mL during the delivery period. In some of these embodiments according to any of the methods described above, the concentration of antimetabolite in the urine is about 10 µg/mL during the delivery period. In some embodiments according to any of the methods described above, the concentration of antimetabolite in the plasma of the individual is less than about 1 µg/mL. In some embodiments according to any of the methods described above, the concentration of the antimetabolite is less than about 0.1 µg/mL. In some embodiments according to any of the methods described above, upon delivery of the antimetabolite, the ratio of antimetabolite in the urine to antimetabolite in the plasma of the individual is greater than about 500:1.

In some embodiments according to any of the methods described above, the antimetabolite is delivered over at least a month, wherein each antimetabolite delivery period is at least one day, and wherein the interval between each antimetabolite delivery period is no more than about a week. In some embodiments according to any of the methods described above, the interval between each antimetabolite period is 14 days. In some embodiments, there is no interval been each antimetabolite delivery period.

In some embodiments, the method comprises a) a first antimetabolite delivery period, wherein the concentration of antimetabolite in the urine of the individual is at least about 0.1 µg/mL; b) a rest period; and c) a second antimetabolite delivery period, wherein the concentration of antimetabolite in the urine of the individual is at least about 0.1 µg/mL for at least a portion of the rest period. In some embodiments, the concentration of antimetabolite in the urine is greater than about 1.0 µg/mL for at least about half of the rest period.

In some embodiments according to any of the methods described above, the method further comprises administering to the individual an effective amount of a second agent. In some of embodiments according to any of the methods described above, the second agent is delivered at the time the antimetabolite delivery is initiated. In some embodiments, the second agent is delivered before the antimetabolite is delivered. In some embodiments, the second agent is delivered after the antimetabolite delivery is initiated. In yet further embodiments, the second agent is delivered after the antimetabolite delivery is terminated.

In some embodiments according to any of the methods described above, the antimetabolite delivery period and the second agent delivery period may overlap with each other. In some embodiments, the antimetabolite delivery period and the second agent delivery period may be non-overlapping.

In some aspects according to any of the methods described above, the second agent is delivered systemically. In other aspects, the second agent is delivered locally. In some embodiments, the second agent is delivered systemically in a first phase of the second agent delivery period, followed by a local delivery at a second phase of the second agent delivery period. In some embodiments, the second agent is delivered locally in a first phase of the second agent delivery period, followed by a systemic delivery at a second phase of the second agent delivery period. In some of embodiments, the first phase of the second agent delivery period and the second phase of the second agent delivery period are separated by at least about a month.

In some embodiments according to any of the methods described above, the antimetabolite and the second agent are delivered simultaneously. In some of these embodiments, the antimetabolite and the second agent are delivered via a single delivery device. In some embodiments according to any of the methods described above, the antimetabolite and the second agent are delivered at the same release rate. In other embodiments, the antimetabolite and the second agent are delivered at a different release rate.

In some embodiments, the second agent is delivered separately from the antimetabolite.

In some embodiments according to any of the methods described above, the second agent is delivered systemically. In some embodiments, the second agent is delivered locally.

In some embodiments according to any of the methods described above, the second agent is a chemotherapeutic agent. In some embodiments according to any of the methods described above, the chemotherapeutic agent is selected from the group consisting of paclitaxel, docetaxel, carboplatin, cisplatin, and oxaliplatin. In some embodiments, the second agent is an immunomodulating agent. In some embodiments, the immunomodulating agent is an immune checkpoint inhibitor. In some embodiments, the immune checkpoint inhibitor is an inhibitor of an immune checkpoint protein selected from the group consisting of PD-L1, CTLA4, PD-L2, PD-1, B7-H3, B7-H4, HVEM, B- and T-lymphocyte attenuator (BTLA), Killer inhibitory receptor (KIR), GAL9, TIM3, A2AR, LAG-3, phosphatidylserine, CD27, TNF-α, CD33, Siglec-5, Siglec-7, Siglec-9, and Siglec-1 1. In some embodiments, the immunomodulating agent is an agonist of a costimulatory immune molecule. In some embodiments, the costimulatory immune molecule is selected from the group consisting of CD40, OX40, ICOS, CD28, CD137/4-1BB, CD27, IL-10, TGF-beta, TOR receptor, and glucocorticoid-induced TNFR-related protein GITR.

In some embodiments according to any of the methods above, the individual does not receive radiation therapy. In other embodiments, the method further comprises radiation therapy.

In some embodiments according to any of the methods above, the antimetabolite is delivered in a neoadjuvant setting. In some embodiments, the antimetabolite is delivered in an adjuvant setting. In some embodiments according to any of the methods described above, the antimetabolite is delivered in a pre-operative treatment. In some embodiments, the antimetabolite is delivered in a perioperative treatment.

In some embodiments according to any of the methods described above, the method further comprises a third therapy comprising surgery. In some of these embodiments, the delivery of the antimetabolite to the individual may be initiated at the time of surgery. In some embodiments according to any of the methods described above, delivery of the antimetabolite to the individual is initiated during a cystoscopy. In some embodiments according to any of the methods described above, delivery of the antimetabolite to the individual is initiated during a cystoscopy prior to surgery. In some embodiments, delivery of the antimetabolite to the individual is initiated during a cystoscopy following surgery.

In some embodiments according to any of the methods described above, the antimetabolite is delivered into the bladder by an intravesicular (intravesical) device. In some of these embodiments according to any of the methods described above, the intravesicular (intravesical) device comprises a housing configured for intravesicular (intravesical) insertion; and a dosage form comprising antimetabolite, wherein the housing holds the dosage form and is configured to release antimetabolite in an amount effective for the treatment of a urothelial carcinoma. In some embodiments according to any of the methods described above, the intravesicular (intravesical) drug delivery device comprises a housing which contains and controllably releases the antimetabolite and is elastically deformable between a retention shape configured to retain the device in the individual's bladder and a deployment shape for passage of the device through the individual's urethra. In some embodiments according to any of the devices described above, the device comprises a drug reservoir lumen bounded by a first wall and a second wall, wherein the first wall is impermeable to the drug and the second wall is permeable to the drug. In some embodiments according to any of the devices described above, the first wall and the second wall are adjacent one another and together form an annular tube defining the drug reservoir lumen. In some embodiments according to any of the devices described above, the second wall is in the form of a strip extending at least a portion of the length of the first wall structure. In some embodiments the first wall is cylindrical. In some embodiments, the second wall is disc-shaped. In some embodiments, the intravesicular (intravesical) drug delivery device comprises at least two drug reservoir lumens.

In some embodiments according to any of the methods described above, when a device is used to deliver the antimetabolite, the antimetabolite is released from the device by osmotic pressure. In some embodiments, the antimetabolite is released from the device by diffusion.

In some embodiments according to any of the devices described above, the antimetabolite contained in the housing is in a non-liquid form. In some of these embodiments, the non-liquid form is selected from the group consisting of tablets, granules, semisolids, powders, capsules, and combinations thereof.

In some embodiments according to any of the methods described above, the method provided herein comprises a method of treating a urothelial carcinoma of the lower tract, wherein the urothelial carcinoma is bladder cancer. In some embodiments, the bladder cancer is locally advanced bladder cancer or metastatic bladder cancer. In some embodiments, the bladder cancer is muscle invasive bladder cancer. In some embodiment, the bladder cancer is non-muscle invasive bladder cancer. In some embodiments according to any of the methods described above, the bladder cancer is carcinoma in situ. In some embodiments, the bladder cancer is BCG (Bacillus Calmette-Guerin) refractory cancer or papillary bladder cancer. In some embodiments, the bladder cancer is BCG unresponsive cancer.

In some embodiments according to any of the methods described above, the method comprises administering to an individual an effective amount of an antimetabolite, wherein the individual is human. In some embodiments according to any of the methods described above, the individual is unfit for systemic therapy. In some embodiments according to any of the methods described above, the individual has a compromised immune system. In some embodiments according to any of the methods described above, the individual has a high level of an immune checkpoint protein. In some embodiments, the individual has a low level of an immune checkpoint protein. In some embodiments according to any of the methods described above, the individual has a high level of a nucleoside transporter. In some embodiments, the individual has a lower level of a nucleoside transporter.

In some embodiments according to any of the methods described above, the antimetabolite is gemcitabine and the method further comprises determining the gemcitabine/metabolite ratio in the urine, wherein a ratio below a threshold value is indicative of effective treatment.

In some embodiments, provided herein are kits for treating a urothelial carcinoma of the lower tract in an induvial comprising: a) an antimetabolite and b) a second agent, wherein the antimetabolite is in a device for local delivery to the bladder. In some of these embodiments, the antimetabolite is gemcitabine. In some embodiments, the second agent is an immunomodulatory agent.

Also provided herein is a device for local delivery of an antimetabolite and a second agent to the bladder of an individual comprising a) an antimetabolite and b) a second agent. In some embodiments, the antimetabolite is gemcitabine. In some embodiments, the second agent is an immunomodulatory agent.

In some embodiments according to any of the devices described above, the intravesicular (intravesical) drug delivery device comprises a housing defining a reservoir; a first unit contained within the reservoir, the first unit comprising an antimetabolite; and a second unit contained within the reservoir in a position distinct from the first unit, wherein the second unit comprises a functional agent that facilitates in vivo release of the drug from the housing. In some embodiments, the functional agent is an osmotic agent, a drug solubilizing agent, or a combination thereof, and the housing includes at least one drug release orifice which is in fluid communication with the reservoir.

### BRIEF DESCRIPTION OF THE DRAWINGS

FIG. 1 shows the tumor size, as determined by net average intensity, in rats treated with 90 µg/ml, 180 µg/ml, or 350 µg/ml gemcitabine compared to control.

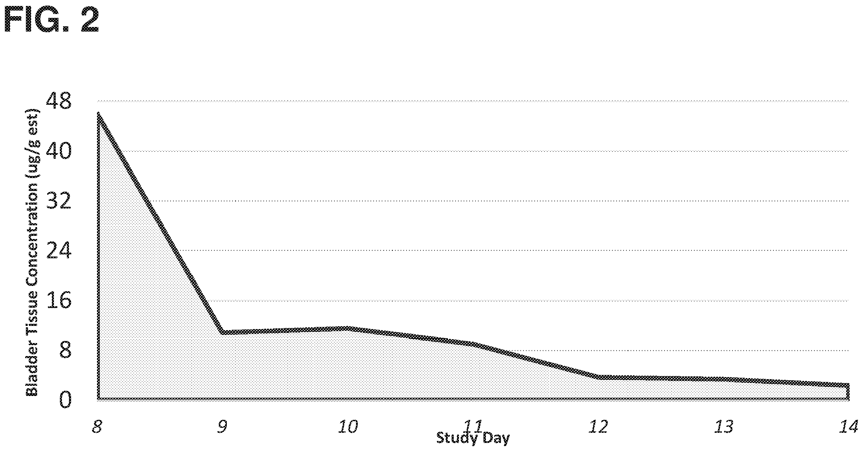

FIG. 2 shows the level of gemcitabine in bladder tissue following continuous delivery of gemcitabine for one week.
FIG. 3 shows the level of gemcitabine in rat bladder tissue layers following continuous delivery of gemcitabine.
FIG. 4 shows the level of activated and regulatory T cells present in the tumor microenvironment after perfusion with 90 of 180 µg/mL gemcitabine.
FIG. 5 shows the percentage of activated CD8+ and CD4+ cells in the spleens of untreated control rats and in rats treated with gemcitabine.
FIG. 6 shows the TGF-β levels in untreated control rats and rats treated with 180 µg/mL gemcitabine.
FIG. 7 shows the IL-10 levels in untreated control rats and rats treated with 180 µg/mL gemcitabine.
FIGS. 8A-8C show an intravesicular (intravesical) device that can be used to provide local and continuous delivery of an antimetabolite. FIG. 8A is a plan view. FIG. 8B is a cross-sectional view taken along line 3-3 in FIG.8A. FIG. 8C is a view of one end portion of the device disposed within the working channel of a deployment instrument, which is shown in partial cross-section.

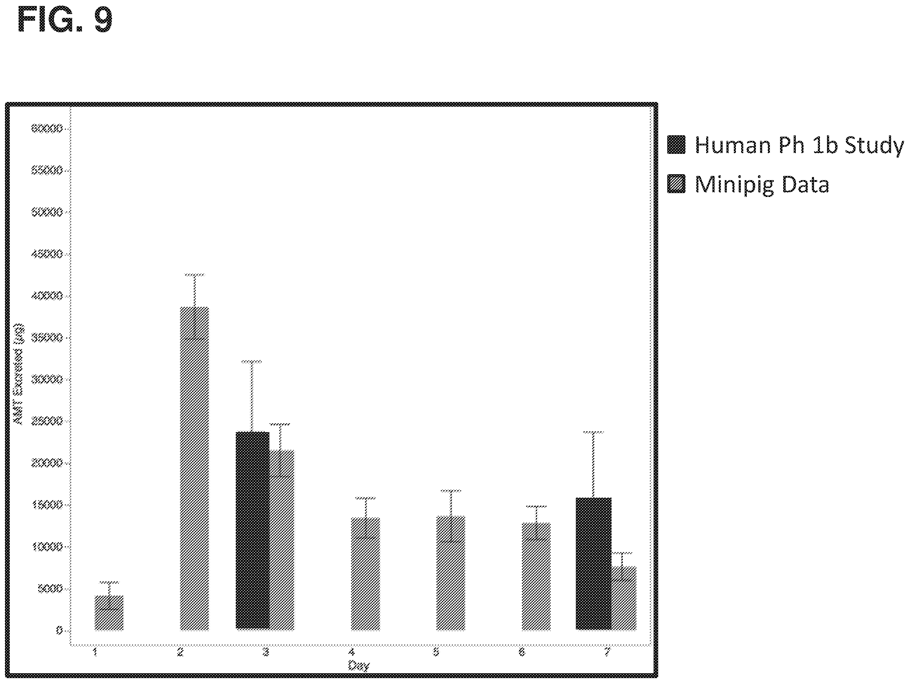

FIG. 9 shows that comparable levels of gemcitabine are excreted in the urine in a minipig study and a human phase IB trial.

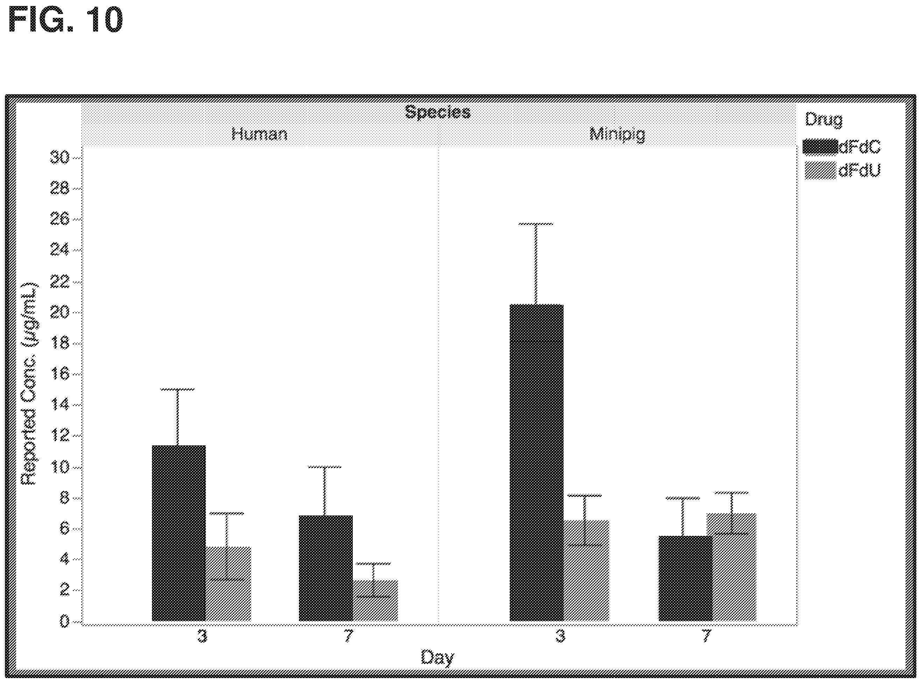

FIG. 10 shows that the urine gemcitabine (dFdC) and metabolite (dFdU) concentrations were comparable in a mini pig study and in a human phase IB study.

### DETAILED DESCRIPTION OF THE INVENTION

The present application provides novel methods for treating a urothelial carcinoma of the lower tract (such as bladder cancer) through local delivery of antimetabolites (such as gemcitabine) to the bladder. The application is based in part on the surprising finding that local delivery of gemcitabine with a specific dosing regimen leads to multiple biological effects, such as modulating immune responses in the tumor microenvironment, inducing systemic immunity and antigen presentation, and inducing cytotoxicity. It was further surprisingly found that gemcitabine, when delivered locally to the bladder with a specific dosing regimen, penetrates deeply into the bladder tissue and persists in the urine for an extended period of time even after the delivery has stopped. Thus, local delivery of antimetabolite to the bladder, as described herein, are useful for treating urothelial carcinoma of the lower tract, particularly when used in combination with a second agent (such as an immunomodulatory agent).

Thus, the present invention in various aspects provide methods of treating a urothelial carcinoma of the lower tract in an individual, enhancing an immune response against a urothelial carcinoma of the lower tract in an individual, reducing recurrence or progression of urothelial carcinoma of the lower tract of an individual, improving tumor microenvironment for cancer immunotherapy, sensitizing an individual having a urothelial carcinoma of the lower tract for radiation therapy comprising administering to the individual an effective amount of an antimetabolite, wherein the antimetabolite is delivered locally to the bladder. In some embodiments, the method further comprises delivery of a second agent such as an immunomodulating agent. In some embodiments, the individual is unsuitable for systemic administration or is immunocompromised.

In another aspect, there are provided kits for treating a urothelial carcinoma of the lower tract in an individual comprising: a) an antimetabolite and b) a second agent, wherein the gemcitabine antimetabolite is in a device for local delivery to the bladder. In another aspect, there are provided devices for local delivery of an antimetabolite and a second agent to the bladder, comprising: a) an antimetabolite and b) a second agent. In some embodiments, the antimetabolite is gemcitabine. In some embodiments, the second agent is an immunomodulatory agent.

### I. Methods of the present invention

The present application in some embodiments provides a method of treating a urothelial carcinoma of the lower tract in an individual comprising delivering to the individual an effective amount of an antimetabolite (such as a nucleoside analog, for example gemcitabine), wherein the antimetabolite (such as a nucleoside analog, for example gemcitabine) is delivered locally to the bladder. In some embodiments, there is provided a method of enhancing an immune response against a urothelial carcinoma of the lower tract in an individual comprising delivering to the individual an effective amount of an antimetabolite (such as a nucleoside analog, for example gemcitabine), wherein the antimetabolite (such as a nucleoside analog, for example gemcitabine) is delivered locally to the bladder. In some embodiments, there is provided a method of reducing recurrence or progression of urothelial carcinoma of the lower tract of an individual comprising delivering to the individual an effective amount of an antimetabolite (such as a nucleoside analog, for example gemcitabine), wherein the antimetabolite (such as a nucleoside analog, for example gemcitabine) is delivered locally to the bladder. In some embodiments, there is provided a method of improving tumor microenvironment for cancer immunotherapy in an individual having a urothelial carcinoma of the lower tract, comprising delivering to the individual an effective amount of an antimetabolite (such as a nucleoside analog, for example gemcitabine), wherein the antimetabolite (such as a nucleoside analog, for example getticitabitie) is delivered locally to the bladder. In some embodiments, the urothelial carcinoma of the lower tract is muscle invasive bladder cancer (MIBC). In some embodiments, the urothelial carcinoma of the lower tract is non-muscle invasive bladder cancer (NMBIC).

The term "continuous" or "continuously" as used herein refers to sustained administration of an antimetabolite (for example gemcitabine) over a period of time, for example over a period from 24 hours to 3 weeks.

The term "individual" as used herein refers to a mammal, including humans. An individual includes, but is not limited to, human, bovine, horse, feline, canine, rodent, or primate. In some embodiments, the individual is human.

Reference to "about" a value or parameter herein includes (and describes) embodiments that are directed to that value or parameter per se. For example, "about 7 days" includes 7 days.

The methods may be practiced in an adjuvant setting. "Adjuvant setting" refers to a clinical setting in which an individual has had a history of a proliferative disease, particularly cancer, and generally (but not necessarily) been responsive to therapy, which includes, but is not limited to, surgery (such as surgical resection), radiotherapy, and chemotherapy. However, because of their history of the proliferative disease (such as cancer), these individuals are considered at risk of development of the disease. Treatment or administration in the "adjuvant setting" refers to a subsequent mode of treatment. The degree of risk (i.e., when an individual in the adjuvant setting is considered as "high risk" or "low risk") depends upon several factors, most usually the extent of disease when first treated. The methods provided herein may also be practiced in a neoadjuvant setting, i.e., the method may be carried out before the primary/definitive therapy. In some embodiments, the individual has previously been treated. In some embodiments, the individual has not previously been treated. In some embodiments, the treatment is a first line therapy.

The term "effective amount" used herein refers to an amount of a compound or composition sufficient to treat a specified disorder, condition or disease such as ameliorate, palliate, lessen, and/or delay one or more of its symptoms. In reference to cancers or other unwanted cell proliferation, an effective amount comprises an amount sufficient to cause a tumor to shrink and/or to decrease the growth rate of the tumor (such as to suppress tumor growth) or to prevent or delay other unwanted cell proliferation. In some embodiments, an effective amount is an amount sufficient to delay development. In some embodiments, an effective amount is an amount sufficient to prevent or delay occurrence and/or recurrence. An effective amount can be administered in one or more administrations, in the case of cancer, the effective amount of the drug or composition may: (i) reduce the number of cancer cells; (ii) reduce tumor size; (iii) inhibit, retard, slow to some extent and preferably stop cancer cell infiltration into peripheral organs; (iv) inhibit (i.e., slow to some extent and preferably stop) tumor metastasis; (v) inhibit tumor growth; (vi) prevent or delay occurrence and/or recurrence of tumor; and/or (vii) relieve to some extent one or more of the symptoms associated with the cancer.

In some embodiments, there is provided a method of decreasing disease progression comprising delivering to the individual an effective amount of an antimetabolite (such as a nucleoside analog, for example gemcitabine), wherein the antimetabolite (such as a nucleoside analog, for example gemcitabine) is delivered locally to the bladder. In some embodiments, there is provided a method of reducing tumor volume comprising delivering to the individual an effective amount of an antimetabolite (such as a nucleoside analog, for example gemcitabine), wherein the antimetabolite (such as a nucleoside analog, for example gemcitabine) is delivered locally to the bladder. In some embodiments, there is provided a method of treating bladder cancer, comprising delivering to the individual an effective amount of an antimetabolite (such as a nucleoside analog, for example gemcitabine), wherein the antimetabolite (such as a nucleoside analog, for example gemcitabine) is delivered locally to the bladder and wherein following treatment, there is no residual exophytic tumor.

In some embodiments, the methods provided herein are useful for improving the quality of life of a patient. For example, the methods provided herein can be used to provide chronic treatment for patients who are unable to undergo cystectomy. In some embodiments, the methods provided herein can be used as a palliative care. In some embodiments, provided herein is a method of reducing pain in an individual having cancer.

In some embodiments, there is provided a method of sensitizing an individual having a urothelial carcinoma of the lower tract for radiation therapy, comprising delivering to the individual an effective amount of an antimetabolite (such as a nucleoside analog, for example gemcitabine), wherein the antimetabolite (such as a nucleoside analog, for example gemcitabine) is delivered locally to the bladder. In some embodiments, the method further comprises subjecting the individual to radiation therapy. In some embodiments, the radiation therapy is carried out after the delivery of the antimetabolite, for example after about any of 1, 2, 3, 4, 5, 10, 15, 20, or 30 days after the delivery of the antimetabolite. Radiation contemplated herein include, for example, X-ray, γ-day, and directed delivery of radioisotopes to tumor cells. Other forms of DNA damaging factors such as microwaves and UV irradiation are also contemplated. The radiation may be given in a single dose or in a series of smaller doses in a dose-fractioned schedule. In some embodiments, the dose of the radiation is lower than conventional doses for radiation therapy. For example, the dose of the radiation may be no more than about any of 95%, 90%, 85%, 80%, 75%, 60%, 50%, 40%, 30%, 20%, or 10% of the conventional dose.

The antimetabolite in some embodiments is continuously delivered into the bladder. For example, in some embodiments, the antimetabolite is continuously delivered to the bladder for at least about 24 hours (such as at least about 2, 3, 4, 5, 6, 7, 8, 9, 10, 15, 20, 25, or 30 days). In some embodiments, the antimetabolite is continuously delivered to the bladder for 7 days. The antimetabolite may be delivered at a single release rate, or at different release rates at different time points. For example, in some embodiments, the antimetabolite is delivered in a first phase of the delivery at a first release rate followed by a second phase of the delivery having a second release rate. In some embodiments, the first release rate is faster (such as at least 2x, 3x, 4x, 5x, or 10x faster) than the second release rate. In some embodiments, the first rate is slower (such as at least 2x, 3x, 4x, 5x, or 10x slower) than the second release rate.

In some embodiments, the antimetabolite is delivered at a dose of from about 1 mg/day to about 300 mg/day, such as any of about 1 mg/day to about 5 mg/day, about 5 mg/day to about 10 mg/day, about 10 mg/day to about 50 mg/day, about 50 mg/day to about 100 mg/day, about 100 mg/day to about 225 mg/day (e.g., about 140 mg, about 160 mg, about 180 mg, about 200 mg, or about 220 mg), about 200 mg/day to about 300 mg/day. In some embodiments, about 100 mg to about 200 mg of gemcitabine is delivered to the individual. In some embodiments, about 160 mg of gemcitabine is delivered to the individual over seven days. In some embodiments, about 100 mg to about 200 mg of gemcitabine is delivered to the individual over 7 days. In some embodiments, about 200 mg to about 225 mg of gemcitabine is delivered to the individual over 21 days. In some embodiment, about 225 mg of antimetabolite is delivered to the individual over 21 days. In some embodiments, 225 mg of gemcitabine is administered to the individual over seven days. In some embodiments, 225 mg of gemcitabine is administered to the individual over 21 days.

In some embodiments, the concentration of the antimetabolite in the urine during the delivery period is about 0.1 µg/mL to about 200 µg/mL, such as about any of 0-0.5, 0.5-1, 1-2, 2-3, 3-4, 4-5, 5-6, 6-7, 7-8, 8-9, 9-10, 10-20, 20-30, 30-40, 40-60, 60-80, 80-100, 100-150, or 150-200 µg/mL. In some embodiments, the antimetabolite concentration in the plasma of the individual is less than about 1 µg/mL, such as less than about any of 0.5, 04, 0.3, 0.2, 0.1, 0.05, 0.04, 0.03, 0.02, or 0.01 µg/mL. In some embodiments, upon delivery of antimetabolite the ratio of antimetabolite in the urine to antimetabolite in the plasma of the individual is greater than about 500:1. In some embodiments, the plasma concentration of dFdU is less than 0.3 µg/mL upon delivery of the antimetabolite. In some embodiments the plasma concentration of dFdU is less than 0.2 µg/mL. upon delivery of the antimetabolite. In some embodiments the plasma concentration of dFdU is less than 0.1 µg/mL upon delivery of the antimetabolite. In some embodiments, the plasma concentration of dFdU is between 0.1 and 0.3 µg/mL upon delivery of the antimetabolite.

In some embodiments, the delivery of the antimetabolite comprises separate antimetabolite delivery periods, with a resting period in between. The dosage or release rate of the antimetabolite during the different delivery periods may be the same or different. For example, in some embodiments, the antimetabolite is delivered over at least a month, wherein each antimetabolite delivery period is at least one day, and wherein the interval between each antimetabolite delivery period is no more than about a week. In some embodiments, the method comprises: a) a first antimetabolite delivery period, wherein the concentration of antimetabolite in the urine of the individual is at least about 0.1 µg/mL; b) a rest period; and c) a second antimetabolite gemcitabine delivery period, wherein the concentration of antimetabolite in the urine of the individual is greater than about 0.1 µg/mL. In some of these embodiments, the first antimetabolite delivery period is seven days, the rest period is fourteen days, and the second antimetabolite delivery period is seven days. In some embodiments, the antimetabolite is delivered on days 1-7 and days 21-28 of a treatment regimen. In some embodiments, the antimetabolite is delivered on days 1-14 and 22-34.

In some embodiments, the method comprises two or more delivery periods that are not separated by a rest period. In some of these embodiments, the first and second delivery periods are both three weeks. In some embodiments, the antimetabolite is delivered over a period of six weeks.

In some embodiments, the antimetabolite persists even after the cessation of the delivery. For example, in some embodiments, the concentration of antimetabolite in the urine is greater than about 0.1 µg/mL (such as greater than about any of 0.2, 0.3, 0.4, 0.5, 0.6, 0.7, 0.8, 0.9, or 1 µg/mL for at least about any of 6, 12, 18, 24, or 36 hours after the cessation of the antimetabolite delivery. In some embodiments the concentration of the antimetabolite is from 0.1 to 1 µg/mL, 0.2 to 0.8 µg/mL, or 0.3 to 0.7 µg/mL for 6 hours to 7 days, for 6 hours to 3 days, or from 6 hours to 24 hours.

Various methods can be used to assess the anti-tumor effects produced by the methods provided herein. For examples, levels of biomarkers such as AKT, CD31, Ki67, and TUNEL can be measured using immunohistochemistry in tumor materials to assess cell death.

The methods described herein are particularly suitable for combination therapy, for example in conjunction with the delivery of a second agent. For example, in some embodiments, there is provided a method of treating a urothelial carcinoma of the lower tract in an individual comprising delivering to the individual a) an effective amount of an antimetabolite (such as a nucleoside analog, for example gemcitabine), and b) an effective amount of a second agent, wherein the antimetabolite (such as a nucleoside analog, for example gemcitabine) is delivered locally to the bladder. In some embodiments, the second agent is delivered at the time the antimetabolite delivery is initiated. In some embodiments, the second agent is delivered before the antimetabolite delivery is initiated. In some embodiments, the second agent is delivered after the antimetabolite delivery is initiated. In some embodiments, the second agent is delivered after the antimetabolite delivery is terminated. The antimetabolite delivery period and the second agent delivery period may or may not overlap with each other.

The antimetabolite and the second agent may be delivered via different routes. For example, in some embodiments the second agent is delivered systematically. In some embodiments, the second agent is delivered locally. In some embodiments, the second agent is delivered systemically in a first phase of the second agent delivery period, followed by a local delivery at a second phase of the second agent delivery period. In some embodiments, the second agent is delivered locally in a first phase of the second agent delivery period, followed by a systemic delivery at a second phase of the second agent delivery period. The first phase of the second agent delivery period and the second phase of the second agent delivery period in some embodiments may be separated, for example by at least about any of 1, 2, 3, 4, 5, 6, or 7 days. In some embodiments, the first phase of the second agent delivery period and the second phase of the second agent delivery period in some embodiments may be separated by at least about any of 1, 2, 3, 4, 5, 6, or 7 weeks.

In some embodiments, the antimetabolite and the second agent are delivered simultaneously, either via the same or different administration routes.

In some embodiments, the antimetabolite and the second agent are delivered via a single delivery device, such as any of the delivery devices described herein. For example, the device may comprise two separate chambers, one for the antimetabolite, the other for the second agent. Alternatively, the antimetabolite and the second agent are present in the same chamber, for example admixed with each other. In some embodiments, the antimetabolite and the second agent are delivered at the same release rate. In some embodiments, the antimetabolite and the second agent are delivered at different release rate. The different release rate can be accomplished, for example, by adopting a specific design of the delivery device to tailor the drug delivery profiles. In some embodiments when the antimetabolite and the second agent are delivered simultaneously (such as via the same delivery device), the method may further comprise administration of an additional dose of the second agent. This can be accomplished, for example, by systemic or local delivery of the second agent.

The second agent can be any therapeutic agent suitable for the methods described herein. In some embodiments, the second agent is a chemotherapeutic agent, for example a chemotherapeutic agent selected form the group consisting of paclitaxel, docetaxel, carboplatin, cisplatin, and oxaliplatin.

In some embodiments, provided herein is a method of treating a urothelial carcinoma of the lower tract comprising administering an antimetabolite (such as gemcitabine) locally to the bladder in combination with BCG. In some of these embodiments, the method comprises delivering gemcitabine locally to the bladder of the individual for 24 hours to 3 weeks and administering BCG. In some embodiments, provided herein do not comprise administering BCG to the individual in combination with gemcitabine.

In some embodiments, the second agent is an immunomodulating agent. For example, in some embodiments, there is provided a method of treating a urothelial carcinoma of the lower tract in an individual comprising administering to the individual a) an effective amount of an antimetabolite (such as a nucleoside analog, for example gemcitabine), and b) an effective amount of an immunomodulating agent, wherein the antimetabolite (such as a nucleoside analog, for example gemcitabine) is delivered locally to the bladder. In some embodiments, the immunomodulating agent is an immune checkpoint inhibitor, including, but not limited to, an inhibitor of an immune checkpoint protein selected from the group consisting of PD-L1, CTLA4, PD-L2, PD-1, B7-H3, B7-H4, HVEM, B- and T-lymphocyte attenuator (BTLA), Killer inhibitory receptor (KIR), GAL9, TIM3, A2AR, LAG-3, phosphatidylserine, CD27, TNF-α, CD33, Siglec-5, Siglec-7, Siglec-9, and Siglec-11. In some embodiments, the immunomodulating agent is agonist of a costimulatory immune molecule, including but not limited to a costimulatory immune molecule selected from the group consisting of CD40, OX40, ICOS, CD28, CD137/4-1BB, CD27, IL-10, TGF-beta, TOR receptor, and glucocorticoid-induced TNFR-related protein GITR.

In some embodiments, the individual does not receive radiation therapy. In some embodiments, the method further comprises radiation therapy. In some embodiments, the antimetabolite is delivered in a neoadjuvant setting. In some embodiments, the antimetabolite is delivered in an adjuvant setting. In some embodiments, the method further comprises a third therapy comprising surgery, and the delivery of antimetabolite to the individual can be initiated at the time of the surgery, prior to the surgery, or after the surgery. In some embodiments, the delivery of antimetabolite to the individual is initiated during a cystoscopy.

The delivery of the antimetabolite (and in some embodiments the second agent) described herein in some embodiments can be carried out using an intravesicular (intravesical) delivery device. Various intravesicular (intravesical) delivery devices are described herein. In some embodiments, the intravesicular (intravesical) device comprises a housing configured for intravesicular (intravesical) insertion; and a dosage form comprising antimetabolite, wherein the housing holds the dosage form and is configured to release antimetabolite in an amount effective for the treatment of a urothelial carcinoma. In some embodiments, the intravesicular (intravesical) drug delivery device comprises a housing which contains and controllably releases the antimetabolite and is elastically deformable between a retention shape configured to retain the device in the individual's bladder and a deployment shape for passage of the device through the individual's urethra. In some embodiments, the device comprises a drug reservoir lumen bounded by a first wall (such as a cylindrical wall) and a second wall (such as a disc shaped wall), wherein the first wall is impermeable to the drug and the second wall is permeable to the antimetabolite. In some other embodiments in which the first wall is impermeable to the antimetabolite and the second wall is permeable to the antimetabolite, the first wall and the second wall are adjacent one another and together form an annular tube defining the drug reservoir lumen. In some of these embodiments, the second wall is in the form of a strip extending at least a portion of the length of the first wall structure. In some embodiments, the device comprises at least two drug reservoir lumens, and in some embodiments each reservoir comprises a different drug contained within.

The antimetabolite may be released from the device by osmotic pressure or by diffusion, depending on the desirable drug release profile. In some embodiments, the antimetabolite contained in the housing is in a non-liquid form, such as a non-liquid form is selected from the group consisting of tablets, granules, semisolids, powders, capsules, and combinations thereof. Various non-liquid forms of drug cores are further described herein.

In some embodiments, the antimetabolite is delivered via passive transport. In some embodiments passive transport is facilitated transport.

In some embodiments, the urothelial carcinoma of the lower tract is bladder cancer. For example, in some embodiments, there is provided a method of treating bladder cancer in an individual comprising administering to the individual a) an effective amount of an antimetabolite (such as a nucleoside analog, for example gemcitabine), and optionally b) an effective amount of a second agent (such as an immunomodulating agent), wherein the antimetabolite (such as a nucleoside analog, for example gemcitabine) is delivered locally to the bladder. In some embodiments, the bladder cancer is locally-advanced bladder cancer or metastatic bladder cancer. In some embodiments, the bladder cancer is muscle invasive bladder cancer. In some embodiments, the bladder cancer is non-muscle invasive bladder cancer. In some embodiments, the bladder cancer is carcinoma in situ. In some embodiments, the bladder cancer is BCG (Bacillus Calmette-Guerin) refractory cancer or papillary bladder cancer.

The individual described herein can be a mammal, preferably a human. In some embodiments, the individual is unsuitable for systemic therapy. In some embodiments, the individual has a compromised immune system. In some embodiments, the individual is resistant to or unsuitable for chemotherapeutic therapy. In some embodiments, the individual is resistant to or unsuitable for other cancer immunotherapy. In some embodiments, the individual has a low neutrophil count. In some embodiments, the individual is ineligible for cisplatin-based combination therapy. In some embodiments, the individual has not received prior radiation therapy to the urinary bladder. In some embodiments, the individual is unwilling or unable to undergo a cystectomy. In some embodiments, the individual may undergo a cystectomy following treatment with the antimetabolite.

In some embodiments, the level of an immune checkpoint protein is used as a basis for selecting an individual for treatment with the methods described herein. In some embodiments, the individual has a high level of an immune checkpoint protein (such as PD-L1). In some embodiments, the individual has a low level of an immune checkpoint protein (such as PD-L1). In some embodiments, the individual is selected for treating based on the level of an immune checkpoint protein (such as PD-L1). In some embodiments, the individual is selected for treating if the individual is determined to have a high level of an immune checkpoint protein (such as PD-L1). In some embodiments, the individual is selected for treatment if the individual has a low level of an immune checkpoint protein (such as PD-L1).

In some embodiments, the level of a nucleoside transporter is used as a basis for selecting an individual for treatment with the methods described herein. In some embodiments, the individual has a high level of a nucleoside transporter (such as hENT1). In some embodiments, the individual has a low level of a nucleoside transporter (such as hENT1). In some embodiments, the individual is selected for treating based on the level of a nucleoside transporter (such as hENT1). In some embodiments, the individual is selected for treating if the individual is determined to have a high level of a nucleoside transporter (such as hENT1). In some embodiments, the individual is selected for treatment if the individual has a low level of a nucleoside transporter (such as hENT1).

The effectiveness of the methods described herein can be assessed by various methods. For example, for methods of treatment, the effectiveness of the method can be evaluated by tumor growth, tumor shrinkage, or survival. In some embodiment, the effectiveness of the method is evaluated based on the level of one or more markers. For example, the effectiveness of the method can be determined based on the levels of TGF-beta or IL-10. In some embodiments, the effectiveness of the methods can be evaluated based on the ratio of the antimetabolite and its metabolic product in the urine. For example, when the antimetabolite is gemcitabine, the effectiveness of the method may be evaluated based on the ratio of gemcitabine and its metabolite (e.g., dFdU) in the urine. For cancer treatment, a ratio below a threshold value may be indicative of effectiveness.

In some embodiments pro-inflammatory cytokine production may be increased and the anti-inflammatory cytokine production may be decreased. The amount of pro-inflammatory cytokines may be increased locally in the bladder or systemically and likewise, the amount of anti-inflammatory cytokines may be decreased locally in the bladder or systemically. For example, in some embodiments, the level IL-10 is increased. In some embodiments, the level of TGFβ is decreased. In some embodiments, the level of interferon gamma (IFN-γ) is increased.

Regulatory T cells, also known as suppressor T cells, or T-regs are an immunosuppressive population of T-cells. In cancer, regulatory T cells are recruited to the tumor micro environment and provide an immunosuppressive tumor microenvironment that suppresses effector T cells and decreases the body's immune response against the tumor. Regulatory T cells carry out their immunosuppressive function by cells secretion of anti-inflammatory cytokines.

Accordingly, in one embodiment, the present methods facilitate treatment of a urothelial carcinoma of the lower tract by decreasing the number, level, or percentage of regulatory T cells upon administration of an antimetabolite (such as a nucleoside analog, for example gemcitabine). One of skill in the art will be aware of various methods to measure the presence of regulatory T cells in a sample, such as immunohistochemically staining for relevant markers or performing flow cytometry or FACS analysis. The level or percentage of regulatory T cells may also be decreased relatively compared to conventional T cells that are CD4+CD25-.

Additionally, or alternatively the activity of regulatory T-cells may be decreased upon delivery of an antimetabolite. Functional assays, such as cytokine release can be used to measure the activity of regulatory T-cells.

The number, level amount, or activity of regulatory T cells may be decreased locally, for example in the bladder, in the tumor microenvironment or the local lymph nodes. The number, level, amount, or activity of regulatory T cells may also be decreased systemically.

Cytotoxic T cells are CD8+ T cells that recognize targets by binding to antigen associated with MHC class I molecules. Cytotoxic T cells recognize and induce apoptosis of infected or damaged cells through the caspase cascade and have been recognized as playing an important role in the body's anti-cancer response. Upon delivery of an antimetabolite, the level, number, and/or activity of cytotoxic T cells may be increased to promote the body's anti-cancer response. For example, the relative level of cytotoxic T cells may be increased compared to suppressive regulatory T-cells, which can be determined by FACS analysis Additionally, or alternatively, the activity of cytotoxic T cells may be increased, which can be measured using functional assays.

Activated effector CD4+ T cells also play a key role in generating an inflammatory response by secreting pro inflammatory cytokines, proteins, or peptides and have been recognized as playing an important role in the body's anti-cancer response. Upon delivery of an antimetabolite, the level, number, and/or activity of effector CD4+ T cells may be increased to promote the body's anti-cancer response. For example, the relative level of effector CD4+ T cells may be increased compared to suppressive regulatory T-cells, which can be determined by FACS analysis Additionally, or alternatively, the activity of effector CD4+ T cell may be increased, which can be measured using functional assays.

In some embodiments, the level of regulatory T cells is decreased compared to the level of both cytotoxic T cells and effector T cells. In some embodiments, the level of regulatory T cell is decreased compared to the level of activated conventional CD4+/CD25-T cells (Tcon).

### Immune Checkpoint Inhibitors

An immunomodulating agent can be an agent which modulates a checkpoint inhibitor. Immune checkpoint proteins signaling proteins that play a role in regulating immune response. Some checkpoint inhibitors are receptors located on the surface of a cell that respond to extracellular signaling. For example, many checkpoints are initiated by ligand-receptor interactions. When activated, checkpoint proteins produce an anti-inflammatory response that can include activation of regulatory T cells and inhibition of cytotoxic or killer T cells. Cancer cells have been shown to express checkpoint proteins as a way to avoid recognition by immune cells. Accordingly, checkpoint inhibitors can be used to activate the immune system in an individual to kill cancer cells. Pardoll, Nature Reviews Cancer 12, 252-264 (2012).

Exemplary checkpoint inhibitors include inhibitors of PD-L1, CTLA4, PD-L2, PD-1, B7-H3, B7-H4, HVEM, B- and T-lymphocyte attenuator (BTLA), Killer inhibitory receptor (KIR), GAL9, TIM3, A2AR, LAG-3, phosphatidylserine, CD27, TNF-α, CD33, Siglec-5, Siglec-7, Siglec-9, and Siglec-1 1.

CTLA-4 signaling inhibits T-cell activation, particularly during strong T-cell responses. CTLA-4 blockade using CTLA-4 inhibitors, such as anti-CTLA-4 monoclonal antibodies, has great appeal because suppression of inhibitory signals results in the generation of an antitumor T-cell response. Both clinical and preclinical data indicate that CTLA-4 blockade results in direct activation of CD4+ and CD8+ effector cells, and anti-CTLA-4 monoclonal antibody therapy has shown promise in a number of cancers, particularly melanoma, Leach et al, Science, 271: 1734-1736 (1996); Wolchok et al, Oncologist, 13: Suppl 4: 2-9 (2008).

Like CTLA-4 signaling, PD-1/PD-L1 modulates T-cell response. Tregs that express PD-1 have been shown to have an immune inhibitor response and PD-1/PD-L1 expression is thus thought to play a role in self-tolerance. In the context of cancer, tumor cells over express PD-1 and PD-L1 in order to evade recognition by the immune system. Anti-cancer therapy that blocks the PD-L1/PD-1 increases effector T cell activity and decreases suppressive Treg activity which allows recognition and destruction of the tumor by an individual's immune system.

Various checkpoint inhibitors may be used. For example, the checkpoint inhibitor may be an antibody that binds to and antagonizes a checkpoint inhibitor protein. Exemplary antibodies include anti-PD1 antibodies (nivolumat), pembrilizomab, pidilizumab), anti-PD-L1 antibodies (atezolizumab, BMS-936559, MPDL-3280A, MEDI7436, AMP224), anti-CTLA4 antibodies (ipilimumab, tremelimumab) and the like. In some embodiments, the checkpoint inhibitor antagonist may be a small molecule or an RNAi that targets a checkpoint inhibitor. In some embodiments, the checkpoint inhibitor may be a peptidomimetic or a polypeptide.

### Immune Costimulatory Molecule Agonists

The immunomodulating agent may also be an immune costimulatory molecule agonist. *Immune* costimulatory molecules are signaling proteins that play a role in regulating immune response. Some immune costimulatory molecules are receptors located on the surface of a cell that respond to extracellular signaling. When activated, immune costimulatory molecules produce a pro-inflammatory response that can include suppression of regulatory T cells and activation of cytotoxic or killer T cells. Accordingly, immune costimulatory molecule agonists can be used to activate the immune system in an individual to kill cancer cells.

Exemplary immune costimulatory molecules include any of CD40, OX40, ICOS, CD28, CD137/4-1BB, CD27, and glucocorticoid-induced TNFR-related protein GITR For example OX40 stimulation suppresses T reg cell function while enhancing effector T cell survival and activity, thereby increasing anti-tumor immunity.

Various immune costimulatory molecule agonists may be used. For example, the immune costimulatory molecule agonist may be an antibody that binds to and activates an immune costimulatory molecule. In some embodiments, the immune costimulatory molecule may be an agonist antibody to CD40, OX30, or GITR. In further embodiments, the immune costimulatory molecule agonist may be a small molecule that targets and activates an immune costimulatory molecule.

### Dosage Regimens

The following section describes various aspects (embodiments) of dosing and treatment regions, any and all of which apply to the methods described herein.

In some embodiments, the invention comprises a method of treating a urothelial carcinoma of the lower tract of an individual comprising delivering an antimetabolite (such as a nucleoside analog, for example gemcitabine) locally to the bladder of the individual. A variety of methods can be used to deliver the antimetabolite. In one embodiment, the drug can be provided by direct instillation of a simple solution into the bladder. For example, a solution of the drug may be pumped into the bladder through a urethral or suprapubic catheter in a continuous or pulsatile manner over the treatment period. In another embodiment, the drug is released from a device or composition deployed in the bladder, wherein the device or composition releases the drug continuously at a rate effective to produce the desired concentration of drug in the urine over a specified treatment period. For example, the drug may be released from an intravesically-inserted device into the urine in bladder and then the drug diffuses from the urine into the bladder. At the end of the treatment period, the device may be retrieved from the bladder, or it may be eliminated by being resorbed, dissolved, excreted, or a combination thereof.

In some embodiments, the antimetabolite (such as a nucleoside analog, for example gemcitabine) is delivered continuously to the bladder. In some embodiments the antimetabolite is delivered continuously into the bladder. In some embodiments the antimetabolite is delivered continuously into the bladder over a period of at least about 6, at least about 12, at least about 18, at least about 24, at least about 36, at least about 48, at least about 60, or at least about 72 hours. In some embodiments, the antimetabolite is delivered to the bladder for at least about 1 day, at least about 2 days, at least about 3 days, at least about 4 days, at least about 5 days, at least about 6 days, at least about 7 days, at least about 8 days, at least about 9 days, at least about 10 days, at least about 11 days, at least about 12 days, at least about 13 days, or at least about 14 days. In some embodiments, the antimetabolite is delivered to the bladder over a period of about 1 day to about 14 days, about 2 days to about 14 days, about 3 days to about 10 days, about 4 days to about 8 days, or about 5 days to about 7 days. In some embodiments, the antimetabolite is delivered to the bladder over a period of about 7 days. In some embodiments, the gemcitabine is delivered to the bladder over a period of about 1 to about 30 days. In some embodiments, the antimetabolite is delivered to the bladder over a period of about 30 days.

In some embodiments, provided herein is a method comprising delivering gemcitabine to the bladder on days 1-7 and 21-28 of a treatment regimen.

In some embodiments, the antimetabolite is delivered continuously into the urine in the bladder.

In some embodiments, an effective amount of antimetabolite is delivered locally to the bladder of an individual. For example, the antimetabolite may be delivered at a dose of about 1 mg/day to about 300 mg/day. In some embodiments, the antimetabolite is delivered at a dose of about 5 mg/day to about 250 mg/day, about 10 mg/day to about 200 mg/day, about 15 mg/day to about 100 mg/day, or about 15 mg/day to about 50 mg/day. In some embodiments, the antimetabolite is delivered at a dose of about 1 mg/day, about 5 mg/day, about 10 mg/day, about 15 mg/day, about 20 mg/day, about 23 mg/day, about 25 mg/day, about 30 mg/day, about 35 mg/day, about 40 mg/day, about 45 mg/day, about 50 mg/day, about 55 mg/day, about 60 mg/day, about 75 mg/day, about 100 mg/day, about 125 mg/day, about 150 mg/day, about 200 mg/day, about 250 mg/day, or about 300 mg/day.

The total amount of antimetabolite delivered to the individual during a delivery period may range from about 50 mg to about 1000 mg, from about 75 mg to about 750 mg, from about 100 mg to about 500 mg, from about 200 mg to about 400 mg, or from about 100 mg to about 200 mg. In some embodiments about 225 mg of antimetabolite is delivered to the individual. In some embodiments, about 100 to about 225 mg of gemcitabine (e.g., about 140 mg, about 160 mg, about 180 mg, about 200 mg, or about 220 mg)of antimetabolite is delivered to the individual over seven days. In some embodiments, about 100 to about 225 mg of gemcitabine (e.g., about 140 mg, about 160 mg, about 180 mg, about 200 mg, or about 220 mg)of antimetabolite is delivered to the individual over three weeks. In some embodiments, about 100 to about 225 mg of gemcitabine (e.g., about 140 mg, about 160 mg, about 180 mg, about 200 mg, or about 220 mg) of gemcitabine is administered to an individual over three weeks. In some embodiments, 225 mg of gemcitabine is administered to an individual for seven days. In some embodiments, 225 mg of gemcitabine is administered to an individual for three weeks.

In some embodiments, the concentration of antimetabolite in the urine is from about 0.1 µg/mL to about 200 µg/mL during the antimetabolite delivery period. In some embodiments, the concentration of antimetabolite in the urine is from about 1.0 µg/mL to about 100 µg/mL, from about 5.0 µg/mL to about 90 µg/mL, from about 10 µg/mL to about 80 µg/mL, from about 20 µg/mL to about 70 µg/mL, or from about 30 µg/mL to about 50 µg/mL. In some embodiments, the concentration of antimetabolite in the urine is about 1.0 µg/mL, about 5 µg/mL, about 10 µg/mL, about 15 µg/mL, about 20 µg/mL, about 25 µg/mL, about 30 µg/mL, about 40µg/mL, about 50 µg/mL, about 60 pg/mL, about 70 µg/mL, about 80 µg/mL, about 90 µg/mL, or about 100 µg/mL.

The concentration of antimetabolite in the urine may vary depending on the amount of urine in the bladder over the course of a bladder voiding cycle. For example, after the contents of the bladder are voided the concentration of antimetabolite in the urine may be from about 50 µg/mL to about 100 µg/mL. On the other hand, just prior to voiding, the concentration of antimetabolite in the urine may be lower, for example from about 0.1 µg/mL to about 10 µg/mL. In some embodiments, the average concentration of antimetabolite over a voiding cycle is about is about 1.0 µg/mL, about 5 µg/mL, about 10 µg/mL, about 15 µg/mL, about 20 µg/mL, about 25 µg/mL, about 30 µg/mL, about 40µg/mL, about 50 µg/mL, about 60 µg/mL, about 70 µg/mL, about 80 µg/mL, about 90 µg/mL, or about 100 µg/mL. In some variations, the maximum concentration of antimetabolite over the voiding cycle is from about 25 µg/mL to about 250 µg/mL, from about 50 µg/mL to about 200 µg/mL, or from about 100 µg/mL to about 200 µg/mL. In some embodiments, the minimum concentration of antimetabolite over the voiding cycle is from about 0.1 µg/mL to about 20 µg/mL, from about 0.1 µg/mL to about 10 µg/mL, or from about 1 µg/mL to about 5 µg/mL.

One advantage of the methods provided herein is that a therapeutically effective amount of antimetabolite persists in the urine after delivery of the antimetabolite is terminated. For example, in some embodiments, the concentration of antimetabolite in the urine is greater than about 1 µg/mL for at least about 1 day, at least about 2 days, at least about 3 days, at least about 4 days, at least about 5 days, at least about 6 days, at least about 7 days, at least about 8 days, at least about 9 days, at least about 10 days, at least about 11 days, at least about 12 days, at least about 13 days, or at least about 14 days after delivery of antimetabolite is terminated. In some embodiments, the concentration of the antimetabolite in the urine is greater than about 1 µg/mL for between 1 day and 14 days, between 1 day to 10 days, between 2 days and 9 days, or between 3 days and 8 days. In some embodiments, the concentration of antimetabolite in the urine is greater than about 5 µg/mL for at least about 1 day, at least about 2 days, at least about 3 days, at least about 4 days, at least about 5 days, at least about 6 days, at least about 7 days, at least about 8 days, at least about 9 days, at least about 10 days, at least about 11 days, at least about 12 days, at least about 13 days, or at least about 14 days after delivery of antimetabolite is terminated. In some embodiments, the concentration of the antimetabolite in the urine is greater than about 5 µg/mL for between 1 day and 14 days, between 1 day to 10 days, between 2 days and 9 days, or between 3 days and 8 days after delivery of antimetabolite is terminated. In some embodiments, the concentration of antimetabolite in the urine is greater than about 10 µg/mL for at least about 1 day, at least about 2 days, at least about 3 days, at least about 4 days, at least about 5 days, at least about 6 days, at least about 7 days, at least about 8 days, at least about 9 days, at least about 10 days, at least about 11 days, at least about 12 days, at least about 13 days, or at least about 14 days after delivery of antimetabolite is terminated. In some embodiments, the concentration of the antimetabolite in the urine is greater than about 1 µg/mL for between 1 day and 14 days, between 1 day to 10 days, between 2 days and 9 days, or between 3 days and 8 days after delivery of antimetabolite is terminated.

Another advantage of the methods provided herein is that the antimetabolite is delivered locally to the bladder of an individual such that a significant amount of antimetabolite is not present in the plasma of the individual. In some embodiments, the concentration of antimetabolite in the plasma of an individual is less than about 5 µg/mL, less than about 3 µg/mL, less than about 1 µg/mL, less than about 0.5 µg/mL, less than about 0.1 µg/mL, less than about 0.001 µg/mL, or less than about 0.0001 µg/mL.

Because the antimetabolite is delivered locally to the bladder, upon delivery of antimetabolite, the concentration of antimetabolite present in the urine of the individual is higher than the plasma, which may be beneficial for reducing side effects of the antimetabolite. For example, local delivery of antimetabolite to the bladder may result in decreased neutropenia, lymphedema, anemia, thrombocytopenia, fatigue, pain, hair loss, reproductive dysfunction, or memory impairment caused by systemic chemotherapy. For example, in some embodiments, the ratio of concentration of antimetabolite in the urine of the individual to the concentration of antimetabolite in the plasma of the individual is greater than about 100: 1, greater than about 200: 1, greater than about 300: 1, greater than about 400: 1, greater than about 500: 1, greater than about 600: 1, greater than about 700: 1, or greater than about 1000: 1.

It may be advantageous to deliver antimetabolite locally to the bladder more than once. For example, in some embodiments, antimetabolite is delivered locally to the bladder of an individual at least twice, at least 3 times, at least 4 times, at least 5 times, or at least 10 times. In some embodiments, antimetabolite is delivered multiple times over a period of at least 2 weeks, at least 3 weeks, at least 4 weeks, at least 5 weeks, at least 6 weeks, or at least 8 weeks. In some embodiments, antimetabolite is delivered multiple times over a period of at least 1 month, at least 2 months, at least 3 months, at least 4 months, at least 5 months, at least 6 months, at least 12 months, or at least 18 months. For example, in some embodiments, antimetabolite is delivered locally to the bladder of an individual twice, 3 times, 4 times, 5 times, or 10 times. In some embodiments, antimetabolite is delivered multiple times over a period one month, one month to 18 months, 2 months to 18 months, 3 months to 18 months, one month to 6 months, or one month to two months. In some embodiments, the antimetabolite is delivered locally to the bladder 4 times. In some of these embodiments, the antimetabolite is delivered locally to the bladder of the individual 4 times, wherein each antimetabolite delivery period is 3 weeks. In some embodiments, the antimetabolite is delivered locally to the bladder of the individual for 12 weeks.

In some embodiments, antimetabolite is delivered multiple times over at least one month, wherein each antimetabolite delivery period is at least one day. In some embodiments, antimetabolite is delivered at least twice over a period of at least one month. In some embodiments, antimetabolite is delivered at least three times over a period of at least one month. In some embodiments, antimetabolite is delivered at least three times over a period of at least one month. In some embodiments, antimetabolite is delivered at least four times over a period of at least two months. In some embodiments, the interval between each delivery period of antimetabolite (rest period) is no more than about 4 weeks, no more than about 3 weeks, no more than about 2 weeks, or no more than about one week. In some embodiments, antimetabolite is delivered over a least one month, wherein each delivery period of antimetabolite is at least one day, and wherein the interval between each delivery period (rest period) is no more than about one week. In some embodiments, the interval between each delivery period (rest period) is 3 to 50 days, 3 to 30 days, 5 to 20 days or 8 to 15 days. In some embodiments the rest period is up to 4 months (for example 1 month, 2, months, 3 months, or 4 months).

In one aspect, periods of antimetabolite delivery may be separated by rest periods, during which no antimetabolite is delivered. For example, in some embodiments, the method comprises a first antimetabolite delivery period; a rest period following the first antimetabolite delivery period; and a second delivery period following the rest period. In some embodiments, the method comprises a first antimetabolite delivery period of 7 days; a rest period of 14 days following the first antimetabolite delivery period; and a second antimetabolite delivery period of 7 days. In some embodiments, the method comprises a first antimetabolite delivery period of 3 weeks followed by a second antimetabolite delivery period of 3 weeks, without a rest period.

Provided herein is a method of treating muscle invasive bladder cancer in an individual comprising delivering gemcitabine locally to the bladder of the individual for 7 days. In some embodiments provided herein is a method of treating muscle invasive bladder cancer in an individual comprising delivering gemcitabine locally and continuously to the bladder of the individual for 7 days. In some embodiments, provided herein is a method of treating muscle invasive bladder cancer in an individual comprising delivering gemcitabine locally and continuously to the bladder of the individual for 7 days, wherein the gemcitabine is delivered by an intravesicular (intravesical) device. In some embodiments, provided herein is a method of treating muscle invasive bladder cancer in an individual comprising (i) placing a gemcitabine releasing intravesicular (intravesical) device into the bladder of the individual, wherein the device remains in the bladder for 7 days and wherein the gemcitabine is continuously delivered to the bladder. In some embodiments, provided herein is a method of treating muscle invasive bladder cancer in an individual comprising (i) placing a gemcitabine releasing intravesicular (intravesical) device into the bladder of the individual, wherein the device remains in the bladder for 7 days and wherein the gemcitabine releasing intravesicular (intravesical) device delivers gemcitabine passively. In some embodiments, provided herein is a method of treating muscle invasive bladder cancer in an individual comprising (i) placing a first gemcitabine releasing intravesicular (intravesical) device into the bladder of the individual on day 0, (ii) removing the first gemcitabine releasing intravesicular (intravesical) device at day 7, (iii) placing a second gemcitabine releasing intravesicular (intravesical) device in the bladder of the individual at day 21, and (iv) removing the second gemcitabine releasing intravesicular (intravesical) device at day 28. In some of these embodiments, the device contains 225 mg of gemcitabine before placement into the bladder.

Also provided herein is a method of treating muscle invasive bladder cancer in an individual comprising delivering about 100-500 mg of gemcitabine locally to the bladder over 7 days. In some embodiments, provided herein is a method of treating muscle invasive bladder cancer in an individual comprising delivering about 100 to about 225 mg of gemcitabine (e.g., about 140 mg, about 160 mg, about 180 mg, about 200 mg, or about 220 mg) locally and continuously to the bladder over 7 days. In some embodiments, provided herein is a method of treating muscle invasive bladder cancer in an individual comprising delivering gemcitabine locally and continuously to the bladder of the individual for 7 days, wherein the gemcitabine is delivered by an intravesicular (intravesical) device.

In some embodiments, provided herein is a method of treating muscle invasive bladder cancer in an individual comprising (i) placing a gemcitabine releasing intravesicular (intravesical) device into the bladder of the individual, wherein the device remains in the bladder for 7 days, and wherein the device contains 225 mg of gemcitabine. In some embodiments, provided herein is a method of treating muscle invasive bladder cancer in an individual comprising (i) placing a first gemcitabine releasing intravesicular (intravesical) device into the bladder of the individual on day 0, wherein the first gemcitabine releasing device contains 225 mg of gemcitabine, (ii) removing the first gemcitabine releasing intravesicular (intravesical) device at day 7, (iii) placing a second gemcitabine releasing intravesicular (intravesical) device in the bladder of the individual at day 21, wherein the second gemcitabine releasing intravesicular (intravesical) device contains 225 mg of gemcitabine, and (iv) removing the second gemcitabine releasing intravesicular (intravesical) device at day 28.

Provided herein is a method of treating muscle invasive bladder cancer in an individual comprising delivering gemcitabine locally to the bladder of the individual for 3 weeks. In some embodiments provided herein is a method of treating muscle invasive bladder cancer in an individual comprising delivering gemcitabine locally and continuously to the bladder of the individual for 3 weeks. In some embodiments, provided herein is a method of treating muscle invasive bladder cancer in an individual comprising delivering gemcitabine locally and continuously to the bladder of the individual for 3 weeks, wherein the gemcitabine is delivered by an intravesicular (intravesical) device.

In some embodiments, provided herein is a method of treating muscle invasive bladder cancer in an individual comprising (i) placing a gemcitabine releasing intravesicular (intravesical) device into the bladder of the individual, wherein the gemcitabine releasing intravesicular (intravesical) device remains in the bladder for 3 weeks, wherein the gemcitabine is continuously delivered to the bladder. In some embodiments, provided herein is a method of treating muscle invasive bladder cancer in an individual comprising (i) placing a gemcitabine releasing intravesicular (intravesical) device into the bladder of the individual, wherein the gemcitabine releasing intravesicular (intravesical) device remains in the bladder for 3 weeks, wherein the gemcitabine releasing intravesicular (intravesical) device delivers gemcitabine passively. In some embodiments, provided herein is a method of treating muscle invasive bladder cancer in an individual comprising (i) placing a first gemcitabine releasing intravesicular (intravesical) device into the bladder of the individual, wherein the first gemcitabine releasing intravesicular (intravesical) device remains in the bladder for 3 weeks (ii) removing the first gemcitabine releasing intravesicular (intravesical) device (iii) placing a second gemcitabine releasing device into the bladder of the individual three weeks after the first gemcitabine releasing intravesicular (intravesical) device is placed in the bladder, wherein the second gemcitabine releasing intravesicular (intravesical) device remains in the bladder for 3 weeks and (iv) removing the second gemcitabine releasing device.

Also provided herein is a method of treating muscle invasive bladder cancer in an individual comprising delivering greater than 225 mg of gemcitabine locally to the bladder over 3 weeks. In some embodiments, provided herein is a method of treating muscle invasive bladder cancer in an individual comprising delivering greater than 225 mg of gemcitabine locally and continuously to the bladder over 3 weeks. In some of these embodiments, about 80% of the gemcitabine is delivered to the bladder in the first week and about 20% of the gemcitabine is delivered to the bladder in the second and third weeks. In some embodiments, provided herein is a method of treating muscle invasive bladder cancer in an individual comprising delivering greater than 225 mg of gemcitabine locally and continuously to the bladder of the individual for 3 weeks, wherein the gemcitabine is delivered by an intravesicular (intravesical) device.

Also provided herein is a method of treating muscle invasive bladder cancer in an individual comprising delivering about 225 mg of gemcitabine locally to the bladder over 3 weeks. In some embodiments, provided herein is a method of treating muscle invasive bladder cancer in an individual comprising delivering about 225 mg of gemcitabine locally and continuously to the bladder over 3 weeks. In some of these embodiments, about 80% of the gemcitabine is delivered to the bladder in the first week and about 20% of the gemcitabine is delivered to the bladder in the second and third weeks. In some embodiments, provided herein is a method of treating muscle invasive bladder cancer in an individual comprising delivering about 225 mg of gemcitabine locally and continuously to the bladder of the individual for 3 weeks, wherein the gemcitabine is delivered by an intravesicular (intravesical) device.

In some embodiments, provided herein is a method of treating muscle invasive bladder cancer in an individual comprising (i) placing a gemcitabine releasing intravesicular (intravesical) device into the bladder of the individual, wherein the device remains in the bladder for three weeks and wherein the device contains 225 mg of gemcitabine. In some embodiments, provided herein is a method of treating muscle invasive bladder cancer in an individual comprising (i) placing a gemcitabine releasing intravesicular (intravesical) device into the bladder of the individual, wherein the device remains in the bladder for three weeks and wherein the gemcitabine releasing intravesicular (intravesical) device delivers about 225 mg gemcitabine passively. In some embodiments, provided herein is a method of treating muscle invasive bladder cancer in an individual comprising (i) placing a first gemcitabine releasing intravesicular (intravesical) device into the bladder of the individual, wherein the first gemcitabine releasing intravesicular (intravesical) device remains in the bladder for 3 weeks, wherein the first gemcitabine releasing intravesicular (intravesical) device contains 225 mg of gemcitabine, (ii) removing the first gemcitabine releasing intravesicular (intravesical) device (iii) placing a second gemcitabine releasing device into the bladder of the individual three weeks after the first gemcitabine releasing intravesicular (intravesical) device is placed in the bladder, wherein the second gemcitabine releasing intravesicular (intravesical) device remains in the bladder for 3 weeks, wherein the second gemcitabine releasing intravesicular (intravesical) device contains 225 mg gemcitabine, and (iv) removing the second gemcitabine releasing device.

In some embodiments, provided herein is a method of treating muscle invasive bladder cancer in an individual comprising (i) placing a gemcitabine releasing intravesicular (intravesical) device into the bladder of the individual, wherein the device remains in the bladder for three weeks and wherein a total of greater than 225 mg of gemcitabine is continuously delivered to the bladder. In some embodiments, provided herein is a method of treating muscle invasive bladder cancer in an individual comprising (i) placing a gemcitabine releasing intravesicular (intravesical) device into the bladder of the individual, wherein the device remains in the bladder for three weeks and wherein the gemcitabine releasing intravesicular (intravesical) device delivers greater than 225 mg gemcitabine passively. In some embodiments, provided herein is a method of treating muscle invasive bladder cancer in an individual comprising (i) placing a first gemcitabine releasing intravesicular (intravesical) device into the bladder of the individual, wherein the first gemcitabine releasing intravesicular (intravesical) device remains in the bladder for 3 weeks, wherein the first gemcitabine releasing intravesicular (intravesical) device delivers greater than 225 mg gemcitabine to the bladder, (ii) removing the first gemcitabine releasing intravesicular (intravesical) device (iii) placing a second gemcitabine releasing device into the bladder of the individual three weeks after the first gemcitabine releasing intravesicular (intravesical) device is placed in the bladder, wherein the second gemcitabine releasing intravesicular (intravesical) device remains in the bladder for 3 weeks, wherein the second gemcitabine releasing intravesicular (intravesical) device delivers greater than 225 mg gemcitabine to the bladder, and (iv) removing the second gemcitabine releasing device.

Provided herein is a method of treating non-muscle invasive bladder cancer in an individual comprising delivering gemcitabine locally to the bladder of the individual for 7 days. In some embodiments provided herein is a method of treating non-muscle invasive bladder cancer in an individual comprising delivering gemcitabine locally and continuously to the bladder of the individual for 7 days. In some embodiments, provided herein is a method of treating non-muscle invasive bladder cancer in an individual comprising delivering gemcitabine locally and continuously to the bladder of the individual for 7 days, wherein the gemcitabine is delivered by an intravesicular (intravesical) device. In some embodiments, provided herein is a method of treating non-muscle invasive bladder cancer in an individual comprising (i) placing a gemcitabine releasing intravesicular (intravesical) device into the bladder of the individual, wherein the device remains in the bladder for 7 days and wherein the gemcitabine is continuously delivered to the bladder. In some embodiments, provided herein is a method of treating non-muscle invasive bladder cancer in an individual comprising (i) placing a first gemcitabine releasing intravesicular (intravesical) device into the bladder of the individual on day 0, (ii) removing the first gemcitabine releasing intravesicular (intravesical) device at day 7, (iii) placing a second gemcitabine releasing intravesicular (intravesical) device in the bladder of the individual at day 21, and (iv) removing the second gemcitabine releasing intravesicular (intravesical) device at day 28.

Also provided herein is a method of treating non-muscle invasive bladder cancer in an individual comprising delivering about 225 mg of gemcitabine locally to the bladder over 7 days. In some embodiments, provided herein is a method of treating non-muscle invasive bladder cancer in an individual comprising delivering about 100 to about 225 mg of gemcitabine (e.g., about 140 mg, about 160 mg, about 180 mg, about 200 mg, or about 220 mg) of gemcitabine locally and continuously to the bladder over 7 days. In some embodiments, provided herein is a method of treating non-muscle invasive bladder cancer in an individual comprising delivering about 100 to about 225 mg of gemcitabine (e.g., about 140 mg, about 160 mg, about 180 mg, about 200 mg, or about 220 mg) of gemcitabine locally and continuously to the bladder of the individual for 7 days, wherein the gemcitabine is delivered by an intravesicular (intravesical) device.

In some embodiments, provided herein is a method of treating non-muscle invasive bladder cancer in an individual comprising (i) placing a gemcitabine releasing intravesicular (intravesical) device into the bladder of the individual, wherein the device remains in the bladder for 7 days and wherein the device contains 225 mg of gemcitabine. In some embodiments, provided herein is a method of treating non-muscle invasive bladder cancer in an individual comprising (i) placing a first gemcitabine releasing intravesicular (intravesical) device into the bladder of the individual on day 0, wherein the first gemcitabine releasing intravesicular (intravesical) device contains 225 mg of gemcitabine, (ii) removing the first gemcitabine releasing intravesicular (intravesical) device at day 7, (iii) placing a second gemcitabine releasing intravesicular (intravesical) device in the bladder of the individual at day 21, wherein the second gemcitabine releasing device contains 225 mg of gemcitabine, and (iv) removing the second gemcitabine releasing intravesicular (intravesical) device at day 28.

In some embodiments, the method comprises a first antimetabolite delivery period, wherein the concentration of antimetabolite in the urine of the individual is greater than about 0. 1 µg/mL; a rest period following the first antimetabolite delivery period; and a second antimetabolite delivery period following the rest period, wherein the concentration of antimetabolite in the urine of the individual is greater than about 0.1 µg/mL. In some embodiments, the method comprises a first antimetabolite delivery period, wherein the concentration of antimetabolite in the urine of the individual is greater than about 5 µg/mL; a rest period following the first antimetabolite delivery period; and a second antimetabolite delivery period following the rest period, wherein the concentration of antimetabolite in the urine of the individual is greater than about 5 µg/mL. In some embodiments, the method comprises a first antimetabolite delivery period, wherein the concentration of antimetabolite in the urine of the individual is greater than about 7 µg/mL; a rest period following the first antimetabolite delivery period; and a second antimetabolite delivery period following the rest period, wherein the concentration of antimetabolite in the urine of the individual is greater than about 7 µg/mL. In some embodiments, the method comprises a first antimetabolite delivery period, wherein the concentration of antimetabolite in the urine of the individual is greater than about 10 µg/mL; a rest period following the first antimetabolite delivery period; and a second antimetabolite delivery period following the rest period, wherein the concentration of antimetabolite in the urine of the individual is greater than about 10 µg/mL. In some embodiments, the method comprises a first antimetabolite delivery period, wherein the concentration of antimetabolite in the urine of the individual is greater than about 15 µg/mL; a rest period following the first antimetabolite delivery period; and a second antimetabolite delivery period following the rest period, wherein the concentration of antimetabolite in the urine of the individual is greater than about 15 µg/mL.

In some embodiments, the method comprises a first antimetabolite delivery period, wherein the concentration of antimetabolite in the urine of the individual is 0.1 to 15 µg/mL; a rest period following the first antimetabolite delivery period; and a second antimetabolite delivery period following the rest period, wherein the concentration of antimetabolite in the urine of the individual is 0.1 to 15 µg/mL. In some embodiments, the method comprises a first antimetabolite delivery period, wherein the concentration of antimetabolite in the urine of the individual is 1 to 15 µg/mL; a rest period following the first antimetabolite delivery period; and a second antimetabolite delivery period following the rest period, wherein the concentration of antimetabolite in the urine of the individual is 1 to 15 µg/mL. In some embodiments, the method comprises a first antimetabolite delivery period, wherein the concentration of antimetabolite in the urine of the individual is 3 to 10 µg/mL; a rest period following the first antimetabolite delivery period; and a second antimetabolite delivery period following the rest period, wherein the concentration of antimetabolite in the urine of the individual is 3 to 10 µg/mL.

The concentration of antimetabolite in the urine of the individual may be elevated during at least a portion of the rest period. For example, the concentration of antimetabolite in the urine may be greater than about 1 µg/mL for at least a portion of the rest period. In some embodiments, the concentration of antimetabolite in the urine of the individual is greater than about 5 µg/mL for at least a portion of the rest period. In some embodiments, the concentration of antimetabolite in the urine of the individual is greater than about 7 µg/mL for at least a portion of the rest period. In some embodiments, the concentration of antimetabolite in the urine of the individual is greater than about 10 µg/mL for at least a portion of the rest period. In some embodiments the concentration of the antimetabolite is 1 µg/mL to 10 µg/mL for at least a portion of the rest period.

In some embodiments, the concentration of antimetabolite in the urine of the individual is greater than about 1 µg/mL for at least ¼ of the rest period. In some embodiments, the concentration of antimetabolite in the urine of the individual is greater than about 5 µg/mL for at least ¼ of the rest period. In some embodiments, the concentration of antimetabolite in the urine of the individual is greater than about 7 µg/mL for at least ¼ of the rest period. In some embodiments, the concentration of antimetabolite in the urine of the individual is greater than about 10 µg/mL for at least ¼ of the rest period. In some embodiments, the concentration of antimetabolite in the urine of the individual is 1 µg/mL to 10 µg/mL for at least ¼ of the rest period.

In some embodiments, the concentration of antimetabolite in the urine of the individual is greater than about 1 µg/mL for at least one half of the rest period. In some embodiments, the concentration of antimetabolite in the urine of the individual is greater than about 5 µg/mL for at least one half of the rest period. In some embodiments, the concentration of antimetabolite in the urine of the individual is greater than about 7 µg/mL for at least one half of the rest period. In some embodiments, the concentration of antimetabolite in the urine of the individual is greater than about 10 µg/mL for at least one half of the rest period. In some embodiments, the concentration of antimetabolite in the urine of the individual is 1 µg/mL to 10 µg/mL for at least one half of the rest period.

In some embodiments, the concentration of antimetabolite in the urine of the individual is greater than about 1 µg/mL for at least ¾ of the rest period. In some embodiments, the concentration of antimetabolite in the urine of the individual is greater than about 5 µg/mL for at least ¾ of the rest period. In some embodiments, the concentration of antimetabolite in the urine of the individual is greater than about 7 µg/mL for at least ¾ of the rest period. In some embodiments, the concentration of antimetabolite in the urine of the individual is greater than about 10 µg/mL for at least ¾ of the rest period. In some embodiments, the concentration of antimetabolite in the urine of the individual is 1 µg/mL to 10 µg/mL for at least ¾ of the rest period.

In some embodiments, the concentration of antimetabolite in the urine of the individual may be elevated for the entire rest period. For example, the concentration of antimetabolite may be greater than about 1 µg/mL for the entire rest period. In some embodiments, the concentration of antimetabolite in the urine of the individual is greater than about 5 µg/m for at the entire rest period. In some embodiments, the concentration of antimetabolite in the urine of the individual is greater than about 7 µg/mL for the entire rest period. In some embodiments, the concentration of antimetabolite in the urine of the individual is greater than about 10 µg/mL for the entire rest period. In some embodiments, the concentration of antimetabolite in the urine of the individual is 1 ug/mL to 10 µg/mL for the entire rest period.

In some embodiments, the method comprises delivering antimetabolite to an individual at least once every three months for about one year. In some embodiments, the method comprises delivering antimetabolite to an individual at least once every three months for about two years. In some embodiments, the method comprises delivering antimetabolite to an individual at least once every three months for about three years. In some embodiments, the method comprises delivering antimetabolite to an individual at least once every six months for about one year. In some embodiments, the method comprises delivering antimetabolite to an individual at least once every six months for about two years. In some embodiments, the method comprises delivering antimetabolite to an individual at least once every six months for about three years.

The rate of release of the antimetabolite may be varied over the course of delivery. For example, in some embodiments, the method comprises delivering the antimetabolite in a first phase of the delivery at a first release rate followed by a second phase of the delivery having a second release rate. In some of these embodiments, the first release rate is faster than the second release rate. In other embodiments, the first release rate is slower than the second release rate. In some embodiments, the method comprises delivering the antimetabolite in a first phase of the delivery at a first dose followed by a second phase of the delivery at a second dose. In some embodiments, the first phase and the second phase are contiguous. In some embodiments, the first and second phases are separated by a resting period.

The second agent may be delivered locally and/or systemically in different delivery phases. For example, in some embodiments, the second agent is delivered systemically in a first phase of the second agent delivery period, followed by a local delivery at a second phase of the second agent delivery period. In some embodiments, the second agent is delivered locally in in a first phase of the second agent delivery period, followed by a systemic delivery at a second phase of the second agent delivery period. In some of these embodiments, the first phase of the second agent delivery period and the second phase of the second delivery period of the second agent delivery period are separated by at least about a month.

The antimetabolite and second agent may be delivered simultaneously or sequentially. In some embodiments, the antimetabolite and second agent are delivered via a single delivery device.

It may be advantageous for the antimetabolite and second agent to be delivered at the same release rates to provide a synergistic effect. In other embodiments, the antimetabolite and second agent may be delivered at different release rates. For example, the antimetabolite may be delivered at a rate that is slower than the rate of delivery second agent, or the antimetabolite may be delivered at a rate that is faster than the rate of delivery second agent.

In some embodiments, the method comprises delivery of antimetabolite and delivery of a second agent such as immunomodulating agent. Various doses of immunomodulating agents may be used, depending on the particular immunomodulating agent employed. The amount of immunomodulating agent delivered to the individual may be based upon the approved dosage for the immunomodulating agent as a single agent therapy, or it may be higher or lower than the dose of immunomodulating agent delivered when used as a single agent therapy. For example, the immunomodulating agent may be delivered at a dose of about 1, about 2, about 3, about 4, about 5, about 6, about 7, about 8, about 9, about 10, about 15, or about 20 mg/kg

In some embodiments, the immunomodulating agent may comprise the anti-PD1 antibody nivolumab and may be delivered at a dose of about 3 mg/kg. In some embodiments, the nivolumab may be delivered at a dose of 3 mg/kg every two weeks. In some embodiments, the nivolumab may be delivered in combination with ipilimumab. In some of these embodiments, the nivolumab may be delivered at a dose of 1 mg/kg followed by ipilimumab on the same day, every 3 weeks for 4 doses, followed by nivolumab at a dose of 3 mg/kg every 2 weeks. In some embodiments, the immunomodulating agent may comprise the anti-PD1 antibody pembrolizumab and may be administered at a dose of 2 mg/kg every 3 weeks.

In some embodiments, the second agent may be delivered prior to initiation of antimetabolite delivery. In some embodiments, the second agent may be delivered after termination of antimetabolite delivery. In some embodiments, the second agent may be administered during an antimetabolite delivery period. In some embodiments, the antimetabolite delivery period and the second agent delivery period overlap with each other. In some embodiments, the antimetabolite delivery period and the second agent delivery period are nonoverlapping.

In some embodiments, the second agent may be delivered during a rest period. In some embodiments, the second agent may be delivered during a first antimetabolite delivery period or a second antimetabolite delivery period.

In some embodiments, the methods provided herein comprise delivering an effective amount of antimetabolite to an individual for a period of at least about 24 hours and delivering an effective amount of a second agent. In some embodiments, the methods provided herein comprise delivering an effective amount of antimetabolite to an individual for a period of at least about 48 hours and delivering an effective amount of a second agent. In some embodiments, the methods provided herein comprise delivering an effective amount of antimetabolite to an individual for a period of at least about 60 hours and administering an effective amount of a second agent. In some embodiments, the methods provided herein comprise delivering an effective amount of antimetabolite to an individual for a period of at least about 72 hours and delivering an effective amount of a second agent. In some embodiments, the methods provided herein comprise delivering an effective amount of antimetabolite to an individual for a period of at least about 7 days and delivering an effective amount of a second agent.

In some embodiments, the methods provided herein comprise delivering an effective amount of antimetabolite to an individual for a period of between 24 hours and about one month and delivering an effective amount of a second agent to the individual. In some embodiments, the methods provided herein comprise delivering an effective amount of antimetabolite to an individual for a period of between about 24 hours and about 21 days and delivering an effective amount of a second agent to the individual. In some embodiments, the methods provided herein comprise delivering an effective amount of antimetabolite to an individual for a period of between about 24 hours and about 14 days and delivering an effective amount of a second agent to the individual. In some embodiments, the methods provided herein comprise delivering an effective amount of antimetabolite to an individual for a period of between about 24 hours to about 7 days and delivering an effective amount of a second agent to the individual.

In some embodiments, the methods provided herein comprise delivering an effective amount of an antimetabolite to an individual for about 7 days and delivering an effective amount of a second agent to the individual. In some embodiments, the methods provided herein comprise delivering an effective amount of an antimetabolite to an individual for about three weeks and delivering an effective amount of a second agent to the individual. In some embodiments, the methods provided herein comprise delivering an effective amount of an antimetabolite to an individual for about six weeks and delivering an effective amount of a second agent to the individual.

In some embodiments, the methods provided herein comprise delivering an effective amount of antimetabolite to an individual for a period of between 24 hours and about one month and delivering an effective amount of a second agent to the individual wherein the concentration of antimetabolite in the urine of the individual is less than about 1 µg/mL when the second agent is delivered. In some embodiments, the methods provided herein comprise delivering an effective amount of antimetabolite to an individual for a period of between about 24 hours and about 21 days and delivering an effective amount of a second agent to the individual wherein the concentration of antimetabolite in the urine of the individual is less than about 1 µg/mL when the second agent is delivered. In some embodiments, the methods provided herein comprise delivering an effective amount of antimetabolite to an individual for a period of between about 24 hours and about 14 days and administering an effective amount of a second agent to the individual wherein the concentration of antimetabolite in the urine of the individual is less than about 1 µg/mL when the immunomodulating agent is delivered. In some embodiments, the methods provided herein comprise delivering an effective amount of antimetabolite to an individual for a period of between about 24 hours to about 7 days and delivering an effective amount of a second agent to the individual wherein the concentration of antimetabolite in the urine of the individual is less than about 1 µg/mL when the second agent is delivered.

In some embodiments, the methods provided herein comprise delivering an effective amount of antimetabolite to an individual for a period of between 24 hours and about one month and administering an effective amount of a second agent to the individual wherein the concentration of antimetabolite in the urine of the individual is less than about 5 µg/mL when the second agent is delivered. In some embodiments, the methods provided herein comprise delivering an effective amount of antimetabolite to an individual for a period of between about 24 hours and about 21 days and delivering an effective amount of a second agent to the individual wherein the concentration of antimetabolite in the urine of the individual is less than about 5 µg/mL when the second agent is delivered. In some embodiments, the methods provided herein comprise delivering an effective amount of antimetabolite to an individual for a period of between about 24 hours and about 14 days and administering an effective amount of a second agent to the individual wherein the concentration of antimetabolite in the urine of the individual is less than about 5 µg/mL when the second agent is delivered. In some embodiments, the methods provided herein comprise delivering an effective amount of antimetabolite to an individual for a period of between about 24 hours to about 7 days and administering an effective amount of a second agent to the individual wherein the concentration of antimetabolite in the urine of the individual is less than about 5 µg/mL when the second agent is delivered.

In some embodiments, the methods provided herein comprise delivering an effective amount of antimetabolite to an individual for a period of between 24 hours and about one month and delivering an effective amount of a second agent to the individual wherein the concentration of antimetabolite in the urine of the individual is less than about 15 µg/mL when the second agent is delivered. In some embodiments, the methods provided herein comprise delivering an effective amount of antimetabolite to an individual for a period of between about 24 hours and about 21 days and delivering an effective amount of a second agent to the individual wherein the concentration of antimetabolite in the urine of the individual is less than about 15 µg/mL when the second agent is delivered. In some embodiments, the methods provided herein comprise delivering an effective amount of antimetabolite to an individual for a period of between about 24 hours and about 14 days and delivering an effective amount of a second agent to the individual wherein the concentration of antimetabolite in the urine of the individual is less than about 15 µg/mL when the second agent is delivered. In some embodiments, the methods provided herein comprise delivering an effective amount of antimetabolite to an individual for a period of between about 24 hours to about 7 days and delivering an effective amount of a second agent to the individual wherein the concentration of antimetabolite in the urine of the individual is less than about 15 µg/mL when the second agent is delivered.

In some embodiments, the methods provided herein comprise delivering an effective amount of antimetabolite to an individual for a period of between 24 hours and about one month and delivering an effective amount of a second agent to the individual wherein the concentration of antimetabolite in the urine of the individual is greater than about 1 µg/mL when the second agent is delivered. In some embodiments, the methods provided herein comprise delivering an effective amount of antimetabolite to an individual for a period of between about 24 hours and about 21 days and delivering an effective amount of a second agent to the individual wherein the concentration of antimetabolite in the urine of the individual is greater than about 1 µg/mL when the second agent is delivered. In some embodiments, the methods provided herein comprise delivering an effective amount of antimetabolite to an individual for a period of between about 24 hours and about 14 days and delivering an effective amount of a second agent to the individual wherein the concentration of antimetabolite in the urine of the individual is greater than about 1 µg/mL when the second agent is delivered. In some embodiments, the methods provided herein comprise delivering an effective amount of antimetabolite to an individual for a period of between about 24 hours to about 7 days and delivering an effective amount of a second agent to the individual wherein the concentration of antimetabolite in the urine of the individual is greater than about 1 µg/mL when the second agent is delivered.

In some embodiments, the methods provided herein comprise delivering an effective amount of antimetabolite to an individual for a period of between 24 hours and about one month and delivering an effective amount of a second agent to the individual wherein the concentration of antimetabolite in the urine of the individual is greater than about 5 µg/mL when the second agent is delivered. In some embodiments, the methods provided herein comprise delivering an effective amount of antimetabolite to an individual for a period of between about 24 hours and about 2 1 days and delivering an effective amount of a second agent to the individual wherein the concentration of antimetabolite in the urine of the individual is greater than about 5 µg/mL when the second agent is delivered. In some embodiments, the methods provided herein comprise delivering an effective amount of antimetabolite to an individual for a period of between about 24 hours and about 14 days and delivering an effective amount of a second agent to the individual wherein the concentration of antimetabolite in the urine of the individual is greater than about 5 µg/mL when the second agent is delivered. In some embodiments, the methods provided herein comprise delivering an effective amount of antimetabolite to an individual for a period of between about 24 hours to about 7 days and delivering an effective amount of a second agent to the individual wherein the concentration of antimetabolite in the urine of the individual is greater than about 5 µg/mL when the second agent is delivered.

In some embodiments, the methods provided herein comprise delivering an effective amount of antimetabolite to an individual for a period of between 24 hours and about one month and delivering an effective amount of a second agent to the individual wherein the concentration of antimetabolite in the urine of the individual is greater than about 15 µg/mL when the second agent is delivered. In some embodiments, the methods provided herein comprise delivering an effective amount of antimetabolite to an individual for a period of between about 24 hours and about 21 days and delivering an effective amount of a second agent to the individual wherein the concentration of antimetabolite in the urine of the individual is greater than about 15 µg/mL when the second agent is delivered. In some embodiments, the methods provided herein comprise delivering an effective amount of antimetabolite to an individual for a period of between about 24 hours and about 14 days and delivering an effective amount of a second agent to the individual wherein the concentration of antimetabolite in the urine of the individual is greater than about 15 µg/mL when the second agent is delivered. In some embodiments, the methods provided herein comprise delivering an effective amount of antimetabolite to an individual for a period of between about 24 hours to about 7 days and delivering an effective amount of a second agent to the individual wherein the concentration of antimetabolite in the urine of the individual is greater than about 15 µg/mL when the second agent is delivered.

The second agent may be delivered locally to the bladder of the individual, or systemically. For example, the second agent may be delivered intravesicularly via an intravesicular (intravesical) device, bladder pump, catheter, or injection. The second agent may also be delivered orally, parenterally, or intravenously.

In some embodiments, the second agent is delivered systemically followed by local delivery of antimetabolite. In some embodiments, the second agent is first delivered systemically and then delivered locally. In some embodiments, the second agent is first administered locally and then administered systemically.

Antimetabolites include cytidine analogs, uracil analogs, purine analogs, deoxyadenosine analogs, deoxycytosine analogs, guanosine analogs, deoxyguanasine analogs, thymidine analogs, and deoxyuradine analogs. Exemplary nucleoside analogs include azalitadine, decitabine, cytanabine, gemcitabine, 5-fluorouracil, capcitabine, azathiopurine, mercaptopurine, thioguanine, fludarabine, vidarabine, cytarabine, lamivudine, zalcitabine, abacavir, acyclovir, entecavir, stavudine, telbivudine, zidovudine, idoxuridine, and trifluridine.

In another aspect, the methods provided herein may comprise delivery of antimetabolite, an immunomodulating agent, and an additional therapeutic agent. In some embodiments, the additional agent is Bacillus Calmette-Guerin (BCG). The additional therapeutic agent may also be a checkpoint inhibitor antagonist or an immune costimulatory molecule as described herein. For example, the additional therapeutic agent may be an anti-PD-1 antibody, an anti-PD-L1 antibody, or an anti-CTLA-4 antibody

The second or additional agent may also comprise an additional chemotherapeutic agent, such as oxaliplatin, cisplatin docetaxel, carboplatin, docetaxel, paclitaxel, an anti-VEGF antibody, antimetabolites (including nucleoside analogs), platinum-based agents, alkylating agents, tyrosine kinase inhibitors, anthracycline antibiotics, vinca alkloids, transitional metal complexes, proteasome inhibitors, macrolides, and topoisomerase inhibitors. In some embodiments, the chemotherapeutic agent is a platinum-based agent, such as carboplatin, rapamycin or its derivatives, and geldanamycin or its derivatives (such as 17-allyl amino geldanamycin (17-AAG)), retinoids, agents that disrupt microtubule formation (such as colchicines and its derivatives), anti-angiogenic agents, therapeutic antibodies, EGFR targeting agents.

In some embodiments, the methods provided herein comprise administering antimetabolite (such as gemcitabine) locally to the bladder in conjunction with radiation therapy or surgery. For example, antimetabolite (such as gemcitabine) delivery may be initiated in an adjuvant setting prior to surgery or a neoadjuvant setting following surgery. In some embodiments, antimetabolite (such as gemcitabine) delivery may be initiated at the time of surgery. In some embodiments, antimetabolite (such as gemcitabine) delivery may be initiated prior to radiation therapy or after radiation therapy. In some embodiments, the individual receiving treatment with antimetabolite (such as gemcitabine) does not receive radiation therapy.

In some embodiments, antimetabolite (such as gemcitabine) delivery may be administered during a cystoscopy.

In some embodiments, the present methods delay the need for a cystectomy. In some embodiments, the present methods may be used to extend the therapeutic window prior to cystectomy.

In some embodiments, the cancer is resected prior to administration of the antimetabolite (such as gemcitabine). In some embodiments, the individual undergoes a TURBT prior to administration of the antimetabolite (such as gemcitabine) to the bladder. In some embodiments the tumor is maximally resected prior to administration of the gemcitabine such that no visible tumor is present. In some embodiments, the patient is T0 after TURBT. In some embodiments, provided herein is a method of treating muscle invasive bladder cancer in an individual comprising a) resecting the tumor and b) administering gemcitabine to the bladder locally for at least 24 hours. In some embodiments, provided herein is a method of treating non-muscle invasive bladder cancer in an individual comprising a) resecting the tumor and b) administering gemcitabine to the bladder locally for at least 24 hours.

### Patient Populations

The methods provided herein are useful for treatment of a range of individuals having urothelial carcinomas. For example in some embodiments, the urothelial carcinoma is a bladder cancer. In some embodiments, the bladder cancer is locally-advanced bladder cancer. In some embodiments, the bladder cancer is a metastatic bladder cancer. In some embodiments, the bladder cancer is muscle invasive bladder cancer. In some embodiments, the bladder cancer is non-muscle invasive bladder cancer. In some embodiments, bladder cancer is carcinoma in situ. In some embodiments the bladder cancer is BCG (Bacillus Calmette-Guerin) refractory cancer. In some embodiments, the bladder cancer is papillary bladder cancer. In some embodiments, the bladder cancer is grade 1/3, 2/3, or 3/3. In some embodiments, the bladder cancer is stage I, stage II, stage III, or stage IV bladder cancer. In some embodiments, the bladder cancer is high grade invasive papillary urothelial carcinoma. In some embodiments, the bladder cancer is non-invasive high grade urothelial carcinoma. In some embodiments, the bladder cancer is multifocal invasive high grade papillary urothelial carcinoma. In some embodiments the bladder cancer is cT2 or cT3. In some embodiments, the bladder is cT2 with carcinoma in situ.

In some embodiments, the methods provided herein include treatment of individuals who have compromised immune systems. In some embodiments, the methods provided herein comprise treatment of an individual with a low level of a checkpoint inhibitor. For example, an induvial with a low expression of PD-L1 and/or PD-1. In some embodiments, the methods provided herein include treatment of patients with low, medium, or high level of a nucleoside transporter. In some of these embodiments, the nucleoside transporter is hENT. In some embodiments, the methods provided herein include treatment of individuals with cytopenia. In some embodiments, the methods provided herein include treatment of individuals who have previously undergone chemotherapy. In some embodiments, the methods provided herein include treatment of individuals who are ineligible for immunomodulatory therapy.

In some embodiments, provided herein is a method of treating bladder cancers, (for example MIBC) in an individual who is not eligible for neoadjuvant cisplatin-based therapy comprising administering an antimetabolite (such as gemcitabine) locally to the bladder. In some embodiments, provided herein is a method of treating bladder cancers, (for example MIBC) in an individual who refuses neoadjuvant cisplatin-based therapy comprising administering an antimetabolite (such as gemcitabine) locally to the bladder. In some embodiments, provided herein is a method of treating bladder cancers, (for example MIBC) in an individual having cT2 disease and an absence of high-risk features such as lymphovascular invasion (LVI), hydronephrosis, and concomitant carcinoma in situ (CIS) comprising administering an antimetabolite (such as gemcitabine) locally to the bladder. In some embodiments, provided herein is a method of treating bladder cancers, (for example MIBC) in an individual who will receive radical cystectomy but is ineligible for cisplatin-based neoadjuvant therapy comprising administering an antimetabolite (such as gemcitabine) locally to the bladder. In some of these embodiments, the individual has cT2 cancer. In some of embodiments, the individual has cT2 muscle invasive bladder cancer. In some embodiments, the antimetabolite is delivered locally to the bladder of the individual for at least 24 hours. In some embodiments, the antimetabolite (such as gemcitabine) is delivered locally to the bladder of the individual for 7 days. In some of these embodiments, 225 mg of gemcitabine is delivered locally to the bladder over 7 days. In some of these embodiments, the method comprises delivering 225 mg of gemcitabine locally to the bladder for 7 days, followed by a 14 day rest period, followed by delivering 225 mg of gemcitabine locally to the bladder for 7 days. In some embodiments, the method comprises delivering an antimetabolite (such as gemcitabine) locally to the bladder for 3 weeks. In some embodiments, the method comprises delivering 225 mg of gemcitabine locally to the bladder over 3 weeks. In some embodiments, the method comprises delivering 450 mg of gemcitabine locally to the bladder over 6 weeks. In some embodiments, the antimetabolite is gemcitabine.

In some embodiments, an individual is ineligible for cisplatin-based therapy based upon co-morbidities including poor performance status, poor renal function, hearing loss, peripheral neuropathy, and cardiac disease. In some embodiments, an individual is ineligible for cisplatin-based therapy based upon the absence of one or more high-risk features such as lymphovascular invasion (LVI), hydronephrosis, and concomitant carcinoma in situ (CIS).

Up until now, neoadjuvant therapy followed by radical cystectomy, or removal of the bladder has been standard therapy for treatment of muscle invasive bladder cancer. Individuals who are unfit for radical cystectomy undergo palliative transurethral resection of bladder tumors (TURBT) to attempt to limit local advancement of untreated and undertreated disease. Such treatments may temporarily manage local symptoms of hematuria, pain and urgency, but are not employed with curative intent. Therefore, in one aspect the present invention provides a method of treating bladder cancers, (for example MIBC) in an individual who is unfit or not eligible for a cystectomy by administering an antimetabolite (such as gemcitabine) locally to the bladder.

In some embodiments, provided herein is a method of treating bladder cancers, (for example MIBC) of the lower tract in an individual who is unfit for cystectomy comprising delivering an antimetabolite (such as gemcitabine) locally to the bladder. In some embodiments, provided herein is a method of treating bladder cancers, (for example MIBC) in an individual who is ineligible for cystectomy comprising administering an antimetabolite (such as gemcitabine)locally to the bladder. In some embodiments, provided herein is a method of treating bladder cancers, (for example MIBC) in an individual who is frail comprising delivering an antimetabolite (such as gemcitabine)locally to the bladder. In some embodiments, provided herein is a method of treating bladder cancers, (for example MIBC) in an individual who cannot tolerate radical cystectomy comprising delivering an antimetabolite (such as gemcitabine) locally to the bladder. In some embodiments, provided herein is a method of treating bladder cancers, (for example MIBC) in an individual without removing the bladder of the individual, comprising delivering an antimetabolite (such as gemcitabine) locally to the bladder. In some embodiments, provided herein is a method of treating bladder cancers, (for example MIBC) in an individual, wherein the individual is unfit or ineligible for a cystectomy, comprising delivering an antimetabolite (such as gemcitabine) locally to the bladder. In some embodiments, provided herein is a method of treating bladder cancer (for example MMIBC) in an individual, wherein the bladder cancer is metastatic bladder cancer. In some embodiments, provided herein is a method of treating bladder cancers, (for example MIBC) in an individual, wherein the individual has cT2-cT3 disease, comprising delivering an antimetabolite (such as gemcitabine) locally to the bladder. Also provided herein is a method of treating bladder cancers, (for example MIBC) in an individual who is unfit or not eligible for a cystectomy comprising a) resecting the tumor and b) delivering gemcitabine locally to the bladder. In some embodiments, provided herein is a method of treating muscle invasive bladder cancer in an individual who is unfit or not eligible for a cystectomy comprising a) performing a TURBT and b) delivering gemcitabine locally to the bladder for at least 7 days. In some embodiments, provided herein is a method of treating muscle invasive bladder cancer in an individual who is unfit or not eligible for a cystectomy comprising a) performing a TURBT and b) delivering gemcitabine locally to the bladder for 7 days to 180 days, 7 days to 90 days, 7 days to 60 days, 7 days to 30 days, 7 days to 21 days, or 7 days to 14 days. In some of these embodiments, the gemcitabine may be delivered continuously to the bladder chronically, or for the lifetime of the individual to improve the quality of life of the individual. In some embodiments, the antimetabolite (such as gemcitabine) is delivered locally to the bladder of the individual for at least 24 hours. In some embodiments, the antimetabolite (such as gemcitabine) is delivered locally to the bladder of the individual for 24 hours to three weeks, 2 to 20 days, 3 to 16 days, or 4 to 14 days. In some embodiments, the antimetabolite (such as gemcitabine) is delivered locally to the bladder of the individual for 7 days. In some of these embodiments, 225 mg of gemcitabine is delivered locally to the bladder over a period of 7 days. In some of these embodiments, the method comprises delivering 225 mg of gemcitabine locally to the bladder for 7 days, followed by a 14 day rest period, followed by delivering 225 mg of gemcitabine locally to the bladder for 7 days. In some embodiments, the method comprises delivering an antimetabolite locally to the bladder for 3 weeks. In some embodiments, the method comprises delivering 225 mg of gemcitabine locally to the bladder over a period of 3 weeks. In some embodiments, the method comprises delivering 450 mg of gemcitabine locally to the bladder over 6 weeks. In some embodiments, the antimetabolite is gemcitabine.

In some embodiments, the individual is ineligible for radical cystectomy under the National Comprehensive Cancer Network (NCCN) guidelines. For example, the individual may be unfit for curative therapy due to frailty. Prior to the present methods, such individuals typically received palliative radiation without chemotherapy (3.5 Gy/fraction - 10 treatments; or 7Gy/fraction - 7 treatments; TURBT; or no treatment). In some embodiments the individual is unfit for platinum-based chemotherapy. In some embodiments, chemotherapy prior to radiation therapy is not recommended for the individual. In some embodiments, the individual does not receive curative therapy or systemic chemotherapy. In some embodiments, the individual has cT2-cT3 disease.

In some embodiments, the individual cannot tolerate radical cystectomy based upon the American Society of Anesthesiology (ASA) guidelines. For example the individual who cannot tolerate radial cystectomy may be deemed medically unfit for surgery requiring general or epidural anesthesia.

In other embodiments, the individual may lack operative post-operative care infrastructure or personal as determined by the Comprehensive Geriatric Assessment provided by the American Society of Anesthesiologists. Under these guidelines, an individual is deemed frail if he or she shows abnormal independent activities of daily living, severe malnutrition, cognitive impairment, or comorbidities cumulative illness rating scale for geriatrics (CISR-G) grades 3-4.

The methods of the present invention also provide important and significant treatment benefits compared to standard therapeutic regimens that call for removal of the bladder. The present invention also has the advantage of being useful as a bladder sparing protocol for individuals who are eligible for a cystectomy, but elect not to have a cystectomy. The present methods result in a greatly improved quality of life for individuals, who may be able to retain their bladder after having bladder cancer, compared to the presently available treatments. Accordingly, in some embodiments, there is provided herein is a bladder sparing method of treating bladder cancers, (for example MIBC) in an individual comprising delivering an antimetabolite (such as gemcitabine) locally to the bladder. In some embodiments, provided herein is a method of treating bladder cancers (for example MIBC) without removing the bladder of the individual comprising delivering an antimetabolite (such as gemcitabine) locally to the bladder. Also provided herein is a method of treating bladder cancers (for example MIBC), in an individual who would otherwise undergo a cystectomy comprising delivering an antimetabolite (such as gemcitabine) locally to the bladder. In some embodiments, provided herein is a method of treating bladder cancers (for example MIBC), in an individual who is eligible for, but elects not to receive, a cystectomy, comprising delivering an antimetabolite (such as gemcitabine) locally to the bladder. In some embodiments, provided herein is a method of bladder preservation as an alternative to radical cystectomy, comprising delivering an antimetabolite (such as gemcitabine) locally to the bladder. In some embodiments, provided herein is a method of treating bladder cancers (for example MIBC), in an individual who elects not to undergo a cystectomy, comprising delivering an antimetabolite (such as gemcitabine) locally to the bladder. In some embodiments, provided herein is a method of preserving the bladder of an individual, comprising delivering an antimetabolite (such as gemcitabine) locally to the bladder. In some embodiments, provided herein is a method of treating bladder cancers (for example MIBC), in an individual, without removing the bladder, comprising delivering an antimetabolite (such as gemcitabine) locally to the bladder. In some embodiments, provided herein is a method of treating a CT2 urothelial carcinoma in an individual who would otherwise receive a cystectomy, comprising delivering an antimetabolite (such as gemcitabine) locally to the bladder of the individual. In some embodiments, the antimetabolite (such as gemcitabine) is delivered locally to the bladder of the individual for at least 24 hours. In some embodiments, the antimetabolite (such as gemcitabine) is delivered locally to the bladder of the individual for a period of 24 hours to three weeks, 2 to 20 days, 3 to 16 days, or 4 to 14 days. In some embodiments, the antimetabolite (such as gemcitabine) is delivered locally to the bladder of the individual for 7 days. In some of these embodiments, 225 mg of gemcitabine is delivered locally to the bladder over 7 days. In some of these embodiments, the method comprises delivering 225 mg of gemcitabine locally to the bladder for 7 days, followed by a 14 day rest period, followed by delivering 225 mg of gemcitabine locally to the bladder for 7 days. In some embodiments, the method comprises delivering an antimetabolite (such as gemcitabine) locally to the bladder for a period of 3 weeks. In some embodiments, the method comprises delivering 225 mg of gemcitabine is delivered locally to the bladder over 3 weeks. In some embodiments, the method comprises delivering 450 mg of gemcitabine locally to the bladder over a period of 6 weeks. In some embodiments, the antimetabolite is gemcitabine.

In some embodiments, the present methods are especially suited for treatment of individual with CT2 patients who would typically receive a radical resection followed by neoadjuvant therapy. The present methods result in local/regional (locoregional) control of the disease, including nodes, and thus can be used for long-term treatment in this bladder sparing population. The present methods also result in freedom from invasive recurrence, good long term bladder function, and low rates of salvage cystectomy, all of which are of major importance in the elderly, relatively frail population of individuals with bladder cancer who have an average age of 70.

### End Points

The methods provide herein are useful for the treatment of urothelial cancer of the lower tract. In some embodiments, the methods provided herein result in decreased time to metastasis. In some embodiments, the methods provided herein prevent metastasis. In some embodiments, the methods provided herein result in prevention of metastasis to the lymph nodes. In some embodiments, the methods provided herein prevent nodal involvement. In some embodiments, the methods provided herein increase the pathologic response rate compared to existing treatments. For example, the methods provided here achieve about a 20%, about a 30%, about a 40%, about a 50%, about a 60%, about a 70%, about a 80%, about a 90% or about a 100% pathologic response rate. In some embodiments, the methods provided herein result in an individual not being upstaged at the time of cystectomy. In some embodiments the methods provided herein result in a decrease in tumor size, or a pathological or clinical down staging of a tumor. In some embodiments, the methods provided herein result in an increased clinical complete response (cCR), partial response (cPR) and/or overall response (cOR) compared to the standard of care as of March, 2017. In some embodiments, the methods provided herein result in a pathologic complete response (pCR) or a pathological partial response (pPR). In some embodiments, pPR is defined as the absence of residual invasive cancer and the presence of residual of non-muscle invasive cancer. In some embodiments, pCR is defined as the absence of residual cancer in the bladder and regional lymph nodes. In some embodiments, the present methods result in an improvement in disease free or overall survival. In some embodiments, the present methods result in a decrease in hematuria.

In some embodiments the methods provided herein result in ablation of a muscle invasive tumor. In some embodiments, the methods provided herein result in complete ablation of the muscle invasive tumor such that upon treatment the histopathological stage is pT0. In some embodiments, the methods provided herein result in ablation of a muscle invasive tumor with residual pTis. In some embodiments, the methods provided herein result in no residual exophytic tumor. In some embodiments, the methods provided herein result in a clear reduction in tumor volume. In some embodiments, the methods provided herein result in tumor shrinkage.

### II. lntravesicular (intravesical) Devices

### Device Shape

In some embodiments, the methods provided herein comprise administering an antimetabolite (such as gemcitabine) using an intravesicular (intravesical) device. In some embodiments, the intravesicular (intravesical) device comprises a deployment shape and a retention shape. For example, the device may be elastically deformable between a relatively straightened or uncoiled shape suited for insertion through a lumen (e.g., the urethra) into the bladder of the individual (the deployment shape) and a retention shape suited to retain the device within the bladder. For the purposes of this disclosure, terms such as "relatively expanded shape," "relatively higher-profile shape," or "retention shape" generally denote any shape suited for retaining the device in the intended implantation location, including but not limited to a pretzel shape or other coiled shape (e.g., comprising bi-oval or overlapping coils) that is suited for retaining the device in the bladder. The retention shape provides that the device resists becoming entrained in urine and excreted when the individual voids. Similarly, terms such as "relatively lower-profile shape" or "deployment shape" generally denote any shape suited for deploying the drug delivery device into the body, for example the bladder, including, but not limited to, including a linear or elongated shape that is suited for deploying the device through the working channel of catheter, cystoscope, or other deployment instrument positioned in the urethra. In embodiments, the drug delivery device may naturally assume the relatively expanded shape and may be deformed, either manually or with the aid of an external apparatus, into the relatively lower-profile shape for insertion into the body. For example, the external apparatus may be an inserter configured for transurethral insertion. Once deployed the intravesicular (intravesical) device may spontaneously or naturally return to the initial, relatively expanded shape for retention in the body. In some embodiments, the device behaves like a spring, deforming in response to a compressive load (e.g., deforming the device into a deployment shape) but spontaneously returning to a retention shape once the load is removed.

In some embodiments, the shape changing functionality of the intravesicular (intravesical) device described in the preceding paragraph may be provided by including a shape retention frame (i.e., a "retention frame") in the device, such as those disclosed in the patent applications publications identified above and incorporated herein by reference. In some embodiments, the device may include a retention frame lumen in which the retention frame, which may be an elastic wire, e.g., a superelastic alloy such as nitinol, is secured. The retention frame may be configured to return spontaneously to a retention shape, such as a "pretzel" shape or another coiled shape, such as those disclosed in the applications previously incorporated. In particular, the retention frame may retain the device in the body, such as in the bladder. The retention shape provides that the device resists becoming entrained in urine and excreted when the individual voids. For example, the retention frame may have an elastic limit and modulus that allows the device to be introduced into the body in a relatively lower-profile shape, permits the device to return to the relatively expanded shape once inside the body, and impedes the device from assuming the relatively lower-profile shape within the body in response to expected forces, such as the hydrodynamic forces associated with contraction of the detrusor muscle and urination. Thus, the device may be retained in the individual's bladder once deployed, limiting or prevent accidental expulsion.

In some other embodiments, the shape changing functionality of the intravesicular (intravesical) device may be provided by forming the device housing at least in part of a thermally shape set elastic polymer.

The material used to form the device body (i.e., the housing), at least in part, may be elastic or flexible to permit moving the device between deployment and retention shapes. When the device is in the retention shape, the retention frame portion may tend to lie inside the drug reservoir portion as shown, although the retention frame portion can be positioned inside, outside, above, or below the drug reservoir portion in other cases. The material used to form the device body may be water permeable so that solubilizing fluid (*e.g.,* urine) can enter the drug reservoir portion to solubilize the non-liquid forms of the antimetabolite, immunomodulating agent, additional therapeutic agent, functional agent, or combination thereof contained in the drug reservoir once the device is deployed into the bladder. For example, silicone or another biocompatible elastomeric material may be used. In other embodiments, the device body may be formed, at least in part, of a water-impermeable material.

In some embodiments, the device body is made of an elastic, biocompatible polymeric material. The material may be non-resorbable or resorbable. Example non-resorbable materials include synthetic polymers selected from poly(ethers), poly(acrylates), poly(methacrylates), poly(vinyl pyrolidones), poly(vinyl acetates), poly(urethanes), celluloses, cellulose acetates, poly(siloxanes), poly(ethylene), poly(tetrafluoroethylene) and other fluorinated polymers, and poly(siloxanes). Example resorbable materials, specifically biodegradable or bioerodible polymers, include synthetic polymers selected from poly(amides), poly(esters), poly(ester amides), poly(anhydrides), poly(orthoesters), polyphosphazenes, pseudo poly(amino acids), poly(glycerol-sebacate), poly(lactic acids), poly(glycolic acids), poly(lactic-co-glycolic acids), poly(caprolactones), poly(caprolactone) (PC) derivatives, amino alcohol-based poly(ester amides) (PEA) and poly (octane-diol citrate) (POC), and other curable bioresorbable elastomers. PC-based polymers may require additional cross-linking agents such as lysine diisocyanate or 2,2- bis(e-caprolacton-4-yl)propane to obtain elastomeric properties. Copolymers, mixtures, and combinations of the above materials also may be employed.

In some embodiments, the device body comprises silicone, thermoplastic polyurethane, ethyl vinyl acetate (EVA), or a combination thereof. In some embodiments, the device body comprises two different thermoplastic materials, one of which is a hydrophilic thermoplastic polyurethane and is drug permeable, with the other being drug-impermeable. The drug impermeable material may be a selected from the group consisting of hydrophilic polyurethane, hydrophilic polyesters, and hydrophilic polyamides. The device body may comprise an annular tube formed by an extrusion or coextrusion process, using one or more these materials, as described in U.S. Publication 2016/0310715.

### Drug Core

In embodiments in which the anti-metabolite is delivered from an intravesical (intravesicular) drug delivery device, the drug may be housed in the device in various forms, which may depend on the particular mechanism by which the device controllably releases the drug into fluid (e.g., urine) in the bladder. In some embodiments, the drug is provided in a solid, semi-solid, or other non-liquid form, which advantageously may facilitate stable storage of the drug before the device is used and advantageously may enable the drug payload of the device to be stored in smaller volume than would be possible if the drug were housed in the form of a liquid solution. In an embodiment, the non-liquid form is selected from tablets, granules, powders, semisolids (e.g., an ointment, cream, paste, or gel), capsules, and combinations thereof. In one embodiment, the drug is in the form of a plurality of tablets, such as mini- tablets described in U.S. Patent No. 8,343,516.

For example, the anti-metabolite, may take such forms as suspensions, solutions, or emulsions in oily or aqueous vehicles, and may contain formulatory agents such as suspending, stabilizing and/or dispersing agents. Alternatively, the active ingredients may be in powder form, obtained by aseptic isolation of sterile solid or by lyophilization from solution, for constitution with a suitable vehicle, e.g., sterile, pyrogen-free water, before use.

In one embodiment, the anti-metabolite is formulated with one or more excipients that include a viscosity enhancing agent to control release of solubilized antimetabolite from a release aperture in the device housing. In another embodiment, the device reservoir includes both antimetabolite and a viscosity enhancing agent, but they are not co-formulated and instead are provide in discrete regions within the reservoir, e.g., as separate tablets. Suitable viscosity enhancing agents, including but not limited to polyethylene oxide (PEO), are known in the pharmaceutical arts. In some variations of the embodiment, the viscosity enhancing agent may be provided, e.g., formulated, with urea or another osmotic agent.

In one embodiment, the anti-metabolite is administered to the individual with a solubility enhancing agent. In an embodiment, the solubility enhancing agent is urea. In one embodiment, the urea is provided in a tablet or other solid form and loaded with the antimetabolite in the drug reservoir of an intravesicular (intravesical) drug delivery device. The urea may also function, depending on the device, as an osmotic agent to facilitate generation of an osmotic pressure in a drug reservoir. In a particular embodiment, the antimetabolite and the osmotic agent are configured as separate tablets (or other solid forms) positioned within different regions of the drug reservoir as described in PCT WO 2015/026813 (Lee et al.) which is incorporated by reference herein.

In some embodiments the device may comprise a drug reservoir lumen. In some of these embodiments, each drug reservoir lumen may hold one or several drug tablets or other solid drug units. In one embodiment, the device holds from about 10 to 100 cylindrical drug tablets, such as mini-tablets, among a number of discrete drug reservoir lumens. In certain embodiments, the mini-tablets may each have a diameter of about 1.0 to about 3.3 mm, such as about 1.5 to about 3.1 mm, and a length of about 1.5 to about 4.7 mm, such as about 2.0to about 4.5 mm.

### Drug Housing

The release of antimetabolite from the intravesicular (intravesical) devices described herein may be driven and controlled by different mechanisms of action. In various embodiments, the drug may be released from the intravesicular (intravesical) drug delivery device by diffusion through a wall of the drug housing, by diffusion through one or more defined apertures in a wall of the drug housing, by osmotic pressure through an aperture in the drug housing, by osmotic pressure through one or more transiently formed microchannels, by erosion of a drug formulation in contact with urine in the bladder, or by a combination thereof. In some embodiments, drug release is controlled by drug diffusion through a drug-permeable polymer or matrix component defining part of the device housing. In one embodiment, the device includes a drug-permeable polymer component.

The size of the housing, including the thickness of the wall, may be selected based on the volume of drug (and functional agent, if any) formulation(s) to be contained, the desired rate of delivery of the drug from the device body/housing, the intended site of implantation of the device within the body, the desired mechanical integrity for the device, the desired release rate or permeability to water and urine, the desired induction time before onset of initial release, and the desired method or route of insertion into the body, among others. In embodiments in which the housing is a tube, the tube wall thickness may be determined based on the mechanical properties and water permeability of the tube material, as a tube wall that is too thin may not have sufficient mechanical integrity while a tube wall that is too thick may experience an undesirably long induction time for initial drug release from the device and/or may not have sufficient flexibility to permit delivery through a urethra or other narrow body lumen.

In some embodiments, the housing may be an elongated, annular tube having an inner diameter from about 2 mm to about 5 mm. The drug, and functional agent if any, may be solid tablets having a diameter substantially the same as the inner diameter of the elongated annular tube. In some embodiments, the housing holds one or more first drug units comprising a drug and one or more second drug units comprising a functional agent which facilitates release of the drug. One or more of the first unit tablets may fill a length from about 1 cm to about 3 cm of the lumen of the tube, and one or more of the second unit tablets may fill a length from about 10 cm to about 15 cm of the lumen of the tube. In one embodiment, the ratio of volume of the first unit(s) to volume of the second unit(s) is from about 0.05 to about 0.5. Other lengths and ratios of the tablet payloads are envisioned.

In some embodiments, the housing may be an elongated, annular tube having a wall thickness from 0.1 to 0.4 mm, such as a wall thickness of 0.2 mm. The housing material may comprise one or more biocompatible elastomers. The housing material may be selected such that the housing has a durometer from 25A to 80A, such as 25A, 50A, 65A, 70A, or 80A.

In various embodiments, the intravesicular (intravesical) device may release the drug continuously or intermittently to achieve a concentration of the drug in the bladder that produces a sustained, therapeutically effective concentration of the drug in urine in the bladder as described in the methods provided herein. For example, over a period from 1 hour to 1 month, for example from 2 hours to 2 weeks, from 6 hours to 1 week, from 24 hours to 72 hours, etc. In certain embodiments, the intravesicular (intravesical) device may release the antimetabolite in an amount of from 1 mg/day to 1000 mg/day, for example from 20 mg/day to 300 mg/day or from 25 mg/day to 300 mg/day. In certain embodiments, these release rates are provided over a treatment period as described herein. In certain embodiments, these release rates are provided over a treatment period from 14 days to 21 days.

### Osmotic and Diffusion Systems

Following in vivo deployment, the device releases the drug. Release may occur, as described above, due to an osmotic pressure gradient between the interior and exterior of the device, the drug passing through one or more orifices or passing pores in the device under the force of osmotic pressure. Release may also occur by diffusion, whereby the drug passes through one or more orifices or passing pores in the device and/or through a drug-permeable wall of the device, due to a drug concentration gradient between the interior and exterior of the device. Combinations of these release modes within a single device are possible, and in some embodiments are preferred in order to achieve an overall drug release profile not readily achievable from either mode individual.

In some embodiments in which the device comprises a drug in a solid form, elution of drug from the device occurs following dissolution of the drug within the device. Bodily fluid enters the device, contacts the drug and solubilizes the drug, and thereafter the dissolved drug diffuses from the device or flows from the device under osmotic pressure or via diffusion. For example, the drug may be solubilized upon contact with urine in cases in which the device is deployed in the bladder. In certain embodiments, a water permeable wall portion of the housing is permeable to the drug in aqueous solution, such that solubilized drug is released via the wall portion, also referred to herein as "trans-wall diffusion." After the device is implanted, water or urine permeates through the wall, enters the reservoir, and solubilizes the functional agent and/or drug. The drug then diffuses directly through the wall at a controlled rate, due to a drug concentration gradient between the interior and the exterior of the device. For example, the housing and/or any water or drug permeable wall portions may be silicone, a thermoplastic polyurethane, ethylene-co-vinyl acetate (EVA), or a combination thereof.

In some embodiments, the intravesicular (intravesical) device may contain a unit concentration of 225 mg of gemcitabine. In some of these embodiments, the device may be configured to deliver about 100 to about 225 mg of gemcitabine (e.g., about 140 mg, about 160 mg, about 180 mg, about 200 mg, or about 220 mg) mg of antimetabolite to the individual over a 7 day period or over a 3 week period.

In a particular embodiment, the drug delivery device may include a permeation system as described in WO2014/145638 and U.S. Publication 2016/0310715, which are herein both incorporated by reference in its entirety. In some embodiments, the drug delivery device includes a housing having a closed drug reservoir lumen bounded by a first wall structure and a hydrophilic second wall structure; and a drug formulation comprising antimetabolite contained in the drug reservoir lumen, wherein the first wall structure is permeable or impermeable to water and impermeable to the drug, and the second wall structure is permeable to the antimetabolite.

In some embodiments, the device housing has walls bounding and defining the drug reservoir of the device that are made of a first material that serves as the first wall structure and a second material that serves as the second wall structure, such that drug release occurs essentially only through the second material. In one embodiment, the device does not include an aperture; drug release is only by diffusion through the second wall structure. As used herein, the terms "impermeable to the drug" and "impermeable to water" refer to the wall structure being substantially impermeable to the drug or to water, such that essentially no drug or water is released via the wall structure over the therapeutic release period. For use in the bladder, it is desirable that the device be compliant (i.e., easily flexed, soft feeling) during detrusor muscle contraction in order to avoid or mitigate discomfort and irritation to the patient. Thus, the durometer of the first and second materials of construction are a design consideration, and the proportion of a high durometer material may be limited in constructing a device housing of a given size while keeping it suitably compliant in the bladder. For example, Tecophilic^{™} thermoplastic polyurethane (Lubrizol Corp.) may have a Shore hardness greater than 70A, such as from 80A to 65D, while silicone tubing which may have a Shore hardness of from 50A to 70A. Accordingly, it can be advantageous to utilize the combination of these two different polymeric materials, rather than making the device entirely of the water-swelling hydrophilic, drug-permeable second material.

The arrangement of the first and second wall structures can take a variety of forms. In certain embodiments, the first wall structure is a cylindrical tube and the second wall structure is an end wall disposed at least one end of the cylindrical tube, or the first wall structure and the second wall structure are adjacent one another and together form a cylindrical tube. That is, drug release is controlled by drug diffusion through a drug- permeable component defining a portion of the closed device housing. The drug-permeable wall structure may be located, dimensioned, and have material properties to provide the desired rate of controlled drug diffusion from the device. In one embodiment, the drug permeable wall may include a disk stabilized in the lumen of a tube at or near an end of the tube, optionally sandwiched between an inner washer and an outer washer. In another embodiment, the drug permeable wall is part of a sidewall of a tubular housing, or part of an end plug located at the end of a tubular housing.

The length and width, e.g., wall portion formed of the water permeable material are selected to provide a desired rate of water flux into the reservoir defined by device housing. In one embodiment, the width of the water permeable wall portion may be quantified by the arc angle defining the wall when viewed in cross-section normal to the luminal axis. The water permeable region(s) of the device housing can be controlled to give a selected area of, and thus rate for, osmotic water imbibition, and yet advantageously maintain suitable overall dimensions and elasticity of the device, formed of suitable biocompatible elastomers. Advantageously by forming the device housing by a co-extrusion process, the structural variations of the water permeable region(s) can be created with conventional co-extrusion equipment by selection of the processing parameters, thereby beneficially providing the ability to cost-effectively manufacture multiple structural device configurations. In some embodiments, the length of the water permeable regions(s) runs along only a portion of the overall length of the device. In such an embodiment, larger arc angles of the water permeable region(s) can therefore be employed while keeping the rate of drug release at a desirable level over an extend period of time.

In some embodiments, the wall may have a varied thickness over the circumference of the wall, for example the drug permeable portion may have a thickness that is less than the thickness of the drug impermeable portion. Moreover, the thinner drug permeable wall structure may be disposed at various positions relative the adjacent, thicker drug impermeable wall structure. In **some embodiments**, drug, release is controlled by drug diffusion through a drug-permeable component defining a portion of the closed device housing. The drug-permeable wall structure may be located, dimensioned, and have material properties to provide the desired rate of controlled drug diffusion from the device.

In some embodiments, the drug delivery device comprises a housing comprising a first wall structure and a second wall structure that are adjacent one another and together form a tube defining a drug reservoir lumen; and a drug contained in the drug reservoir lumen, wherein: (i) the second wall structure, or both the first wall structure and the second wall structure, are permeable to water, (ii) the first wall structure is impermeable to the drug and the second wall structure is permeable to the drug, such that the drug is releasable *in vivo* by diffusion through the second wall structure, (iii) the second wall structure comprises less than 90 percent of a cross sectional area of the tube, in a cross section normal to the longitudinal axis of the tube, ( i v ) and the first wall structure comprises a first polyurethane composition.

In some embodiments, the device comprises an elongated, elastic housing having a drug reservoir lumen extending between a first closed end and a second closed end; and a drug contained in the drug reservoir lumen, wherein (i) the housing comprises a tubular wall structure which comprises: a first annular segment formed entirely of a first material which is impermeable to the drug, and a second annular segment formed at least partially of a second material which is permeable to the drug and configured to release the drug *in vivo* by diffusion through the second material in the second annular segment, and (ii) the first annular segment has a first end which is integrally formed and connected with a first end of the second annular segment.

In some embodiments, the walls that define the drug reservoir lumens may have varying thickness. Housings with walls of different thicknesses may improve the housing's flexibility, compressibility, or both. Different wall thicknesses also may aid in securing a solid drug unit in the drug reservoir lumens.

In some embodiments, the intravesicular (intravesical) device body, or housing, may include openings (e.g., at the opposed ends of an annular tube) in need of sealing following loading of the drug reservoir with the drug pay load, during the assembly process. Any of these defined openings or ends of the housings, including the monolithic housing and modular housing units, may be sealed, if desired to close off an opening. This sealing may be accomplished with a sealing substance or structure. The sealing structure may be formed of biocompatible material, including a metal such as stainless steel, a polymer such as silicone, a ceramic, or sapphire, or adhesive, among others or combinations thereof. The sealing substance or structure may be biodegradable or bioerodible. In one embodiment, a medical grade silicone adhesive or other adhesive is loaded into the opening in a fluid or workable form and then cure within the housing opening to seal it. In some embodiments, the housing includes one or more predefined apertures for release of the drug from the device. These drug-release apertures are not the defined openings which are sealed. In other embodiments, the housing does not include a predefined drug-release aperture.

In some embodiments the device releases drug without a predefined drug release aperture (i.e., orifice). Release of drug from a device without a predefined drug-release aperture may be driven by diffusion or osmotic pressure. Examples of such suitable "no-orifice" release systems are described in PCT Patent Application Publication No. WO 2014/144066 (TB 130) and U.S. Patent Application Publication No. 2014/0276636 (TB 134), which are incorporated herein by reference.

In a particular embodiment, the drug delivery device may include an osmotic system as described in U.S. Publication 2016/0199544, U.S. Patent 8,679,094, and U.S. Publication 2016/0008271, which are herein incorporated by reference.

In some embodiments, the device comprises a housing defining a reservoir; a first unit contained within the reservoir, the first unit comprising a drug; and a second unit contained within the reservoir in a position distinct from the first unit, wherein the second unit comprises a functional agent that facilitates in vivo release of the drug from housing. In some embodiments, the first unit comprises one or more solid tablets which comprise at least one drug (e.g., an antimetabolite, such as gemcitabine), and the second unit comprises one or more solid tablets (e.g., which comprise an osmotic agent, such as urea). In some embodiments, the housing is in the form of an elongated elastomeric tube having a lumen (i.e., the reservoir) in which all of the solid tablets of the first and second units are aligned and contained. The diameter of the solid tablets may be substantially the same as the diameter of the lumen.

When osmotic release is the desired drug release mode, the functional agent in the second units may include an osmotic agent that facilitates osmotic release of the drug. For example, the osmotic agent may have a higher solubility than the drug, such that the osmotic agent expedites solubilization and/or subsequent release of the drug. This beneficially allows for the delivery of low solubility or other drugs typically only delivered via diffusion, from osmotic delivery-based devices. The device may exhibit an induction period while a sufficient volume of functional agent and/or drug are solubilized to achieve the osmotic pressure gradient.

Subsequently, the device may exhibit a zero-order release rate for an extended period, followed by a reduced, non-zero-order release rate over a decay period. A desired delivery rate can be achieved by controlling/selecting various parameters of the device, including but not limited to the surface area and thickness of the water permeable wall; the permeability to water of the material used to form the wall; the shape, size, number and placement of the apertures; and the dissolution profiles of the drug and functional agent.

The devices described herein may also be configured to release drug via diffusion, alone or in combination with osmotic release. The device may be configured to allow the solubilized drug to pass through a portion of the housing or one or more apertures therein.

Alternatively, or in combination with a water permeable wall portion, the housing may include at least one aperture configured to permit a fluid to enter the reservoir in vivo. The housing may also include one or more apertures or passing pores configured to permit solubilized drug to pass there through.

In some embodiments of the osmotic system, the device housing includes a first elastomeric material that is water permeable and a second elastomeric material that is water impermeable, wherein both materials are selected to be impermeable to the drug contained in the housing.

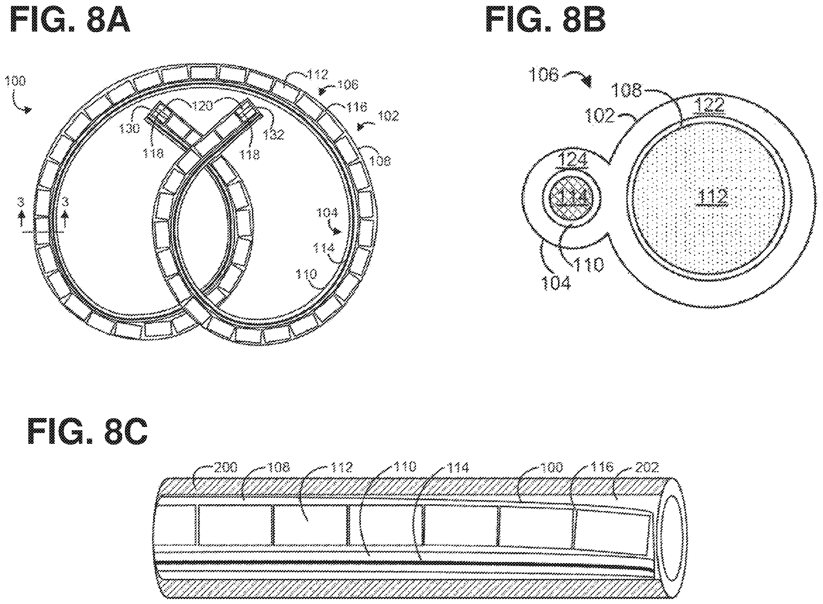

FIGS. 8A-8C illustrate one embodiment of an intravesical device useful in the methods described herein. The device 100 includes a drug reservoir portion 102 and a retention frame portion 104. In FIG. 8A, the device 100 is shown in a relatively expanded shape suited for retention within the urinary bladder of an individual. In FIG. 8C, the device 100 is shown in a relatively lower-profile shape for deployment through the working channel 202 of a deployment instrument 200, such as a cystoscope or other catheter, e.g., for insertion into and through the urethra and into the bladder of the patient. Following deployment (release of the device) into the bladder, the device 100 may assume the relatively expanded shape to retain the drug delivery device in the bladder. In the illustrated embodiment, the drug reservoir and retention frame portions 102, 104 of the drug delivery device 100 are longitudinally aligned and are integrally formed or otherwise coupled to each other along their length.

The drug delivery device 100 includes an elastic or flexible device body 106 that defines a drug reservoir lumen 108 and a retention frame lumen 110. The drug reservoir lumen 108 is configured to house a drug (e.g., an antimetabolite) which is in the form of a plurality of solid drug units 112, to form the drug reservoir portion 102. Interstices 116 or breaks formed between adjacent drug units 112 permit the drug tablets 112 to move with reference to each other so that the device 100 is flexible despite being loaded with drug in solid form. The retention frame lumen 110 is configured to house a retention frame 114 to form the retention frame portion 104.

As shown in the cross-sectional view of FIG. 8B, the device body 106 includes a tube or wall 122 that defines the drug reservoir lumen 108 and a tube or wall 124 that defines the retention frame lumen 110. The tubes 122, 124 and lumens 108, 110 can be substantially cylindrical, with the drug reservoir lumen 108 having a relatively larger diameter than the retention frame lumen 110, although other configurations can be selected based on, for example, the amount of drug to be delivered, the diameter of the retention frame, and deployment considerations such as the inner diameter of the deployment instrument. The device body 106 may be formed integrally, such as via molding or extrusion, although separate construction and assembly of the tubes 122, 124 is possible. The wall 124 that defines the retention frame lumen 110 may extend along the entire length of the wall 122 that defines the drug reservoir lumen 108, so that the retention frame lumen 110 has the same length as the drug reservoir lumen 108 as shown, although one wall may be shorter than the other wall in other embodiments. Further, the two walls 122, 124 are attached along the entire length of the device in the illustrated embodiment, although intermittent attachment can be employed.

As shown in FIG. 8A, the drug reservoir lumen 108 is loaded with a number of drug units 112 in a serial arrangement. For example, between about 10 and about 100 drug units 112 may be loaded, such as between about 20 and about 80 drug units 112. The drug units may, for example, be tablets, beads, or capsules. Essentially any number of drug units may be used, depending upon the sizes of the reservoir and the drug units. The drug reservoir lumen 108 includes open ends 130 and 132, which are shown as relatively circular openings at opposite ends of the drug reservoir lumen 108. At least one of the openings provides ingress for the drug units 112 to be placed into the drug reservoir lumen 108 during device loading and assembly.

End plugs 120 block openings 130 and 132 following loading of the drug units 112. The end plugs 120 may be cylindrical and may be secured in the drug reservoir lumen 108 by frictional engagement and/or an adhesive or other fastening means. Each end plug 120 includes an aperture 118, as illustrated, to provide a passageway for releasing drug from the drug reservoir lumen 108. In some alternative embodiments, only one of the end plugs includes an aperture. In some other alternative embodiments, neither of the end plugs includes an aperture, and in some of those embodiments, the tube wall 122 includes a defined aperture for release of drug therethrough.

The retention frame lumen 110 is loaded with the retention frame 114, which may be an elastic wire, such as a nitinol wire, (thermally) shape-set into the overlapping coiled shape shown in FIG. 8A. The retention frame 114 may have an elastic limit and modulus that allows the device 100 to be introduced into the body in a relatively lower-profile shape, permits the device 100 to return the relatively expanded shape once inside the body, and impedes the device from assuming the relatively lower-profile shape within the body in response to expected forces, such as the hydrodynamic forces associated with contraction of the detrusor muscle and urination.

### Erosion-Based Systems

In some embodiments, which may be used with tablets comprising low-solubility drugs, the drug is provided in tablet form secured in the device with exposed tablet faces, such that release of drug from the device occurs by controlled erosion/dissolution, as described in U.S. Patent No. 9,107,816. In some embodiments, the device may comprise modular housings. The modular housings are typically formed from at least two separate housing units, each unit housing at least one solid drug unit. The material from which each housing unit is formed defines at least one drug reservoir lumen capable of housing a solid drug unit. The drug reservoir lumens may have one or more defined openings. For example, the drug reservoir lumen may have two opposed openings which expose correspondingly opposed end surfaces of the at least one solid drug unit housed therein. In certain embodiments, the at least two separate housing units in the modular housings are connected, directly or indirectly, by a retention frame. In some embodiments, the modular housing units may be placed on the retention frame to form a "bracelet" design. The devices may have one housing unit or a plurality of housing units. The number of housing units may be limited only by the size of the retention frame by which they are connected.

In some embodiments, one or more of the separate housing units includes a retention frame lumen through which a shared retention frame is extended. In certain embodiments, the retention frame lumen and the drug reservoir lumen of each housing unit are arranged parallel to each other. In particular embodiments, the retention frame lumen and the drug reservoir lumen of each housing unit are arranged perpendicular to each other. In further embodiments, the retention frame lumen and the drug reservoir lumen of each housing unit are arranged at an angle other than 0° (parallel) and 90° (perpendicular), such as 5, 10, 30, 45, 60, or 85°. In further embodiments, the devices described herein include two or more housing units with at least two of the following configurations: (1) the retention frame lumen and drug reservoir lumen are arranged substantially parallel to each other, (2) the retention frame lumen and drug reservoir lumen are arranged substantially perpendicular to each other, and (3) the retention frame lumen and drug reservoir lumen are arranged at an angle other than 0° (parallel) and 90° (perpendicular).

### Integrated Silicone-Drug Delivery Systems

In some embodiments, the device may comprise an elastic polymer-drug matrix as described in WO2015/200752, which is herein incorporated by reference in its entirety.

### Devices with Multiple Release Portions

In particular embodiments, the device includes at least two drug release portions, at least one release portion releasing drug at a different rate than another release portion as described in WO2011/031855 which is herein incorporated by reference in its entirety. The release portions may achieve different release rates by having different configurations, by housing different drug formulations, or by employing different release mechanisms, among others or combinations thereof. The release portions may be combined to achieve a desired release profile. For example, the device may include release portions that exhibit different induction or lag times before the onset of initial release, that release drug at different rates or according to different release curves after the onset of release, or that release drug for different periods before the drug load is substantially exhausted, among others or combinations thereof. The disparate release portions may be combined to achieve a desired release profile from the drug delivery device as a whole, such as a release profile that demonstrates a relatively short initial lag time and thereafter demonstrates continued release at a relatively constant rate over an extended period.

In some embodiments, the devices are loaded with drugs in the form of a number of solid drug tablets, which may be smaller in size than conventional drug tablets. Because the devices control release of the drug into the body, the drug itself may include little or no excipients that control drug release. Instead, the excipients present in the drug tablets may be present primarily or completely to facilitate the tableting process or solubilization in vivo. Thus, the devices may provide a high drug payload on a volume or weight basis, yet the devices may be small enough for in vivo deployment in a minimally invasive manner.

The drug housing also permits the egress of drug, in either liquid or semi-solid form as implanted or following *in vivo* solubilization. The wall may be formed from a drug-permeable material that permits drug efflux through the drug housing along its entire length. The wall also may be formed from a material that is semi-permeable to the drug depending at least in part on the drug form. For example, the wall may be permeable to the drug in one form, such as a charged form, but not another form, such as uncharged form (e.g., base form versus salt form). The wall also may include one or more openings or passageways formed completely through it that permit drug to exit the drug housing.

The drug housing houses a drug in the form of a number of solid drug tablets, which are aligned within the drug housing in a serial arrangement and are enclosed within the drug housing with sealing structures, such as plugs, that close entry openings on opposite ends of the drug housing. Interstices or breaks formed between adjacent drug tablets permit the drug tablets to move with reference to each other so that the device is flexible despite being loaded with drug in solid form.

The drug portion can have any combination of the characteristics or configurations described herein, meaning the aperture may be provided, omitted, substituted with a passing pore, or augmented with additional apertures or passing pores; the housing may have a porous wall with an open-cell structure or a closed-cell structure; one or more degradable timing structures or release modulating structures may be associated with the housing, or any combination thereof.

The drug tablets may be aligned in any arrangement other than a serial arrangement, depending on the configuration of the drug housing. The drug tablets may fill any portion of the drug housing other than the entire drug housing as illustrated. A filling material such as silicone adhesive can be used to fill any portion of the drug housing that is not loaded with drug tablets, or air may be used, increasing the buoyancy of the device. The composition of the drug tablets may be the same or may vary along the device. The drug also may be in forms other than a drug tablet, such as other liquid, semi- solid, or solid forms (e.g., granules).

In particular embodiments, the drug delivery device includes at least two discrete or segregated drug portions associated with a single retention portion. The drug portions may be separate drug housings each associated with the retention portion, or the drug portions may be separate areas within a single drug housing that is associated with the retention portion.

Each drug portion may be defined by a portion of the wall of the drug housing and at least one partition structure, which separates the drug portion from a second drug portion. The partition structure may be a plug inserted into the housing, such as a cylinder, sphere, or disk, among others, which is secured in place due to its size or with an adhesive. The partition structure also may be a portion of the housing formed directly therein, such as by molding.

A device with at least two discrete portions may be suited for controlled release of at least two drug payloads from a corresponding number of drug reservoirs. The two discrete portions may have the same configurations or different configurations as described herein. The two drug payloads may be the same as each other or may differ from each other with reference to content, such as active ingredient content or excipient content; form, such as salt form or base form; state, such as liquid, semi-solid, or solid state; among others or combinations thereof. Thus, the two discrete portions may release the two drug payloads at the same time or at different times, at the same rate or at different rates, via the same release mechanisms or different release mechanisms, or any combination thereof.

For example, one drug portion may be configured to release its drug payload relatively quickly after implantation and another drug portion may be configured to experience an induction time before beginning release, or a combination thereof. The onset of release of two payloads in different drug portions can be staged. Examples of quick release drug portions include a drug portion that operates as a relatively fast-acting osmotic pump, such as a silicone tube having a relatively thinner wall, a drug portion that is loaded with drug in a quick release form, such as liquid form or a specially formulated solid form, a drug portion associated with a relatively fast-acting degradable timing structure, or combinations thereof. Thus, the device may release drug during an initial, acute phase and during a maintenance phase.

As another example, one drug portion may be configured to release its drug payload at a relatively faster rate than the other drug payload. For example, one drug portion may house a drug payload with low water solubility for diffusive release that is initiated relatively soon after implantation, and another drug portion may house a drug payload that is highly water soluble for osmotic release after an induction period. As another example, one drug portion may house a drug payload in a liquid state for quick release through an aperture having a fast-acting degradable timing membrane, and another drug portion may house another drug payload of solid tablets for slow release following solubilization in vivo. As still another example, one drug portion may have a relatively solid wall while another drug portion may have a number of apertures or pores formed through its wall, which may increase the release rate due to diffusion, or a closed-cell porous wall, which may increase the release rate due to increased permeation of water or drug through the wall.

The release portions may be combined to achieve a desired release profile. For example, the device may include release portions that exhibit different induction or lag times before the onset of initial release, that release drug at different rates or according to different release curves after the onset of release, or that release drug for different periods before the drug load is substantially exhausted, among others or combinations thereof. The disparate release portions may be combined to achieve a desired release profile from the drug delivery device as a whole, such as a release profile that demonstrates a relatively short initial lag time and thereafter demonstrates continued release at a relatively constant rate over an extended period.

By combining multiple distinct drug portions in a single device, the device may exhibit a desired release profile of an anti-metabolite. The release profile from the device as a whole may be the sum of the release profiles of the discrete portions, for example, with the first portion exhibiting minimal lag time before the onset of release, the second portion exhibiting a short induction period as the osmotic pressure gradient develops, and the third portion exhibiting a longer delay before onset as the degradable structure dissolves or degrades. Once release begins from any one portion, the release rate may be relatively zero-order for an extended period, followed by a period of decay. It should be noted that the three discrete portions are examples, and that any number or combination of discrete portions may be used to achieve the desired release profile.

Because the different drug portions are merely segregated areas within in a single tubular housing, the device advantageously may be relatively simple to construct and deploy, and yet the different drug portions exhibit different release profiles due to the different drug payloads, aperture placement, and degradable timing structures. In other embodiments in which the drug portions use, for example, walls of different materials, thicknesses, or porous cell structures, the housing may vary along its length or separate drug housings may be used. Thus, controlled release may be achieved in a range of manners.

### Gels

In another embodiment, a coating substance may be intravesically applied to the bladder wall (e.g., to an area of the urothelium inside the urinary bladder), wherein the coating substance includes the antimetabolite or other drug and one or more excipient materials that promote adherence of the coating substance to the bladder wall and provides continuous controlled release of the drug over the treatment period. The coating substance may be a mucoadhesive formulation, such as gels, ointments, creams, pastes, films, emulsion gels, tablets, polymers, or a combination thereof. Mucoadhesive formulation polymers may include hydrogels or hydrophilic polymers, polycarbophil (i.e. Carbopols, etc.), chitosan, polyvinylpyrrolidone (PVP), lectin, polyethyleneglycolated polymers, celluloses, or a combination thereof. Suitable celluloses include methyl cellulose (MC), carboxymethyl cellulose (CMC), hydroxypropyl cellulose (HPC), or combinations thereof. The coating substance may include a permeation enhancer. Non-limiting examples of permeation enhancers include dimethyl sulfoxide (DMSO), sodium carboxymethyl cellulose (NaCMC), lipids, surfactants, or combinations thereof. A coating substance may be deployed in the bladder so that the coating substance engages the bladder wall.

The coating substance may be deployed in the bladder using a deployment instrument. The deployment instrument may be any device designed to navigate natural lumens of the body to reach the intended implantation site. For deployment in the bladder, the deployment instrument is sized and shaped for passing through a urethra of a patient to a bladder. The deployment instrument may be a known device, such as a catheter or cystoscope, or a specially designed device. The deployment instrument is used to deploy the coating substance into the body and is subsequently removed from the body, leaving the coating substance wholly implanted in the body. Once so implanted, the coating substance may release drug into the body for an extended period. A comparable procedure can be used to deploy any of the devices or drugs described herein into other parts of the body through other natural lumens. For example, a deployment instrument can be used to deploy a liquid drug or drug formulation into the bladder by passing the deployment instrument through a urethra.

### Devices Comprising an Anti-Metabolite and a Second Agent

In some embodiments, the intravesicular (intravesical) devices provided herein comprise an immunomodulating agent. In some of these embodiments, the intravesicular (intravesical) devices provided herein comprise an immunomodulating agent and an antimetabolite. In some embodiments the immunomodulating agent and the antimetabolite are delivered at different rates.

### III. Kits

Provided herein are kits including an antimetabolite and an immunomodulating agent. In some embodiments, the kit includes an intravesicular (intravesical) device comprising an antimetabolite. In some embodiments, the kit includes an intravesicular (intravesical) device comprising antimetabolite packaged with an immunomodulating agent. In some embodiments, the kit includes an intravesicular (intravesical) device comprising an antimetabolite and an immunomodulating agent.

### IV. Exemplary Embodiments

Embodiment 1. A method of treating a urothelial carcinoma of the lower tract in an individual comprising administering to the individual an effective amount of an antimetabolite, wherein the antimetabolite is delivered locally to the bladder.

Embodiment 2. A method of enhancing an immune response against a urothelial carcinoma of the lower tract in an individual comprising administering to the individual an effective amount of an antimetabolite, wherein the antimetabolite is delivered locally to the bladder.

Embodiment 3. A method of reducing recurrence or progression of a urothelial carcinoma of the lower tract of an individual comprising administering to the individual an effective amount of an antimetabolite, wherein the antimetabolite is delivered locally to the bladder.

Embodiment 4. A method of improving tumor microenvironment for cancer immunotherapy in an individual having a urothelial carcinoma of the lower tract, comprising administering to the individual an effective amount of an antimetabolite, wherein the antimetabolite is delivered locally to the bladder.

Embodiment 5. A method of sensitizing an individual having a urothelial carcinoma of the lower tract for radiation therapy, comprising administering to the individual an effective amount of an antimetabolite, wherein the antimetabolite is delivered locally to the bladder.

Embodiment 6. The method of any one of embodiments 1-5, wherein the antimetabolite is a nucleoside analog.

Embodiment 7. The method of embodiment 6, wherein the antimetabolite is gemcitabine.

Embodiment 8. The method of any of embodiments 1-7, wherein the antimetabolite is delivered continuously into the bladder over a period of at least about 24 hours.

Embodiment 9. The method of any of embodiments 1-8, wherein the antimetabolite is delivered in a first phase of the delivery at a first release rate followed by a second phase of the delivery having a second release rate.

Embodiment 10. The method of any one of embodiments 1-9, wherein the antimetabolite is delivered in a first phase of the delivery at a first dose followed by a second phase of the delivery at a second dose.

Embodiment 11. The method of embodiment 9 or 10, wherein the first phase and the second phase are contiguous.

Embodiment 12. The method of embodiment 9 or 10, wherein the first phase and the second phase are separated by a resting period.

Embodiment 13. The method of any of embodiments 1-9, wherein the antimetabolite is delivered at a dose of from about 1 mg/day to about 300 mg/day.

Embodiment 14. The method of any of embodiments 1-13, wherein the concentration of gemcitabine in the urine is from about 0.1 µg/mL to about 200 µg/mL during the delivery period.

Embodiment 15. The method embodiment 14, wherein the concentration of antimetabolite in the urine is from about 1 µg/mL to about 10 µg/mL during the delivery period.

Embodiment 16. The method of embodiment 15, wherein the concentration of antimetabolite in the urine is about 10 µg/mL during the delivery period.

Embodiment 17. The method of any of embodiments 1-16, wherein the antimetabolite concentration in the plasma of the individual is less than about 1 µg/ml.

Embodiment 18. The method of any of embodiments 1-17, wherein upon delivery of antimetabolite the ratio of antimetabolite in the urine to antimetabolite in the plasma of the individual is greater than about 500:1.

Embodiment 19. The method of any of embodiments 1-18, wherein the antimetabolite is delivered over at least a month, wherein each antimetabolite delivery period is at least one day, and wherein the interval between each antimetabolite delivery period is no more than about a week.

Embodiment 20. The method of any of embodiments 1-19, comprising a) a first antimetabolite delivery period, wherein the concentration of antimetabolite in the urine of the individual is at least about 0.1 µg/mL; b) a rest period; and c) a second antimetabolite delivery period, wherein the concentration of antimetabolite in the urine of the individual is greater than about 0.1 µg/mL.

Embodiment 21. The method of embodiment 20, wherein the concentration of antimetabolite in the urine is greater than about 1 µg/mL for at least half of the rest period.

Embodiment 22. The method of any one of embodiments 1-21, further comprising administering to the individual an effective amount of a second agent.

Embodiment 23. The method of embodiment 22, wherein the second agent is delivered at the time the antimetabolite delivery is initiated.

Embodiment 24. The method of embodiment 22, wherein the second agent is delivered before the antimetabolite delivery is initiated.

Embodiment 25. The method of embodiment 22, wherein the second agent is delivered after the antimetabolite delivery is initiated.

Embodiment 26. The method of embodiment 22, wherein the second agent is delivered after the antimetabolite delivery is terminated.

Embodiment 27. The method of any one of embodiments 22-26, wherein the antimetabolite delivery period and the second agent delivery period overlap with each other.

Embodiment 28. The method of any one of embodiments 22-26, wherein the antimetabolite delivery period and the second agent delivery period are non-overlapping. Embodiment 29. The method of any one of embodiments 22-28, wherein the second agent is delivered systemically.

Embodiment 30. The method of any one of embodiments 22-28, wherein the second agent is delivered locally.

Embodiment 31. The method of any one of embodiments 22-30, wherein the second agent is delivered systemically in a first phase of the second agent delivery period, followed by a local delivery at a second phase of the second agent delivery period.

Embodiment 32. The method any one of embodiments 22-30, wherein the second agent is delivered locally in a first phase of the second agent delivery period, followed by a systemic delivery at a second phase of the second agent delivery period.

Embodiment 33. The method of embodiment 31 and 32, wherein the first phase of the second agent delivery period and the second phase of the second agent delivery period are separated by at least about a month.

Embodiment 34. The method of embodiment 22, wherein the antimetabolite and the second agent are delivered simultaneously.

Embodiment 35. The method of embodiment 34, wherein the antimetabolite and the second agent are delivered via a single delivery device.

Embodiment 36. The method of embodiment 35, wherein the antimetabolite and the second agent are delivered at the same release rate.

Embodiment 37. The method of embodiment 35, wherein the antimetabolite and the second agent are delivered at different release rate.

Embodiment 38. The method of any one of embodiments 34-37, further comprising delivering the second agent separately from the antimetabolite.

Embodiment 39. The method of embodiment 38, wherein the second agent is delivered systemically.

Embodiment 40. The method of embodiment 38, wherein the second agent is delivered locally.

Embodiment 41. The method of any one of embodiments 22-40, wherein the second agent is a chemotherapeutic agent.

Embodiment 42. The method of embodiment 41, wherein the second agent is selected form the group consisting of paclitaxel, docetaxel, carboplatin, cisplatin, and oxaliplatin.

Embodiment 43. The method of any one of embodiments 22-40, wherein the second agent is an immunomodulating agent.

Embodiment 44. The method of embodiment 43, wherein the immunomodulating agent is an immune checkpoint inhibitor.

Embodiment 45. The method of embodiment 44, wherein the immune checkpoint inhibitor is an inhibitor of an immune checkpoint protein selected from the group consisting of PD-L1, CTLA4, PD-L2, PD-1, B7-H3, B7-H4, HVEM, B- and T-lymphocyte attenuator (BTLA), Killer inhibitory receptor (KIR), GAL9, TIM3, A2AR, LAG-3, phosphatidylserine, CD27, TNF-α, CD33, Siglec-5, Siglec-7, Siglec-9, and Siglec-11.

Embodiment 46. The method of embodiment 43, wherein the immunomodulating agent is an agonist of a costimulatory immune molecule.

Embodiment 47. The method of embodiment 46, wherein the costimulatory immune molecule is selected from the group consisting of CD40, OX40, ICOS, CD28, CD137/4-1BB, CD27, IL-10, TGF-beta, TOR receptor, and glucocorticoid-induced TNFR-related protein GITR.

Embodiment 48. The method of any one of embodiments 1-47, wherein the individual does not receive radiation therapy.

Embodiment 49. The method of any one of embodiments 1-48, wherein the method further comprises radiation therapy.

Embodiment 50. The method of any one of embodiments 1-49, wherein the antimetabolite is delivered in a neoadjuvant setting.

Embodiment 51. The method of any one of embodiments 1-49, wherein the antimetabolite is delivered in an adjuvant setting.

Embodiment 52. The method of any one of embodiments 1-51, further comprising a third therapy comprising surgery, wherein delivery of antimetabolite to the individual is initiated at the time of the surgery.

Embodiment 53. The method of any one of embodiments 1-52, wherein delivery of antimetabolite to the individual is initiated during a cystoscopy.

Embodiment 54. The method of any one of embodiments 1-53, wherein the antimetabolite is delivered into the bladder by an intravesicular delivery device.

Embodiment 55. The method of embodiment 54, wherein the intravesicular device comprises a housing configured for intravesicular insertion; and a dosage form comprising antimetabolite, wherein the housing holds the dosage form and is configured to release antimetabolite in an amount effective for the treatment of a urothelial carcinoma.

Embodiment 56. The method of any one of embodiments 54-55, wherein the intravesicular drug delivery device comprises a housing which contains and controllably releases the antimetabolite and is elastically deformable between a retention shape configured to retain the device in the individual's bladder and a deployment shape for passage of the device through the individual's urethra.

Embodiment 57. The method of embodiment 56, wherein the device comprises a drug reservoir lumen bounded by a first wall and a second wall, wherein the first wall is impermeable to the drug and the second wall is permeable to the antimetabolite.

Embodiment 58. The method of embodiment 57, wherein the first wall is cylindrical.

Embodiment 59. The method of any one of embodiments 57-58, wherein the second wall is a disc-shaped.

Embodiment 60. The method of any one of embodiments 54-59, wherein the intravesicular drug delivery device comprises at least two drug reservoir lumens.

Embodiment 61. The method of any one of embodiments 54-60, wherein the antimetabolite is released from the device by osmotic pressure.

Embodiment 62. The method of any one of embodiments 54-61, wherein the antimetabolite is released from the device by diffusion.

Embodiment 63. The method of any one of embodiments 54-62, wherein the antimetabolite contained in the housing is in a non-liquid form.

Embodiment 64. The method of embodiment 63, wherein the non-liquid form is selected from the group consisting of tablets, granules, semisolids, capsules, and combinations thereof.

Embodiment 65. The method of any one of embodiments 1-64, wherein the urothelial carcinoma is bladder cancer.

Embodiment 66. The method of embodiment 65, wherein the bladder cancer is locally-advanced bladder cancer or metastatic bladder cancer.

Embodiment 67. The method of any one of embodiments 64-65, wherein the bladder cancer is muscle invasive bladder cancer.

Embodiment 68. The method of any one of embodiments 64-65, wherein the bladder cancer is non-muscle invasive bladder cancer.

Embodiment 69. The method of any one of embodiments 64-65, wherein the bladder cancer is carcinoma in situ.

Embodiment 70. The method of any one of embodiments 64-65, wherein the bladder cancer is BCG (Bacillus Calmette-Guerin) refractory cancer or papillary bladder cancer.

Embodiment 71. The method of any one of embodiments 1-70, wherein the individual is human.

Embodiment 72. The method of any one of embodiments 1-71, wherein the individual is unsuitable for systemic therapy.

Embodiment 73. The method of any one of embodiments 1-72, wherein the individual has a compromised immune system.

Embodiment 74. The method any one of embodiments 1-73, wherein the individual has a high level of an immune checkpoint protein.

Embodiment 75. The method of any one of embodiments 1-73, wherein the individual has a low level of an immune checkpoint protein.

Embodiment 76. The method of any one of embodiments 1-75, wherein the individual has a high level of a nucleoside transporter.

Embodiment 77. The method of any one of embodiments 1-75, wherein the individual has a low level of a nucleoside transporter.

Embodiment 78. The method of any one of embodiments 1-77, wherein the antimetabolite is gemcitabine, and wherein the method further comprise determining the gemcitabine/metabolite ratio in the urine, wherein a ratio below a threshold value is indicative of effective treatment.

Embodiment 79. A kit for treating a urothelial carcinoma of the lower tract in an individual comprising: a) an antimetabolite and b) a second agent, wherein the antimetabolite is in a device for local delivery to the bladder.

Embodiment 80. The kit of embodiment 79, wherein the antimetabolite is gemcitabine.

Embodiment 81. The kit of embodiment 79 or 80, wherein the second agent is an immunomodulatory agent.

Embodiment 82. A delivery device for local delivery of an antimetabolite and a second agent to the bladder of an individual, comprising: a housing containing an antimetabolite and an immunomodulatory agent, wherein the housing is configured to provide local release of the antimetabolite and the second agent into the bladder of the individual.

Embodiment 83. The delivery device of embodiment 82, wherein the antimetabolite is gemcitabine.

Embodiment 84. The delivery device of embodiment 82 or 83, wherein the second agent is an immunomodulatory agent.

Embodiment 85. The method of any of embodiments 54-56, wherein the intravesicular drug delivery device comprises a housing defining a reservoir; a first unit contained within the reservoir, the first unit comprising an antimetabolite; and a second unit contained within the reservoir in a position distinct from the first unit, wherein the second unit comprises a functional agent that facilitates in vivo release of the drug from the housing.

Embodiment 86. A method of treating muscle invasive bladder cancer in an individual comprising delivering an effective amount of gemcitabine locally to the bladder, wherein gemcitabine is delivered continuously for at least 24 hours.

Embodiment 87. A method of bladder preservation in an individual comprising delivering an effective amount of gemcitabine locally to the bladder, wherein gemcitabine is delivered continuously for at least 24 hours.

Embodiment 88. The method of embodiment 86 or 87, wherein the gemcitabine is delivered by an intravesicular device.

Embodiment 89. The method of embodiment 88, wherein the intravesicular device comprises a housing configured for intravesicular insertion; and a dosage form comprising antimetabolite, wherein the housing holds the dosage form and is configured to release antimetabolite in an amount effective for the treatment of a urothelial carcinoma.

Embodiment 90. The method of embodiment 88 or 89, wherein the intravesicular drug delivery device comprises a housing which contains and controllably releases the antimetabolite and is elastically deformable between a retention shape configured to retain the device in the individual's bladder and a deployment shape for passage of the device through the individual's urethra.

Embodiment 91. The method of any one of embodiments 88-90, wherein the intravesicular device contains 225 mg of gemcitabine.

Embodiment 92. The method of any one of embodiments 86-91, wherein the gemcitabine is delivered continuously to the bladder to the individual for a period of 24 hours to three weeks.

Embodiment 93. The method of any one of embodiments 86-92, wherein the gemcitabine is delivered continuously to the bladder of the individual for 7 days.

Embodiment 94. The method of any one of embodiments 86-93, wherein the method comprises two gemcitabine delivery periods.

Embodiment 95. The method of embodiment 94, wherein the first and second gemcitabine delivery periods are each 7 days.

Embodiment 96. The method of embodiment 94 or 95, wherein the first and second gemcitabine delivery periods are separated by a rest period of 14 days.

Embodiment 97. The method of any one of embodiments 86-96, wherein the individual is unfit for radical cystectomy.

Embodiment 98. The method of any one of embodiments 86-97, wherein the individual cannot tolerate systemic chemotherapy and/or chemotherapy with an agent other than the antimetabolite.

Embodiment 99. The method of any one of embodiments 86-98, wherein the individual does not receive a radical cystectomy.

Embodiment 100. A method of treating non-muscle invasive bladder cancer in an individual comprising delivering gemcitabine locally to the bladder, wherein gemcitabine is delivered continuously for at least 24 hours.

Embodiment 101. The method of embodiment 100, wherein the gemcitabine is delivered by an intravesicular device.

Embodiment 102. The method of embodiment 101, wherein the intravesicular device comprises a housing configured for intravesicular insertion; and a dosage form comprising antimetabolite, wherein the housing holds the dosage form and is configured to release antimetabolite in an amount effective for the treatment of a urothelial carcinoma.

Embodiment 103. The method of embodiment 101 or 102, wherein the intravesicular drug delivery device comprises a housing which contains and controllably releases the antimetabolite and is elastically deformable between a retention shape configured to retain the device in the individual's bladder and a deployment shape for passage of the device through the individual's urethra.

Embodiment 104. The method of any of embodiments 101-103, wherein the intravesicular device contains 225 mg of gemcitabine.

Embodiment 105. The method of any one of embodiments 100-105, wherein the gemcitabine is delivered continuously to the bladder to the individual for a period of 24 hours to three weeks.

Embodiment 106. The method of any one of embodiments 100-106, wherein the gemcitabine is delivered continuously to the bladder of the individual for 7 days.

Embodiment 107. The method of any one of embodiments 100-106, wherein the method comprises a first gemcitabine delivery period and a second gemcitabine delivery period.

Embodiment 108. The method of embodiment 107, wherein the first and second gemcitabine delivery periods are each 7 days.

Embodiment 109. The method of embodiment 107 or 108, wherein the first and second gemcitabine delivery periods are separated by a rest period of 14 days.

Embodiment 110. The method of any one of embodiments 86-109, wherein the individual is human.

Embodiment 111. The method of any one of embodiments 7-78, wherein the gemcitabine is delivered continuously to the bladder of the individual for 7 days.

Embodiment 112. The method of any one of embodiments 7-78, wherein the gemcitabine is delivered continuously to the bladder of the individual for three weeks.

Embodiment 113. The method of any one of embodiments 7-78, 111, or 112, wherein the method comprises two gemcitabine delivery periods.

Embodiment 114. The method of embodiment 113, wherein the first and second gemcitabine delivery periods are each 7 days.

Embodiment 115. The method of embodiment 113, wherein the first and second gemcitabine delivery periods are each 3 weeks.

Embodiment 114. The method of any of embodiments 113-115, wherein the first and second gemcitabine delivery periods are separated by a rest period.

Embodiment 115. The method of embodiment 114, wherein the rest period is 14 days.

Embodiment 116. The method of embodiment 114, wherein the rest period is 14 days to 12 weeks.

Embodiment 117. The method of any of embodiments 100-104, wherein the gemcitabine is delivered continuously to the bladder of the individual for three weeks.

Embodiment 118. The method of embodiment 107, wherein the first gemcitabine delivery period and the second gemcitabine delivery period are each three weeks.

### EXAMPLES

### Example 1

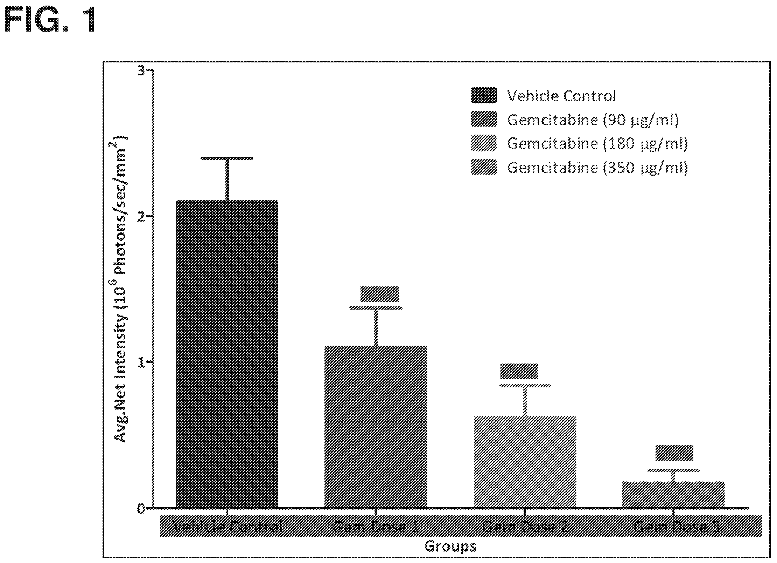

An orthotopic bladder cancer model was developed in rats to allow evaluation of the efficacy of continuous administration of gemcitabine to the bladder on treatment of a human bladder cancer cell line. Athymic nude rats were cannulated on day 0. The rats were injected with fluorescently labeled T24 human bladder cancer cells on day 3. On day 5 of the study, tumors were observable. Gemcitabine perfusion started on day 6 and continued until day 11. Three concentrations of gemcitabine were tested: 90 µg/ ml, 180 µg/ ml, and 350 µg/ ml. As shown in FIG. 1, continuous administration of 90 µg/ ml of gemcitabine over the five day study period lead to a significant reduction in tumor volume compared to untreated control. The antitumor effect was increased with administration of increasing concentrations of gemcitabine.

After day 11, the bladders were removed and subject to histological analysis. A strong inflammatory response was observed for all three gemcitabine concentrations that were tested. At the higher concentrations, larger areas of necrosis and infiltration of inflammatory cells were observed at the site of drug release.

No major significant differences were found in urine and blood parameters that were measured in the rats treated with gemcitabine. Hematuria was observed in all perfusion animals.

Gemcitabine was tolerable in all perfusion groups with mild to moderate body weight loss. Mortality (2/6) was observed in high dose treatment of gemcitabine (350 µg/ ml).

### Example 2

A minipig model was developed to investigate the effect of administering gemcitabine to the bladder locally and continuously using an intravesicular (intravesical) device. For this study, gemcitabine was continuously administered to minipigs for one week using an intravesicular (intravesical) device. The intravesicular (intravesical) device was removed at day 7. The concentration of gemcitabine and active metabolite was measured in the urine of the mini pigs following removal of the device. Based upon the level of gemcitabine and active metabolite in the urine, the therapeutic gemcitabine levels in the bladder tissue were estimated. Surprisingly, therapeutically relevant levels of gemcitabine persisted well after the device was removed.

### Example 3

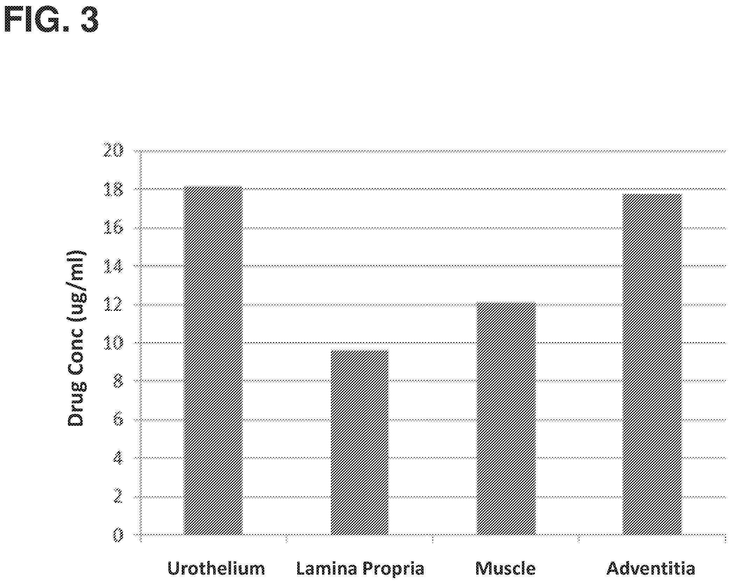

A pilot study performed was using male Sprague-Dawley rats to determine the pharmacokinetics and pharmacodynamics properties of administering gemcitabine locally and continuously to the bladder. Rats were fitted with intra-urinary bladder cannula (IUBC) and radiolabeled ¹⁴C-gemcitabine was perfused for 6 or 24 hours at a rate of 300 µL/hr at a concentration of 3.85 mg/mL of gemcitabine. The concentration of gemcitabine in various tissue layers were measured. Surprisingly, as shown in FIG. 3, gemcitabine is able to penetrate into deeper bladder tissues. The radiolabel was most concentrated in the adventitia and epithelium, followed by muscle and lamina propria, in that order.

### Example 4

A syngeneic rat model was developed to assess treatment efficacy and immune effects of continuous local administration to the bladder. Wistar rats were cannulated on day 0 and injected with NBT-II rat bladder tumor cells on day 3. Tumors were allowed to grow for 5 days prior to the start of perfusion on day 8. On days 8-13 of the study gemcitabine was perfused at a concentration of 90 µg/ ml or 180 µg/ ml. The rats were euthanized on day 14. Samples from the tumor, spleen, blood and plasma were collected for histopathology and immunohistochemistry, flow cytometry, and cytokine profiling.

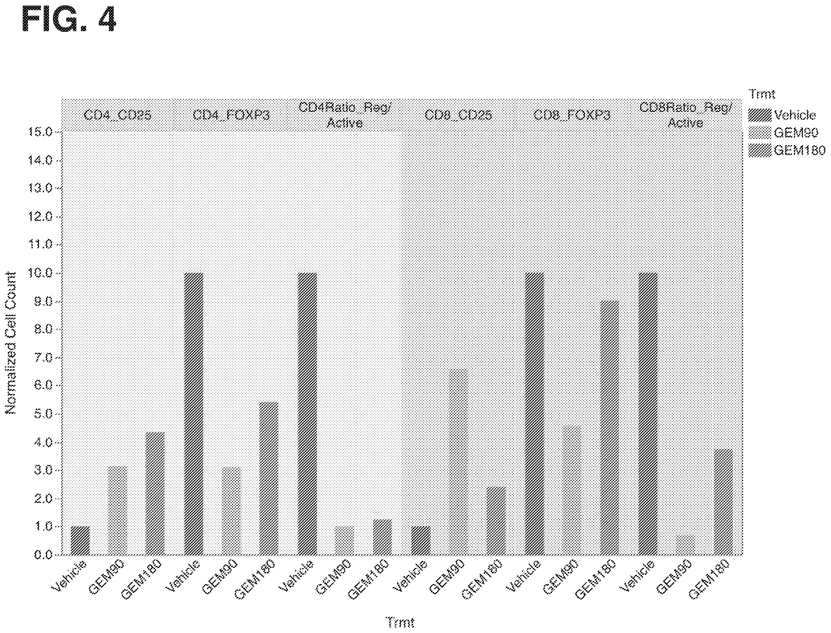

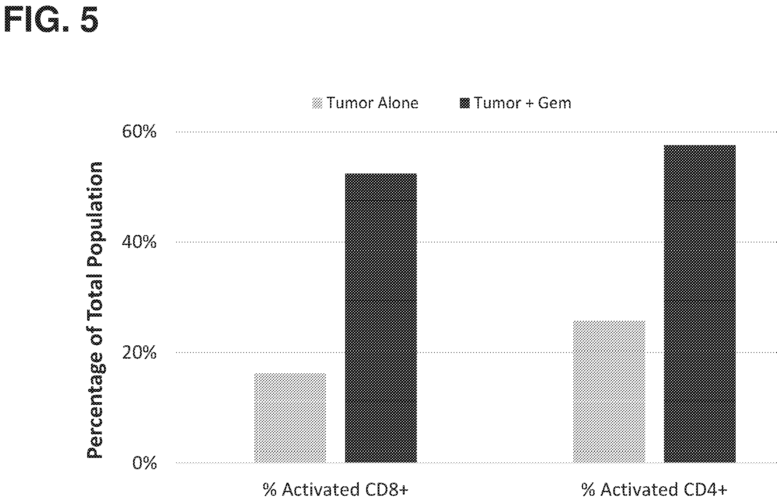

The level of activated CD8+ and CD4+ T cells were also measured in the tumor microenvironment compared to the level of CD+4 and CD8+ regulatory cells. As shown in FIG. 4, treatment with gemcitabine decreased the ratio of regulatory T cells to activated CD+4 and CD8+ T cells in the tumor microenvironment. The level of activated CD4+ and CD8+ cells was increased in the spleens of rats treated with gemcitabine (FIG. 5). Without being bound by theory, the observed effect on activated CD4+ and CD8+ cells may result from bladder tumor antigen release into the bloodstream followed by enhanced T cell trafficking and clonal expansion.

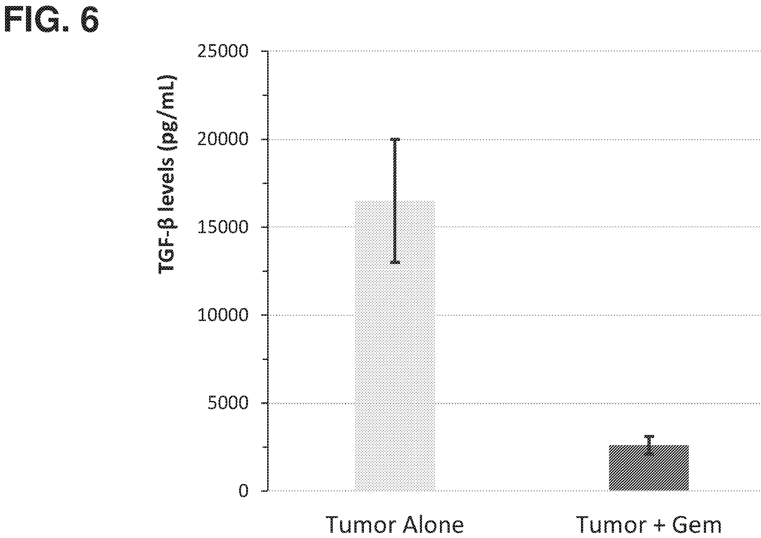

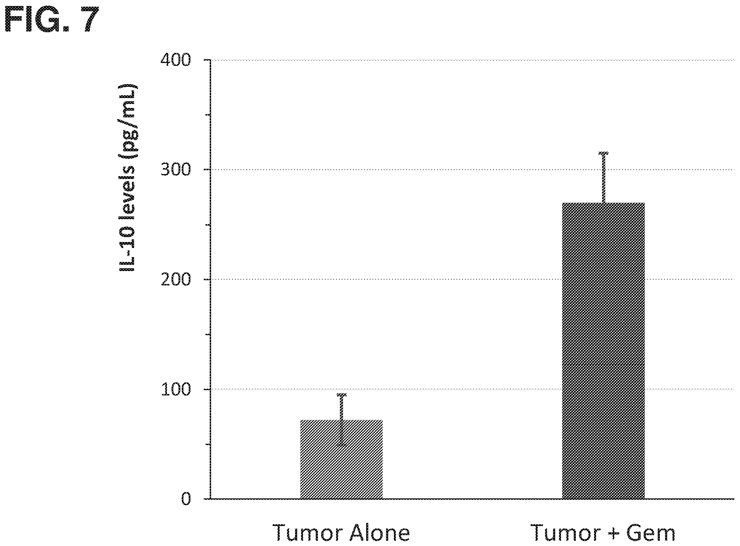

Plasma TGFβ and IL-10 levels were also measured following delivery of gemcitabine. As shown in FIG. 6, the level of TGFP was decreased in rats treated with gemcitabine compared to control animals. Inhibition of TGFβ is known to impair Treg activation. As shown in FIG. 7, IL-10 levels were increased upon delivery of gemcitabine.

These results demonstrate that continuous and local delivery of gemcitabine causes an immune response.

### Example 5

The ongoing Phase 1b study, TAR-200-101, is designed to evaluate the safety and tolerability of up to 2 dose cycles of gemcitabine administered intravesicularly at a dose of 225 mg for 7 days separated by a rest period of 14 days using the GemRIS system, over a 28-day period in patients with confirmed muscle-invasive transitional cell carcinoma of the bladder (>/= clinical and pathologic stage pT2a), who are scheduled to undergo radical cystectomy (RC) with concomitant lymphadenectomy.

Gemcitabine Releasing Intravesical System (GemRIS) is placed into the bladder through an inserter on Study Day 0 and is removed on Study Day 7. GemRIS releases gemcitabine gradually during the 7 day indwelling time. A second GemRIS is placed in the bladder on Study Day 21 and is removed on Study Day 28 which is the day of the TURBT. GemRIS is a passive, nonresorbable gemcitabine-releasing intravesicular (intravesical) system whose primary mode of action is the controlled release of gemcitabine into the bladder over a 7 day period.

Arm 1 is Residual Tumor following TURBT. TAR-200 (GemRIS) is placed into the bladder through an inserter on Study Day 0 and is removed on Study Day 7. TAR-200 releases gemcitabine gradually during the 7 day indwelling time. A second TAR-200 is placed in the bladder on Study Day 21 and is removed on Study Day 28, which is the day of the Radical Cystectomy (RC).

Arm 2 is No Residual Tumor Following TURBT. TAR-200 (GemRIS)is placed into the bladder through an inserter on Study Day 0 and is removed on Study Day 7. TAR-200 releases gemcitabine gradually during the 7 day indwelling time. A second TAR-200 is placed in the bladder on Study Day 21 and is removed on Study Day 28, which is the day of the Radical Cystectomy(RC).

Patients have histological proof of muscle-invasive transitional cell carcinoma of the bladder (stage II-IIIb)). Patients with evidence of metastatic nodal disease to the obuturator or presacral lymph nodes only may be included. Patients must have residual visible tumor following TURBT measuring no less than 3 cm. Patients included in the study are deemed ineligible for cisplatin-based chemotherapy or have refused cisplatin-based chemotherapy. Prior radiation therapy is allowed, provided that no radiation therapy was administered to the urinary bladder. Patients must be eligible and willing to undergo a cystoscopy on study for investigational product removal and a radical cystectomy following treatment.

The primary outcome measures are the number of participants with treatment emergent adverse events (TEAEs) coded with MedDRA and graded for severity with CTCAE v4.0. The secondary outcome measures are the number and percentage of participants who are tolerant of GemRIS indwelling on days 1-7 and 21-28. Plasma and urine levels of dFdC and dFdU are also measured. Secondary efficacy measures also include:
1. Number of participants who are tolerant of TAR-200 indwelling [Time Frame: From Day 0 up to Day 7]
2. Percentage of participants who are tolerant of TAR-200 indwelling[Time Frame: From Day 0 up to Day 7]
3. Number of participants who are tolerant of TAR-200 indwelling [Time Frame: From Day 21 up to Day 28]
4. Percentage of participants who are tolerant of TAR-200 indwelling [Time Frame: From Day 21 up to Day 28]
5. Cmax, plasma dFdU Analysis of Cmax (maximum concentration achieved over time) of diflourodeoxyuridine (dFdU) in plasma. [Time Frame: From Day 0 up to Day 28]
6. Tmax, plasma dFdU. Analysis of Tmax (Day at which maximum concentration was achieved) of diflourodeoxyuridine (dFdU) in plasma. [Time Frame: From Day 0 up to Day 28]
7. Cavg, plasma dFdU. Analysis of Descriptive statistics (e.g. sample size, mean and median, quartiles, minimum and maximum and box plots) of the concentration of diflourodeoxyuridine (dFdU) in plasma. [Time Frame: From Day 0 up to Day 28]
8. Cmax, plasma dFdC. Analysis of Cmax (maximum concentration achieved over time) of gemcitabine (deoxydifluorocytidine hydrochloride dFdC) in plasma. [Time Frame: From Day 0 up to Day 28]
9. Tmax, plasma dFdC. Analysis of Tmax (Day at which maximum concentration was achieved) of gemcitabine (deoxydifluorocytidine hydrochloride - dFdC) in plasma [Time Frame: From Day 0 up to Day 28]
10. Cavg, plasma dFdC. Analysis of Descriptive statistics (e.g. sample size, mean and median, quartiles, minimum and maximum and box plots) of the concentration of gemcitabine (deoxydifluorocytidine hydrochloride - dFdC) in plasma [Time Frame: From Day 0 up to Day 28].
11. Cmax, urine dFdU (Arm 1 only). Analysis of Cmax (maximum concentration achieved over time) of diflourodeoxyuridine (dFdU) in urine. [Time Frame: From Day 0 up to Day 28]
12. Tmax, urine dFdU (Arm 1 only). Analysis of Cmax (maximum concentration achieved over time) of diflourodeoxyuridine (dFdU) in urine.[Time Frame: From Day 0 up to Day 28]
13. Tmax, urine dFdU (Arm 1 only). Analysis of Cmax (maximum concentration achieved over time) of diflourodeoxyuridine (dFdU) in urine. [Time Frame: From Day 0 up to Day 28]
14. Cavg, urine dFdU (Arm 1 only). Analysis of Descriptive statistics (e.g. sample size, mean and median, quartiles, minimum and maximum and box plots) of the concentration of diflourodeoxyuridine (dFdU) in urine. [Time Frame: From Day 0 up to Day 28]
15. Cmax, urine dFdC (Arm 1 only). Analysis of Cmax (maximum concentration achieved over time) of gemcitabine (deoxydifluorocytidine hydrochloride - dFdC) in urine [Time Frame: From Day 0 up to Day 28]
16. Tmax, urine dFdC (Arm 1 only). Analysis of Tmax (Day at which maximum concentration was achieved) of gemcitabine (deoxydifluorocytidine hydrochloride - dFdC) in urine [Time Frame: From Day 0 up to Day 28].
17. Cavg, urine dFdC (Arm 1 only). Analysis of Descriptive statistics (e.g. sample size, mean and median, quartiles, minimum and maximum and box plots) of the concentration of gemcitabine (deoxydifluorocytidine hydrochloride - dFdC) in urine. [Time Frame: From Day 0 up to Day 28]
18. Preliminary anti-tumor effects assessed in tumor material (post-treatment) for immunohistochemical tissue biomarkers of drug-induced cell death (AKT) (Arm 1) [Time Frame: Anti-tumor analysis will occur at study visit Day 28.]
19. Preliminary anti-tumor effects assessed in tumor material (post-treatment) for immunohistochemical tissue biomarkers of drug-induced cell death (CD31) (Arm 1). [Time Frame: Anti-tumor analysis will occur at study visit Day 28.]
20. Preliminary anti-tumor effects assessed in tumor material (post-treatment) for immunohistochemical tissue biomarkers of drug-induced cell death (Ki67) (Arm 1) [Time Frame: Anti-tumor analysis will occur at study visit Day 28.]
21. Preliminary anti-tumor effects assessed in tumor material (post-treatment) for immunohistochemical tissue biomarkers of drug-induced cell death (TUNEL) (Arm 1) [Time Frame: Anti-tumor analysis will occur at study visit Day 28.]
22. Preliminary anti-tumor effects assessed in tumor material (post-treatment) for immunohistochemical tissue biomarkers of drug-induced cell death (CD4) (Arm 1)[Time Frame: Anti-tumor analysis will occur at study visit Day 28.]
23. Preliminary anti-tumor effects assessed in tumor material (post-treatment) for immunohistochemical tissue biomarkers of drug-induced cell death (CD8) (Arm 1) [Time Frame: Anti-tumor analysis will occur at study visit Day 28.]
24. Preliminary anti-tumor effects assessed in tumor material (post-treatment) for immunohistochemical tissue biomarkers of drug-induced cell death (PD-L1) (Arm 1) [Time Frame: Anti-tumor analysis will occur at study visit Day 28.]
25. Preliminary anti-tumor effects assessed in tumor material (post-treatment) for immunohistochemical tissue biomarkers of drug-induced cell death (AKT) (Arm 2) [Time Frame: Anti-tumor analysis will occur at study visit Day 42.]
26. Preliminary anti-tumor effects assessed in tumor material (post-treatment) for immunohistochemical tissue biomarkers of drug-induced cell death (CD31) (Arm 2) [Time Frame: Anti-tumor analysis will occur at study visit Day 42.]
27. Preliminary anti-tumor effects assessed in tumor material (post-treatment) for immunohistochemical tissue biomarkers of drug-induced cell death (Ki67) (Arm 2) [Time Frame: Anti-tumor analysis will occur at study visit Day 42.]
28. Preliminary anti-tumor effects assessed in tumor material (post-treatment) for immunohistochemical tissue biomarkers of drug-induced cell death (TUNEL) (Arm 2) [Time Frame: Anti-tumor analysis will occur at study visit Day 42.] 29. Preliminary anti-tumor effects assessed in tumor material (post-treatment) for immunohistochemical tissue biomarkers of drug-induced cell death (CD4) (Arm 2) [Time Frame: Anti-tumor analysis will occur at study visit Day 42.]
29. Preliminary anti-tumor effects assessed in tumor material (post-treatment) for immunohistochemical tissue biomarkers of drug-induced cell death (CD8) (Arm 2) [Time Frame: Anti-tumor analysis will occur at study visit Day 42.]
30. Preliminary anti-tumor effects assessed in tumor material (post-treatment) for immunohistochemical tissue biomarkers of drug-induced cell death (PD-L1]) (Arm 2) [Time Frame: Anti-tumor analysis will occur at study visit Day 42.]

The following eligibility criteria are used: Minimum age 18. Histological proof of muscle-invasive transitional cell carcinoma of the bladder (stage II-III). Subjects with evidence of metastatic nodal disease to the obuturator or presacral lymph nodes only may be included (N1 M0). Subjects with any degree of fixation of the pelvic sidewall are not eligible. In Arm 1, subjects must have residual visible tumor following TURBT. In Arm 2, subjects must be fully resected (i.e., no visible tumor or as little tumor as possible) after restaging TURBT 2-6 weeks prior to Study Day 0. Adequate bone marrow, liver, and renal function, as assessed by the following requirements conducted within 21 days prior to dosing: a. Hemoglobin ≥ 9.0 g/dL b. Absolute neutrophil count (ANC) ≥ 1,500/mm3 c. Platelet count ≥ 100,000/mm3 d. Total bilirubin ≤ 1.5xULN (upper limit of normal) e. Alanine aminotransferase (ALT) and Aspartate aminotransferase (AST) ≤ 2.5xULN f. Glomerular Filtration Rate (GFR) ≥ 30% (≥ 30 ml/min/1.73 m2) Subjects must be willing to undergo a cystoscopy on study for investigational product removal. Eligible for and willing to undergo RC per the attending urologist. Subjects must be deemed ineligible for cisplatin-based combination chemotherapy by the attending medical oncologist. Subjects medically eligible for neoadjuvant cisplatin-based combination chemotherapy who refuse this therapeutic option and understand the risks and benefits of doing so. Prior radiation therapy is allowed provided that no radiation therapy was administered to the urinary bladder. Written informed consent and Health Insurance Portability and Accountability Act of 1966 (HIP AA) authorization for release of personal health information. Age > 18 years at the time of consent.

The following exclusion criteria are used: active malignancies within 12 months with the exception of those with a negligible risk of metastasis or death treated with expected curative outcome. Prior systemic chemotherapy for transitional cell carcinoma of the bladder. Any other prior systemic chemotherapy for a non-urothelial carcinoma must have been completed > 5 years prior to initiation of study. Previous exposure to gemcitabine instillations. Currently receiving other intravesical chemotherapy. Concurrent clinically significant infections as determined by the treating investigator. Presence of any bladder or urethral anatomic feature that in the opinion of the investigator may prevent the safe placement, indwelling use or removal of TAR-200. Documented history of vesicoureteral reflux or the presence of an indwelling ureteral stent or nephrostomy tube at the time of screening. Pelvic radiotherapy administered within less than 6 months prior to enrollment. Subjects who received radiotherapy > 6 months prior to enrollment must demonstrate no cystoscopic evidence or symptoms of radiation cystitis. Bladder Post-Void Residual Volume (PVR) of > 250-mL. Active, uncontrolled urogenital bacterial, viral or fungal infections, including urinary tract infection that in the opinion of the investigator, contraindicates participation. Skin/nail fungal infections are not exclusionary. Subjects with active shingles (varicella zoster infection) will be excluded from the study. History or presence of any significant cardiovascular, pulmonary, hepatic, renal, gastrointestinal, gynecological, endocrine, immunological, dermatological, neurological or psychiatric disease or disorder that, in the opinion of the investigator, contraindicates participation. History of diagnosis of neurogenic bladder. Concomitant immunosuppressive medications, such as methotrexate or TNF inhibitors, within 2 weeks of Study Day 0, exclusive of steroid doses ≤ 5 mg daily. Difficulty providing blood samples. Unwilling or unable to provide informed consent or comply with the requirements of this protocol, including the presence of any condition (physical, mental or social) that is likely to affect the subject's return for scheduled visits and follow-up. Other unspecified reasons that, in the opinion of the investigator or TARIS, make the subject unsuitable for enrollment.

10 subjects have been dosed. There have been no treatment-related significant adverse events or discontinuations and no subject has requested that GemRIS be removed. No cystitis or gross hematuria has been reported. Moreover, there was no evidence of treatment-related anemia, neutropenia or thrombocytopenia, which are typically seen with IV or systemic administration of gemcitabine and no events resulting in a delay in RC. There was also no chemical cystitis. Preliminary pharmacokinetic analyses for 10 subjects have been completed and demonstrate measurable urine levels of gemcitabine and dFdU (an intracellular metabolite). Plasma concentrations of gemcitabine have been below the assay limit of quantitation (0.1 mcg/mL) in all plasma samples analyzed (n=55). Low but quantifiable plasma concentrations of dFdU have been occasionally observed in 10 of 55 plasma samples analyzed, ranging from 0.104 to 0.284 mcg/mL.

All enrolled subjects had a residual, grossly visible, exophytic, papillary tumor measuring no less than 3-cm in size, consistent with bulky muscle-invasive disease. Strikingly, five of these 10 treated subjects had no tumor grossly visible at the time of RC, suggesting a treatment effect in a 28-day dosing period. Of the 5 remaining subjects with visible tumor at RC, 3 exhibited a marked reduction in exhophytic tumor volume and the remaining 2 (each of whom had pT3 stage disease) had persistent disease. Four of 10 subjects had no histological evidence of residual muscle-invasive disease at cystectomy (<pT2), including one subject with a complete pathologic response. Unexpectedly, 9 of 10 subjects lacked any nodal involvement at the time of RC. Historical rates of nodal involvement are 20-40%. Furthermore, there has been no pathologic upstaging from diagnosed clinical stage (cT2, cT3) to final pathologic stage in any subject through RC. This is a surprising finding, given that 42% of patients with clinically staged MIBC are subsequently upstaged at final pathologic staging. Moreover, recent studies suggest that pathologic downs staging at the time of RC indicates a better prognosis. Preliminary pathologic efficacy results to date are summarized in Table 1. Preliminary anti-tumor effects can be assessed in tumor material (post-treatment) for assessment of immunohistochemical tissue biomarkers of drug-induced cell death (CD4, CD8, PD-L1, AKT, CD3 1, Ki67, TUNEL).

Several patients treated with GemRIS exhibited a striking antitumor effect, accompanied by peritumoral inflammation, yet the uninvolved surrounding urothelial appears normal. This is consistent with gross pathology observed after treatment with immunotherapy.

In addition to the remarkably benign safety profile seen to date, GemRIS demonstrates a striking and unexpected preliminary clinical efficacy with the 28-day window of treatment currently allowed in the cystectomy eligible population. GemRIS may provide a substantially higher benefit for cystectomy-ineligible patients in whom even longer duration of treatment is possible.

**Table 1: TAR-200-101 - Gross and Pathological Efficacy Results, as of January 6, 2017.**

| **Subject** | **Visible Tumor at Cystecomy** | **Clinical Stage of Tumor at Diagnosis** | **Final Histopathologic Stage** | **Nodal Involvment at Cystectomy** |
|---|---|---|---|---|
| OOl-USe: | Absent | cT2 | Muscle invasive tumor ablated; questionable residual pTis (non-invasive) | No |
| 003-USC | Absent | cT2 | Muscle invasive tumor ablated; residual pTis (non-invasive) | No |
| 004-USC | Absent | cT2 | Muscle invasive tumor ablated | No |
| 006-OSU | Absent | cT2 | Residual pT2b | No |
| 009-USC | Significant shrinkage | cT2 | Muscle invasive tumor ablated: residual pT1/pTis (non-invasive) | No |
| 002-USC: | Absent | cT3 | Residual pT2b; Sarcomatoid/micropappillary variant, chemo-insensitive (located in bed of scar) | No |
| 005-USC | Significant shrinkage | cT3 | Residual pT3a | No |
| 007-OSU | Significant shrinkage | cT3 | Residual pT3b | Yes |
| 011-UChi | Present | cT3 | Residual pT3a | No |
| 012-OSU | Present | cT3 | Residual pT3a | No |

### Example 5

An ongoing Phase 1b study called TAR-200-102, is designed to evaluate the safety and tolerability of up to 2 dose cycles of gemcitabine administered intravesicularly at a dose of 225 mg for 7 days separated by a rest period of 14 days using the GemRIS system, over a 28-day period in patients with recurrent low or intermediate risk non-muscle invasive bladder cancer (NMIBC) between diagnosis and transurethral resection of bladder tumors (TURBT).

Gemcitabine Releasing Intravesical System (GemRIS) is placed into the bladder through an inserter on Study Day 0 and is removed on Study Day 7. GemRIS releases gemcitabine gradually during the 7 day indwelling time. A second GemRIS is placed in the bladder on Study Day 21 and is removed on Study Day 28 which is the day of the TURBT. GemRIS is a passive, nonresorbable gemcitabine-releasing intravesicular (intravesical) system whose primary mode of action is the controlled release of gemcitabine into the bladder over a 7 day period.

Arm 1: Experimental: 7-Day RegimenTAR-200 (GemRIS) is placed into the bladder through an inserter on Study Day 0 and is removed on Study Day 7. TAR-200 releases gemcitabine gradually during the 7 day indwelling time. A second TAR-200 is placed in the bladder on Study Day 21 and is removed on Study Day 28, which is the day of the TURBT. Drug: Gemcitabine-Releasing Intravesical System (GemRIS/TAR-200). TAR-200 is a passive, nonresorbable gemcitabine releasing intravesical system whose primary mode of action is the controlled release of gemcitabine into the bladder over an indwelling period.

Arm 2: Experimental: 21-Day Regimen TAR-200 is placed into the bladder through an inserter on Study Day 0 and is removed on Study Day 21. TAR-200 releases gemcitabine gradually during the 21 day indwelling time. A second TAR-200 is placed in the bladder on Study Day 21 and is removed on Study Day 42. Drug: Gemcitabine-Releasing Intravesical System (GemRIS)/TAR-200 TAR-200 is a passive, nonresorbable gemcitabine releasing intravesical system whose primary mode of action is the controlled release of gemcitabine into the bladder over an indwelling period.

Patients that are included in the study have a documented history of histologically-confirmed low or intermediate risk urothelial carcinoma of the bladder, excluding carcinoma in situ (pTis) pathological stage pT1 (invasive into lamina propria) and high-grade disease, judged not to be muscle infiltrating (pT2 or greater) and accessible for resection.

The primary outcome measures are the number of participants with treatment emergent adverse events (TEAEs) coded with MedDRA and graded for severity with CTCAE v4.0 [Time Frame: From the point of signing the informed consent form through last study visit, up to 59 days.]. The secondary outcome measures are the number and percentage of participants who are tolerant of GemRIS indwelling on days 1-7 and 21-28. Plasma and urine levels of dFdC and dFdU are also measured.

Preliminary anti-tumor effects are assessed in tumor material (post-treatment) for assessment of immunohistochemical tissue biomarkers of drug-induced cell death (AKT, CD31, Ki67, TUNEL). The secondary outcome measures include:
1. Number of participants who are tolerant of TAR-200 indwelling (Arm l).[Time Frame: From Day 0 up to Day 7]
2. Percentage of participants who are tolerant of TAR-200 indwelling (Ann 1). [Time Frame: From Day 0 up to Day 7]
3. Number of participants who are tolerant of TAR-200 indwelling (Arm 1). [Time Frame: From Day 21 up to Day 28]
4. Percentage of participants who are tolerant of TAR-200 indwelling (Arm 1). [Time Frame: From Day 21 up to Day 28]
5. Cmax, plasma dFdU (Arm 1).Analysis of Cmax (maximum concentration achieved over time) of diflourodeoxyuridine (dFdU) in plasma. [Time Frame: From Day 0 up to Day 32]
6. Tmax, plasma dFdU (Arm 1). Analysis of Tmax (Day at which maximum concentration was achieved) of diflourodeoxyuridine (dFdU) in plasma. [Time Frame: From Day 0 up to Day 32]
7. Cavg, plasma dFdU (Arm 1). Analysis of Descriptive statistics (e.g. sample size, mean and median, quartiles, minimum and maximum and box plots) of the concentration of diflourodeoxyuridine (dFdU) in plasma. [Time Frame: From Day 0 up to Day 32]
8. Cmax, plasma dFdC (Arm 1). Analysis of Cmax (maximum concentration achieved over time) of gemcitabine (deoxydifluorocytidine hydrochloride -dFdC) in plasma. [Time Frame: From Day 0 up to Day 32]
9. Tmax, plasma dFdC (Arm 1). Analysis of Tmax (Day at which maximum concentration was achieved) of gemcitabine (deoxydifluorocytidine hydrochloride - dFdC) in plasma [Time Frame: From Day 0 up to Day 32]
10. Cavg, plasma dFdC (Arm 1). Analysis of Descriptive statistics (e.g. sample size, mean and median, quartiles, minimum and maximum and box plots) of the concentration of gemcitabine (deoxydifluorocytidine hydrochloride - dFdC) in plasma. [Time Frame: From Day 0 up to Day 32]
11. Cmax, urine dFdU (Arm 1). Analysis of Cmax (maximum concentration achieved over time) of diflourodeoxyuridine (dFdU) in urine. [Time Frame: From Day 0 up to Day 32]
12. Tmax, urine dFdU (Arm 1) Analysis of Tmax (Day at which maximum concentration was achieved) of diflourodeoxyuridine (dFdU) in urine [Time Frame: From Day 0 up to Day 32]
13. Cavg, urine dFdU (Arm 1). Analysis of Descriptive statistics (e.g. sample size, mean and median, quartiles, minimum and maximum and box plots) of the concentration of diflourodeoxyuridine (dFdU) in urine. [Time Frame: From Day 0 up to Day 32]
14. Cmax, urine dFdC (Arm 1). Analysis of Cmax (maximum concentration achieved over time) of gemcitabine (deoxydifluorocytidine hydrochloride - dFdC) in urine. [Time Frame: From Day 0 up to Day 32]
15. Tmax, urine dFdC (Arm 1). Analysis of Tmax (Day at which maximum concentration was achieved) of gemcitabine (deoxydifluorocytidine hydrochloride -- dFdC) in urine. [Time Frame: From Day 0 up to Day 32]
16. Cavg, urine dFdC (Arm 1). Analysis of Descriptive statistics (e.g. sample size, mean and median, quartiles, minimum and maximum and box plots) of the concentration of gemcitabine (deoxydifluorocytidine hydrochloride - dFdC) in urine [Time Frame: From Day 0 up to Day 32]
17. Preliminary anti-tumor effects will be assessed in tumor material (post-treatment) for assessment of immunohistochemical tissue biomarkers of drug-induced cell death (AKT, CD31, Ki67, TUNEL). (Arm 1) [Time Frame: Anti-tumor analysis will occur at the following study day visit Day 28]
18. Number of participants who are tolerant of TAR-200 indwelling (Arm 2) [Time Frame: From Day 0 up to Day 21]
19. Percentage of participants who are tolerant of TAR-200 indwelling (Arm 2) [Time Frame: From Day 0 up to Day 21]
20. Number of participants who are tolerant of TAR-200 indwelling (Arm 2) [Time Frame: From Day 21 up to Day 42]
21. Percentage of participants who are tolerant of TAR-200 indwelling (Arm 2) [Time Frame: From Day 21 up to Day 42]
22. Cmax, plasma dFdU (Arm 2) Analysis of Cmax (maximum concentration achieved over time) of diflourodeoxyuridine (dFdU) in plasma. [Time Frame: From Day 0 up to Day 47]
23. Tmax, plasma dFdU (Amr 2) Analysis of Tmax (Day at which maximum concentration was achieved) of diflourodeoxyuridine (dFdU) in plasma. [Time Frame: From Day 0 up to Day 47]
24. Cavg, plasma dFdU (Arm 2). Analysis of Descriptive statistics (e.g. sample size, mean and median, quartiles, minimum and maximum and box plots) of the concentration of diflourodeoxyuridine (dFdU) in plasma. [Time Frame: From Day 0 up to Day 47]
25. Cmax, plasma dFdC (Arm 2). Analysis of Cmax (maximum concentration achieved over time) of gemcitabine (deoxydifluorocytidine hydrochloride - dFdC) in plasma. [Time Frame: From Day 0 up to Day 47]
26. Tmax, plasma dFdC (Arm 2). Analysis of Tmax (Day at which maximum concentration was achieved) of gemcitabine (deoxydifluorocytidine hydrochloride - dFdC) in plasma [Time Frame: From Day 0 up to Day 47]
27. Cavg, plasma dFdC (Arm 2). Analysis of Descriptive statistics (e.g. sample size, mean and median, quartiles, minimum and maximum and box plots) of the concentration of gemcitabine (deoxydifluorocytidine hydrochloride - dFdC) in plasma.
28. Cmax, urine dFdU (Arm 2). Analysis of Cmax (maximum concentration achieved over time) of diflourodeoxyuridine (dFdU) in urine. [Time Frame: From Day 0 up to Day 47]
29. Tmax, urine dFdU (Arm 2). Analysis of Tmax (Day at which maximum concentration was achieved) of diflourodeoxyuridine (dFdU) in urine [Time Frame: From Day 0 up to Day 47]
30. Cavg, urine dFdU (Arm 2). Analysis of Descriptive statistics (e.g. sample size, mean and median, quartiles, minimum and maximum and box plots) of the concentration of diflourodeoxyuridine (dFdU) in urine. [Time Frame: From Day 0 up to Day 47]
31. Cmax, urine dFdC (Arm 2). Analysis of Cmax (maximum concentration achieved over time) of gemcitabine (deoxydifluorocytidine hydrochloride - dFdC) in urine. [Time Frame: From Day 0 up to Day 47]
32. Tmax, urine dFdC (Arm2). Analysis of Tmax (Day at which maximum concentration was achieved) of gemcitabine (deoxydifluorocytidine hydrochloride - dFdC) in urine. [Time Frame: From Day 0 up to Day 47]
33. Cavg, urine dFdC (Arm 2) Analysis of Descriptive statistics (e.g. sample size, mean and median, quartiles, minimum and maximum and box plots) of the concentration of gemcitabine (deoxydifluorocytidine hydrochloride - dFdC) in urine [Time Frame: From Day 0 up to Day 47]
34. Preliminary anti-tumor effects will be assessed in tumor material (post-treatment) for assessment of immunohistochemical tissue biomarkers of drug-induced cell death (AKT, CD31, Ki67, TUNEL). (Arm 2) [Time Frame: Anti-tumor analysis will occur at the following study day visit Day 42]

Inclusion criteria are: A documented history of histologically-confirmed low or intermediate risk urothelial carcinoma of the bladder, excluding carcinoma in situ (pTis), pathologic stage pT1 (invasive into lamina propria) and high- Grade disease, judged not to be muscle infiltrating (pT2 or greater) and accessible for resection. Adequate laboratory parameters. Screening urinalysis showing no clinically significant abnormalities except those attributable to bladder cancer. Not undergoing active treatment in last 3 months for prior or concurrent neoplastic disease and have fully recovered from treatment effects. Patients undergoing concurrent hormonal therapy treatment for prostate cancer are allowed to enroll.

Exclusion criteria are: Exposure to BCG therapy and/or any other intravesical. Chemotherapeutic agent less than 1 year prior to enrollment, except single postoperative instillations. Absence of visible tumor at Screening. Any previous exposure to intravesical gemcitabine instillations within the past 12 months. Presence of any bladder or urethral anatomical feature that in the opinion of the investigator may prevent the safe placement, indwelling use or removal of TAR-200 (i.e. bladder diverticula, complete incontinence). Patients with a high-Grade urine cytology at recurrence. Currently receiving other systemic or intravesical chemotherapy. Pelvic radiotherapy administered within 6 months prior to enrollment. Patients who received radiotherapy ≥ 6 months prior to enrollment must demonstrate no cystoscopic evidence or clinical symptoms of radiation cystitis. Bladder Post-Void Residual Volume (PVR) of > 250-mL. Active, uncontrolled urogenital bacterial, viral, or fungal infections, including urinary tract infection. Skin/nail fungal infections are not exclusionary. Subjects with active shingles (varicella zoster infection) will be excluded from the study. History or presence of any significant cardiovascular, pulmonary, hepatic, renal, gastrointestinal, gynecological, endocrine, immunological, dermatological, neurological or psychiatric disease or disorder that, in the opinion of the investigator, contraindicates participation. Concomitant immunosuppressive medications, such as methotrexate or TNF inhibitors, within 2 weeks of Study Day 0, exclusive of steroid doses <5 mg daily. Female subject who is pregnant (as verified by urine test at time of screening) or lactating, or of childbearing potential and not using acceptable methods of contraception. Unwilling or unable to provide informed consent or comply with the requirements of this protocol, including the presence of any condition (physical, mental or social) that is likely to affect the subject's return for scheduled visits and follow-up. Other unspecified reasons that, in the opinion of the investigator or TARIS, make the patient unsuitable for enrollment.

**Table 2: TAR-200-102 Data**

| **Subject** | **Number of Tumors Visible at Enrollment** | **Visible Evidence of Ablation at TUR** | **Gross Features at TUR** | **Final Histopathologic Stage** |
|---|---|---|---|---|
| 001-UMC | 2 | No | Some peri-tumoral inflammation | Grade 1Ta |
| 002-UMC | 3 | Yes | Small satellite tumors ablated; large tumor devitalized | Grade 2 Ta |
| 003- UMC | 1 | No | Some devitalization with necrosis | Grade 3 Ta |
| 004- UMC | 2 | No | Some peri-tumoral inflammation | Grade 2 Ta |
| 005- UMC | 2 | Yes | No tumor; only erythema visible | Ablated |
| 006- UMC | 2 | Yes | Only 1 of 2 tumors visible | Pending |
| 007- UMC | 2 | Yes | Only 1 of 2 tumors visible | Pending |
| 008- UMC | 1 | No | Pending | Ablated |

The invention is further described in the following numbered clauses:
1. A method of treating a urothelial carcinoma of the lower tract in an individual comprising administering to the individual an effective amount of an antimetabolite, wherein the antimetabolite is delivered locally to the bladder, wherein the urothelial carcinoma of the lower tract is muscle invasive bladder cancer.
2. The method of clause 1, wherein the individual is unfit for radical cystectomy.
3. The method of clause 1 or 2, wherein the individual cannot tolerate systemic chemotherapy and/or chemotherapy with an agent other than the antimetabolite.
4. The method of any one of clauses 1-3, wherein the individual does not receive a radical cystectomy.
5. A method of treating a urothelial carcinoma of the lower tract in an individual comprising administering to the individual an effective amount of an antimetabolite, wherein the antimetabolite is delivered locally to the bladder, wherein the urothelial carcinoma of the lower tract is non-muscle invasive bladder cancer.
6. A method of treating a urothelial carcinoma of the lower tract in an individual comprising administering to the individual an effective amount of an antimetabolite, wherein the antimetabolite is delivered locally to the bladder, wherein the method further comprises administering to the individual an effective amount of a second agent.
7. The method of any of clauses 1-6, wherein the antimetabolite is a nucleoside analog.
8. The method of any one of clauses 1-7, wherein the antimetabolite is gemcitabine.
9. The method of clause 8, wherein the gemcitabine is delivered continuously to the bladder of the individual for 24 hours to 6 weeks.
10. A method of bladder preservation in an individual comprising delivering an effective amount of gemcitabine locally to the bladder, wherein gemcitabine is delivered continuously to the bladder of the individual for 24 hours to 6 weeks, wherein the individual has a urothelial carcinoma of the lower tract.
11. The method of any one of clauses 8-10, wherein the gemcitabine is delivered continuously to the bladder of the individual for a period of 24 hours to three weeks.
12. The method of any one of clauses 8-11, wherein the gemcitabine is delivered continuously to the bladder of the individual for 7 days.
13. The method of any one of clauses 8-11, wherein the gemcitabine is delivered continuously to the bladder of the individual for 3 weeks.
14. The method of any one of clauses 8-13, wherein the method comprises a first gemcitabine delivery period and a second gemcitabine delivery period.
15. The method of clause 14, wherein the first and second gemcitabine delivery periods are each 7 days.
16. The method of clause 14, wherein the first and second gemcitabine delivery periods are each 3 weeks.
17. The method of any one of clauses 14-16, wherein the first and second gemcitabine delivery periods are separated by a rest period of 14 days.
18. The method of any of clauses 1-17, wherein the antimetabolite is delivered in a first phase of the delivery at a first release rate followed by a second phase of the delivery having a second release rate.
19. The method of any of clauses 1-18, wherein the antimetabolite is delivered at a dose of from about 1 mg/day to about 300 mg/day.
20. The method of any of clauses 1-19, wherein the concentration of antimetabolite in the urine is from about 0.1 µg/mL to about 200 µg/mL during the delivery period.
21. The method of clause 20, wherein the concentration of antimetabolite in the urine is from about 1 µg/mL to about 10 µg/mL during the delivery period.
22. The method of clause 20, wherein the concentration of antimetabolite in the urine is about 10 µg/mL during the delivery period.
23. The method of any of clauses 1-22, wherein the antimetabolite concentration in the plasma of the individual is less than about 1 µg/ml.
24. The method of any of clauses 1-23, wherein upon delivery of antimetabolite the ratio of antimetabolite in the urine to antimetabolite in the plasma of the individual is greater than about 500:1.
25. The method of any of clauses 1-24, comprising
   a) a first antimetabolite delivery period, wherein the concentration of antimetabolite in the urine of the individual is at least about 0.1 µg/mL;
   b) a rest period; and
   c) a second antimetabolite delivery period, wherein the concentration of antimetabolite in the urine of the individual is greater than about 0.1 µg/mL.
26. The method of clause 25, wherein the concentration of antimetabolite in the urine is greater than about 1 µg/mL for at least half of the rest period.
27. The method of any one of clauses 1-26, further comprising administering to the individual an effective amount of a second agent.
28. The method of clause 27, wherein the second agent is delivered systemically.
29. The method of clause 27, wherein the second agent or second chemotherapeutic agent is delivered locally.
30. The method of clause 29, wherein the antimetabolite and the second agent are delivered via a single delivery device.
31. The method any one of clauses 28-30, wherein the second agent is an immunomodulating agent.
32. The method of clause 31, wherein the immunomodulating agent is an immune checkpoint inhibitor.
33. The method of clause 32, wherein the immune checkpoint inhibitor is an inhibitor of an immune checkpoint protein selected from the group consisting of PD-L1, CTLA4, PD-L2, PD-1, B7-H3, B7-H4, HVEM, B- and T-lymphocyte attenuator (BTLA), Killer inhibitory receptor (KIR), GAL9, TIM3, A2AR, LAG-3, phosphatidylserine, CD27, TNF-a, CD33, Siglec-5, Siglec-7, Siglec-9, and Siglec-11.
34. The method of clause 32, wherein the immunomodulating agent is an agonist of a costimulatory immune molecule.
35. The method of clause 34, wherein the costimulatory immune molecule is selected from the group consisting of CD40, OX40, ICOS, CD28, CD137/4-1BB, CD27, IL-10, TGF-beta, TOR receptor, and glucocorticoid-induced TNFR-related protein GITR.
36. The method of clauses 27-30, wherein the second agent is a second chemotherapeutic agent.
37. The method of clause 36, wherein the chemotherapeutic agent is selected form the group consisting of paclitaxel, docetaxel, and oxaliplatin.
38. The method of any of clauses 1-37, wherein the individual does not receive radiation therapy.
39. The method of any one of clauses 1-38, wherein the method further comprises radiation therapy.
40. The method of any of clauses 1-39, wherein the antimetabolite is delivered in a neoadjuvant setting.
41. The method of any of clauses 1-39, wherein the antimetabolite is delivered in an adjuvant setting.
42. The method of any of clauses 1-41, further comprising a third therapy comprising surgery, wherein delivery of antimetabolite to the individual is initiated at the time of the surgery.
43. The method of any of clauses 1-42, wherein the antimetabolite is delivered into the bladder by an intravesicular delivery device.
44. The method of clause 43, wherein the intravesicular device contains 100 mg to 500 mg of gemcitabine.
45. The method of clause 44, wherein the intravesicular device contains 225 mg of gemcitabine.
46. The method of any of clauses 43-45, wherein the intravesicular device comprises a housing configured for intravesicular insertion; and a dosage form comprising antimetabolite, wherein the housing holds the dosage form and is configured to release antimetabolite in an amount effective for the treatment of a urothelial carcinoma of the lower tract.
47. The method of any one of clauses 43-46, wherein the intravesicular drug delivery device comprises a housing defining a reservoir;
   a first unit contained within the reservoir, the first unit comprising an antimetabolite; and
   a second unit contained within the reservoir in a position distinct from the first unit, wherein the second unit comprises a functional agent that facilitates in vivo release of the antimetabolite from the housing.
48. The method of any one of clauses 43-47, wherein the intravesicular drug delivery device comprises a housing which contains and controllably releases the antimetabolite and is elastically deformable between a retention shape configured to retain the device in the individual's bladder and a deployment shape for passage of the device through the individual's urethra.
49. The method of any one of clauses 43-48, wherein the device comprises a drug reservoir lumen bounded by a first wall and a second wall, wherein the first wall is impermeable to the drug and the second wall is permeable to the antimetabolite.
50. The method of any of clauses 43-49, wherein the intravesicular drug delivery device comprises at least two drug reservoir lumens.
51. The method of any one of clauses 43-50 wherein the antimetabolite is released from the device by osmotic pressure.
52. The method of any one of clauses 43-51, wherein the antimetabolite is released from the device by diffusion.
53. The method of any of clauses 43-52, wherein the antimetabolite contained in the housing is in a non-liquid form.
54. The method of clause 53, wherein the non-liquid form is selected from the group consisting of tablets, granules, powders, semisolids, capsules, and combinations thereof.
55. The method of any of clauses 6-54, wherein the urothelial carcinoma of the lower tract is bladder cancer.
56. The method of clause 55, wherein the bladder cancer is muscle invasive bladder cancer.
57. The method of clause 55, wherein the bladder cancer is non-muscle invasive bladder cancer.
58. The method of any of clauses 1-4, or 6-55, wherein the bladder cancer is locally-advanced bladder cancer or metastatic bladder cancer.
59. The method of any one of clauses 5-55, wherein the bladder cancer is carcinoma in situ.
60. The method of any one of clauses 1-55, wherein the bladder cancer is BCG (Bacillus Calmette-Guerin) refractory or BCG resistant cancer or papillary bladder cancer.
61. The method of any one of clauses 1-60, wherein the individual is human.
62. The method of any one of clauses 1-61, wherein the individual is unsuitable for systemic chemotherapy.
63. The method of any one of clauses 1-62, wherein the individual has a compromised immune system.
64. The method any one of clauses 1-63, wherein the individual has a high level of an immune checkpoint protein.
65. The method of any one of clauses 1-63, wherein the individual has a low level of an immune checkpoint protein.
66. The method of any one of clauses 1-65, wherein the individual has a high level of a nucleoside transporter.
67. The method of any one of clauses 1-65, wherein the individual has a low level of a nucleoside transporter.
68. The method of any one of clauses 8-67, wherein the method further comprise determining the gemcitabine/metabolite ratio in the urine, wherein a ratio below a threshold value is indicative of effective treatment.
69. A kit for treating a urothelial carcinoma of the lower tract in an individual comprising: a) an antimetabolite and b) an immunomodulating agent, wherein the antimetabolite is in a device for local delivery to the bladder.
70. A kit for treating a urothelial carcinoma of the lower tract in an individual comprising: a) an antimetabolite and b) a second agent, wherein the antimetabolite is in a device for local delivery to the bladder, wherein the urothelial carcinoma of the lower tract is muscle invasive bladder cancer.
71. The kit of clause 70, wherein the second agent is an immunomodulatory agent.
72. The kit of clause 69 or 70, wherein the antimetabolite is gemcitabine.
73. A delivery device for local delivery of an antimetabolite and a second agent to the bladder of an individual, comprising: a housing containing an antimetabolite and an immunomodulatory agent, wherein the housing is configured to provide local release of the antimetabolite and the second agent into the bladder of the individual.
74. The delivery device of clause 73, wherein the antimetabolite is gemcitabine.
75. The delivery device of clause 73 or 74, wherein the second agent is an immunomodulatory agent.
76. A method of enhancing an immune response against a urothelial carcinoma of the lower tract in an individual comprising administering to the individual an effective amount of an antimetabolite, wherein the antimetabolite is delivered locally to the bladder.

## Claims

1. Gemcitabine for use in a method of treating muscle invasive bladder cancer in an individual comprising administering to the individual an effective amount of gemcitabine, wherein about 225 mg of gemcitabine is administered locally to the bladder of the individual for about three weeks.

2. The gemcitabine for use of claim 1, wherein administration of gemcitabine comprises placing an intravesicular device comprising about 225 mg of gemcitabine into the bladder of the individual and removing the device about three weeks later.

3. The gemcitabine for use of any one of claims 1-2, wherein the individual is unfit for radical cystectomy and/or does not receive a radical cystectomy.

4. The gemcitabine for use of any one of claims 1-3, further comprising performing a TURBT prior to administering the gemcitabine, optionally wherein the tumor is maximally resected following TLTRBT, optionally wherein the individual is T0 following TURBT.

5. The gemcitabine for use of any one of claims 1-4, wherein the individual has >/= clinical and pathologic stage pT2a bladder cancer, optionally wherein the individual has cT2, cT3, or T4 bladder cancer.

6. The gemcitabine for use of any one of claims 1-5, wherein the individual has MO bladder cancer.

7. The gemcitabine for use of any one of claims 1-6, wherein the individual has transitional cell carcinoma of the bladder.

8. The gemcitabine for use of any one of claims 1-2, or 4-7, wherein the individual is scheduled to undergo a radical cystectomy.

9. The gemcitabine for use of any one of claims 1-8, further comprising administering a second agent to the individual.

10. The gemcitabine for use of claim 9, wherein the second agent is an antibody that binds to PD-1.

11. The gemcitabine for use of any one of claims 1-10, wherein the individual has refused or is not eligible for neoadjuvant cisplatin-based therapy.

12. The gemcitabine for use of any one of claims 1-11, wherein the gemcitabine is administered multiple times over a period of at least three months.

13. The gemcitabine for use of any one of claims 2-12, wherein gemcitabine is released from the intravesical device at a rate of about 10 mg/day to about 50 mg/day.

14. The gemcitabine for use of any one of claims 2-13, wherein the intravesicular device comprises a housing configured for intravesicular insertion and a dosage form comprising the gemcitabine, wherein the housing holds the dosage form and is configured to release gemcitabine in an amount effective for the treatment of muscle invasive bladder cancer;
wherein the intravesicular device comprises a housing defining a reservoir;
a first unit contained within the reservoir, the first unit comprising the gemcitabine; and a second unit contained within the reservoir in a position distinct from the first unit, wherein the second unit comprises a functional agent that facilitates in vivo release of the gemcitabine from the housing; and/or wherein the intravesicular device comprises a housing which contains and controllably releases the gemcitabine and is elastically deformable between a retention shape configured to retain the device in the individual's bladder and a deployment shape for passage of the device through the individual's urethra.

15. The gemcitabine for use of any one of claims 2-14, wherein the intravesicular device comprises a drug reservoir lumen bounded by a first wall and a second wall, wherein the first wall is impermeable to the gemcitabine and the second wall is permeable to the gemcitabine, and/or, wherein the gemcitabine is released from the intravesicular device by osmotic pressure.
